(19) Europäisches Patentamt
European Patent Office
Office européen des brevets

(11) EP 3 054 014 A2

(12) EUROPEAN PATENT APPLICATION

(43) Date of publication:
**10.08.2016 Bulletin 2016/32**

(21) Application number: **16168978.1**

(22) Date of filing: **10.05.2016**

(51) Int Cl.:
***C12N 15/82*** *(2006.01)*
***A01N 35/00*** *(2006.01)*
***A01N 37/00*** *(2006.01)*
***A01N 43/00*** *(2006.01)*
***A01N 47/00*** *(2006.01)*
***A01N 57/00*** *(2006.01)*
***A01N 59/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **BASF Plant Science Company GmbH**
**67056 Ludwigshafen (DE)**

(72) Inventor: **The designation of the inventor has not yet been filed**

(74) Representative: **BASF IP Association**
**BASF SE**
**ZRX-C6**
**67056 Ludwigshafen (DE)**

Remarks:
The complete document including Reference Tables and the Sequence Listing can be downloaded from the EPO website

# (54) USE OF A FUNGICIDE ON TRANSGENIC PLANTS

(57) The present invention relates to a method for controlling fungal infection and/or increasing plant health in cultivated plant in transgenic plants comprising application of a fungicide to the transgenic plant, transgenic plant parts, transgenic plant cells transgenic plant propagation materials, and/or at their locus of growth and/or at the locus where they are to grow.

EP 3 054 014 A2

## Description

The present invention relates to a method for controlling fungal infection and/or increasing plant health in cultivated plant in transgenic plants comprising
application of a fungicide to the transgenic plant, transgenic plant parts, transgenic plant cells transgenic plant propagation materials, and/or at their locus of growth and/or at the locus where they are to grow.

One typical problem arising in the field of pest control lies in the need to reduce the dosage rates of the active ingredient in order to reduce or avoid unfavorable environmental or toxicological effects as well increase plant health whilst still allowing effective fungus control.

It was therefore an object of the present invention to provide a method, which solves the problems of reducing the dosage rate, controlling fungal infection and/or increasing fungal health.

Surprisingly, it has now been found that the application of the fungicides to transgenic plants transformed with exogenous fungal resistance genes has a synergistic effect.

Especially, it has been found that application of the fungicide as defined in this application on transgenic plants, transgenic plant parts, transgenic plant cells, transgenic plant propagation materials and/or at their locus of growth leads and/or at the locus where they are to grow to synergistically enhanced action against fungi and/or increased plant health compared to the control rates that are possible
with the fungicide application to wildtype plants, wild type plant parts, wild type plant cells, wildtype propagation material and/or at their locus of growth and/or at the locus where are they to grow or
with transgenic plants, transgenic plant parts, transgenic plant cells, transgenic plant propagation materials without application of a fungicide.

The present invention relates to a method for controlling fungal infection and/or increasing plant health in transgenic plants, transgenic plant parts, transgenic plant cells and/or transgenic plant propagation materials comprising the application of a fungicide to the transgenic plant, transgenic plant parts, transgenic plant cells, transgenic plant propagation materials, and/or at their locus of growth and/or at the locus where are they to grow, wherein the fungicide is selected from the group consisting of

a) Respiration inhibitors,

b) Sterol biosynthesis inhibitors,

c) Nucleic acid synthesis inhibitors,

d) Inhibitors of cell division and cytoskeleton,

e) Inhibitors of amino acid and protein synthesis,

f) Signal transduction inhibitors,

g) Lipid and membrane synthesis inhibitors,

h) Inhibitors with Multi Site Action,

i) Cell wall synthesis inhibitors,

j) Plant defence inducers, and

k) Unknown mode of action

and combinations thereof.

The present invention relates to a method for controlling fungal infection and/or increasing plant health in the transgenic plant, transgenic plant parts, transgenic plant cells and/or transgenic plant propagation materials, wherein the transgenic plant, transgenic plant parts, transgenic plant cells and/or transgenic plant propagation materials is transformed with a vector construct comprising at least one exogenous gene increasing fungal resistance in a plant, plant parts, plant cells and/or plant propagation materials compared with the wildtype plant, wild type plant part, wild type plant cell and/or wildtype propagation material thereof and the above defined fungicides or combinations thereof are applied to transgenic plant, transgenic plant parts, transgenic plant cells, transgenic plant propagation materials, and/or at their locus of growth and/or at the locus where are they to grow.

## Detailed description of the invention

The present invention may be understood more readily by reference to the following detailed description of the preferred embodiments of the invention and the examples included herein.

### Definitions

Unless otherwise noted, the terms used herein are to be understood according to conventional usage by those of ordinary skill in the relevant art. In addition to the definitions of terms provided herein, definitions of common terms in molecular biology may also be found in Rieger et al., 1991 Glossary of genetics: classical and molecular, 5th Ed., Berlin: Springer-Verlag; and in Current Protocols in Molecular Biology, F.M. Ausubel et al., Eds., Current Protocols, a joint venture between Greene Publishing Associates, Inc. and John Wiley & Sons, Inc., (1998 Supplement). It is to be understood that as used in the specification and in the claims, "a" or "an" can mean one or more, depending upon the context in which it is used. Thus, for example, reference to "a cell" can mean that at least one cell can be utilized. It is to be understood that the terminology used herein is for the purpose of describing specific embodiments only and is not intended to be limiting.

Throughout this application, various publications are referenced. The disclosures of all of these publications and those references cited within those publications in their entireties are hereby incorporated by reference into this application in order to more fully describe the state of the art to which this invention pertains. Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al., 1989 Molecular Cloning, Second Edition, Cold Spring Harbor Laboratory, Plainview, N.Y.; Maniatis et al., 1982 Molecular Cloning, Cold Spring Harbor Laboratory, Plainview, N.Y.; Wu (Ed.) 1993 Meth. Enzymol. 218, Part I; Wu (Ed.) 1979 Meth Enzymol. 68; Wu et al., (Eds.) 1983 Meth. Enzymol. 100 and 101; Grossman and Moldave (Eds.) 1980 Meth. Enzymol. 65; Miller (Ed.) 1972 Experiments in Molecular Genetics, Cold Spring Harbor Laboratory, Cold Spring Harbor, N.Y.; Old and Primrose, 1981 Principles of Gene Manipulation, University of California Press, Berkeley; Schleif and Wensink, 1982 Practical Methods in Molecular Biology; Glover (Ed.) 1985 DNA Cloning Vol. I and II, IRL Press, Oxford, UK; Hames and Higgins (Eds.) 1985 Nucleic Acid Hybridization, IRL Press, Oxford, UK; and Setlow and Hollaender 1979 Genetic Engineering: Principles and Methods, Vols. 1-4, Plenum Press, New York. Abbreviations and nomenclature, where employed, are deemed standard in the field and commonly used in professional journals such as those cited herein.

"Homologues" of a protein encompass peptides, oligopeptides, polypeptides, proteins and/or enzymes having amino acid substitutions, deletions and/or insertions relative to the unmodified protein in question and having the same, essentially the same biological activity or similar as the unmodified protein from which they are derived.

"Homologues" of a nucleic acid encompass nucleotides and/or polynucleotides having nucleic acid substitutions, deletions and/or insertions relative to the unmodified nucleic acid in question, wherein the protein coded by such nucleic acids has the same, essentially the same or similar biological activity as the unmodified protein coded by the unmodified nucleic acid from which they are derived. In particular, homologues of a nucleic acid may encompass substitutions on the basis of the degenerative amino acid code.

The terms "identity", "homology" and "similarity" are used herein interchangeably. "Identity" or "homology" or "similarity" between two nucleic acids sequences or amino acid sequences refers in each case over at least 70%, at least 80%, at least 90%, at least 95%, at least 96%, at least 97%, at least 98%, at least 99%, preferably over the entire length of the respective nucleic acid sequence or the respective amino acid sequence.

Preferably, "percentage of sequence identity" is calculated by comparing two optimally aligned sequences over a particular region, determining the number of positions at which the identical base or amino acid occurs in both sequences in order to yield the number of matched positions, dividing the number of such positions by the total number of positions in the region being compared and multiplying the result by 100.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity or similarity or homology and performs a statistical analysis of the identity or similarity or homology between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity/homology/identity may also be determined using one of the methods available in the MatGAT software package (Campanella et al., BMC Bioinformatics. 2003 Jul 10;4:29. MatGAT: an application that generates similarity/homology/identity matrices using protein or DNA sequences.).

Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1); 195-7).

The sequence identity may also be calculated by means of the Vector NTI Suite 7.1 program of the company Informax (USA) employing the Clustal Method (Higgins DG, Sharp PM. Fast and sensitive multiple sequence alignments on a microcomputer. Comput Appl. Biosci. 1989 Apr; 5(2):151-1) with the following settings:
Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |
| Pairwise alignment parameter: | |
| FAST algorithm | on |
| K-tuple size | 1 |
| Gap penalty | 3 |
| Window size | 5 |
| Number of best diagonals | 5 |

Alternatively the identity may be determined according to Chenna, Ramu, Sugawara, Hideaki, Koike, Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, the web page: http://www.ebi.ac.uk/Tools/clustalw/index.html# and the following settings

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Sequence identity between the nucleic acid or protein useful according to the present invention may be optimized by sequence comparison and alignment algorithms known in the art (see Gribskov and Devereux, Sequence Analysis Primer, Stockton Press, 1991, and references cited therein) and calculating the percent difference between the nucleotide or protein sequences by, for example, the Smith-Waterman algorithm as implemented in the BESTFIT software program using default parameters (e.g., University of Wisconsin Genetic Computing Group).
A preferred program for aligning of two sequences is "NEEDLE" (The European Molecular Biology Open Software Suite (EMBOSS)). The algorithm of Needleman and Wunsch, describing how to align sequences, like ((1970) J Mol Biol 48: 443-453) is implemented in the above listed program.

Preferably the "percentage of sequence identity" is then calculated directly from the created pairwise sequence alignment: % Identity is calculated by dividing the number of identical residues through the number of all residues over the complete length of the alignment. This is multiplied by 100 % to result in % identity. A global identity calculated in this way is directly reported by programs NEEDLE with their default parameter.
A "deletion" refers to removal of one or more amino acids from a protein or to the removal of one or more nucleic acids from DNA, ssRNA and/or dsRNA.

An "insertion" refers to one or more amino acid residues or nucleic acid residues being introduced into a predetermined site in a protein or the nucleic acid.

A "substitution" refers to replacement of amino acids of the protein with other amino acids having similar properties (such as similar hydrophobicity, hydrophilicity, antigenicity, propensity to form or break $\alpha$-helical structures or beta-sheet structures).

On the nucleic acid level a substitution refers to a replacement of one or more nucleotides with other nucleotides within a nucleic acid, wherein the protein coded by the modified nucleic acid has essentially the same or a similar function. In particular homologues of a nucleic acid encompass substitutions on the basis of the degenerative amino acid code.

Amino acid substitutions are typically of single residues, but may be clustered depending upon functional constraints placed upon the protein and may range from 1 to 10 amino acids; insertions or deletion will usually be of the order of about 1 to 10 amino acid residues. The amino acid substitutions are preferably conservative amino acid substitutions. Conservative substitution tables are well known in the art (see for example Taylor W.R. (1986) The classification of amino acid conservation J Theor Biol., 119:205-18 and Table 1 below).

**Table 1:** Examples of conserved amino acid substitutions

| Residue | Conservative Substitutions | | Residue | Conservative Substitutions |
|---|---|---|---|---|
| A | G, V, I, L, M | | L | M, I, V, A, G |
| C | S, T | | N | Q |
| E | D | | Q | N |
| D | E | | P | |
| G | A, V, I, L, M | | S | T, C |
| F | Y, W | | R | K, H |
| I | V, A, G, L, M | | T | S, C |
| H | R, K | | W | Y, F |
| K | R, H | | V | I, A, G, L, M |
| M | L, I, V, A, G | | Y | F, W |

Amino acid substitutions, deletions and/or insertions may readily be made using peptide synthetic techniques well known in the art, such as solid phase peptide synthesis and the like, or by recombinant DNA manipulation.

Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gene in vitro mutagenesis (USB, Cleveland, OH), QuickChange Site Directed mutagenesis (Stratagene, San Diego, CA), PCR-mediated site-directed mutagenesis or other site-directed mutagenesis protocols.

The terms "encode" or "coding for" is used for the capability of a nucleic acid to contain the information for the amino acid sequence of a protein via the genetic code, i.e., the succession of codons each being a sequence of three nucleotides, which specify which amino acid will be added next during protein synthesis. The terms "encode" or "coding for" therefore includes all possible reading frames of a nucleic acid. Furthermore, the terms "encode" or "coding for" also applies to a nucleic acid, which coding sequence is interrupted by non-coding nucleic acid sequences, which are removed prior translation, e.g., a nucleic acid sequence comprising introns. As used herein the terms "controlling fungal infection" means reducing or preventing or delaying an infection of a trangenic plant, trangenic plant part, trangenic plant cell or trangenic plant propagation material by fungi as compared to a wild type plant, wild type plant part, wild type plant cell or wildtype propagation material or reducing the numbers of sprays or dosages of fungicides or combinations thereof.

As used herein the terms "controlling Phakopsoracea infection" means reducing or preventing or delaying an infection of a transgenic soy plant, transgenic soy plant part, transgenic soy plant cell or transgenic soy plant propagation material by Phakopsoracea, in particular Phakopsora, more particularly soybean rust or Asian Soybean Rust (ASR), more particularly Phakopsora pachyrhizi, Phakopsora meibomiae as compared to a wild type plant, wild type plant part, wild type plant cell or wild type propagation material or reducing the numbers of sprays or dosages of fungicides or combinations thereof.

As used herein the terms "controlling fusarium infection" means reducing or preventing or delaying an infection of a transgenic corn plant, transgenic corn plant part, transgenic corn plant cell or transgenic corn plant propagation material by Fusarium, in particular Fusarium graminearum, Fusarium sporotrichioides, Fusarium pseudograminearum, Fusarium culmorum, Fusarium poae, Fusarium verticillioides (Fusarium moniliforme), Fusarium subglutinans, Fusarium proliferatum, Fusarium fujikuroi), Fusarium avenaceum, Fusarium oxysporum, Fusarium virguliforme, Fusarium solani and/or preventing and/or reducing the amount of mycotoxin(s) produced by Fusarium compared to a wild type plant, wild type plant part, wild type plant cell or wild type propagation material or reducing the numbers of sprays or dosages of fungicides or combinations thereof.

Mycotoxins may be selected form the group consisting of
Fumonisins, e.g. Fumonisin B1 (FB1) and/or Fumonisin B2 (FB2),
trichothecenes, e.g. type A like T-2 toxin and/or HT-2 toxin,type B: deoxynivalenol (DON), nivalenol (NIV), 3- and 15-acetyldeoxynivalenol (3-ADON, 15-ADON),
zearalenone (ZEA),
fusarin,
fusaric acid and
combinations thereof.

The level of fungal resistance of a plant can be determined in various ways, e.g. by scoring/measuring the infected leaf area or three-dimensional space in relation to the overall area or three-dimensional space. Another possibility to determine the level of resistance is to count the number of fungal colonies on the plant or to measure the amount of spores produced by these colonies. Another way to resolve the degree of fungal infestation is to specifically measure the amount of fungal DNA by quantitative (q) PCR. Specific probes and primer sequences for most fungal pathogens are available in the literature (Frederick RD, Snyder CL, Peterson GL, et al. 2002 Polymerase chain reaction assays for the detection and discrimination of the rust pathogens Phakopsora pachyrhizi and P. meibomiae, Phytopathology 92(2) 217-227). (Nicolaisen M, Suproniene S, Nielsen LK, Lazzaro I, Spliid NH, Justesen AF. 2009 Real-time PCR for quantification of eleven individual Fusarium species in cereals. J Microbiol Methods. 2009 Mar; 76(3): 234-40.)
Another way of evaluating fungal biomass is to biochemically determining the amount of fungal specific compounds, such as ergosterol or chitin (L. M. Reid, R. W. Nicol, T. Ouellet, M. Savard, J. D. Miller, J. C. Young, D. W. Stewart, and A. W. Schaafsma (1999) Interaction of Fusarium graminearum and F. moniliforme in Maize Ears: Disease Progress, Fungal Biomass, and Mycotoxin Accumulation Phytopathology 89(11) 1028-1037; CA Roberts, RR Marquardt, AA Frohlich, RL McGraw, RG Rotter, JC Henning (1991) Chemical and spectral quantification of mold in contaminated barley; Cereal Chemistry 68(3):272-275).

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The term "hybridization" as used herein includes "any process by which a strand of nucleic acid molecule joins with a complementary strand through base pairing" (J. Coombs (1994) Dictionary of Biotechnology, Stockton Press, New York). Hybridization and the strength of hybridization (i.e., the strength of the association between the nucleic acid molecules) is impacted by such factors as the degree of complementarity between the nucleic acid molecules, stringency of the conditions involved, the Tm of the formed hybrid, and the G:C ratio within the nucleic acid molecules.

As used herein, the term "Tm" is used in reference to the "melting temperature." The melting temperature is the temperature at which a population of double-stranded nucleic acid molecules becomes half dissociated into single strands. The equation for calculating the Tm of nucleic acid molecules is well known in the art. As indicated by standard references, a simple estimate of the Tm value may be calculated by the equation: Tm=81.5+0.41 (% G+C), when a nucleic acid molecule is in aqueous solution at 1 M NaCl (see e.g., Anderson and Young, Quantitative Filter Hybridization, in Nucleic Acid Hybridization (1985). Other references include more sophisticated computations, which take structural as well as sequence characteristics into account for the calculation of Tm. Stringent conditions, are known to those skilled in the art and can be found in Current Protocols in Molecular Biology, John Wiley & Sons, N.Y. (1989), 6.3.1-6.3.6.

In particular, the term "stringency conditions" refers to conditions, wherein 100 contigous nucleotides or more, 150 contigous nucleotides or more, 200 contigous nucleotides or more or 250 contigous nucleotides or more which are a fragment or identical to the complementary nucleic acid molecule (DNA, RNA, ssDNA or ssRNA) hybridizes under conditions equivalent to hybridization in 7% sodium dodecyl sulfate (SDS), 0.5 M NaPO4, 1 mM EDTA at 50°C with washing in 2 X SSC, 0.1 % SDS at 50°C or 65°C, preferably at 65°C, with a specific nucleic acid molecule (DNA; RNA, ssDNA or ss RNA). Preferably, the hybridizing conditions are equivalent to hybridization in 7% sodium dodecyl sulfate (SDS), 0.5 M NaPO4, 1 mM EDTA at 50°C with washing in 1 X SSC, 0.1 % SDS at 50°C or 65°C, preferably 65°C, more preferably the hybridizing conditions are equivalent to hybridization in 7% sodium dodecyl sulfate (SDS), 0.5 M NaPO4, 1 mM EDTA at 50°C with washing in 0,1 X SSC, 0.1% SDS at 50°C or 65°C, preferably 65°C. Preferably, the complementary nucleotides hybridize with a fragment or the whole exogenous nucleic acids as defined pursuant to this invention. Alternatively, preferred hybridization conditions encompass hybridisation at 65°C in 1x SSC or at 42°C in 1x SSC and 50% formamide, followed by washing at 65°C in 0.3x SSC or hybridisation at 50°C in 4x SSC or at 40°C in 6x SSC and 50% formamide, followed by washing at 50°C in 2x SSC. Further preferred hybridization conditions are 0.1 % SDS, 0.1 SSD and 65°C.

The term "plant" is intended to encompass plants at any stage of maturity or development, as well as any tissues or organs (plant parts) taken or derived from any such plant unless otherwise clearly indicated by context. Plant parts include, but are not limited to, plant cells, stems, roots, flowers, ovules, stamens, seeds, leaves, embryos, meristematic regions, callus tissue, anther cultures, gametophytes, sporophytes, pollen, microspores, protoplasts, hairy root cultures, and/or the like. The present invention also includes seeds produced by the plants of the present invention. Preferably, the seeds comprise the exogenous nucleic acids as defined pursuant to this invention. In one embodiment, the seeds can develop into plants with increased resistance to fungal infection as compared to a wild-type variety of the plant seed. As used herein, a "plant cell" includes, but is not limited to, a protoplast, gamete producing cell, and a cell that regenerates into a whole plant. Tissue culture of various tissues of plants and regeneration of plants therefrom is well known in the art and is widely published.

Reference herein to an "endogenous" nucleic acid and / or protein refers to the nucleic acid and / or protein in question as found in a plant in its natural form (i.e., without there being any human intervention, in particular without genetechnological methods).

The term "exogenous" nucleic acid refers to a nucleic acid that has been introduced in a plant by means of genetechnology. An "exogenous" nucleic acid can either not occur in a plant in its natural form, be different from the nucleic acid in question as found in a plant in its natural form, or can be identical to a nucleic acid found in a plant in its natural form, but integrated not within their natural genetic environment. The corresponding meaning of "exogenous" is applied in the context of protein expression. For example, a transgenic plant containing a transgene, i.e., an exogenous nucleic acid, may, when compared to the expression of the endogenous gene, encounter a substantial increase of the expression of the respective gene or protein in total. A transgenic plant according to the present invention includes an exogenous nucleic acid optionally in combination with one or more exogenous nucleic acid(s) nucleic acids integrated at any genetic loci and optionally the plant may also include the endogenous gene within the natural genetic background corresponding to the exogenous gene. Preferably the plant, plant part or plant cell does not include the endogenous gene within the natural genetic background corresponding to the exogenous gene. The term exogenous nucleic acid may also refer to an endogenous nucleic acid in a plant that has been modified by means of genetechnology resulting in a substantial increase of the expression of the respective gene or protein in total. The modified nucleic acid may be for example a promoter or a gene, in particular a gene regulating overexpression of othe gens. Such methods may be for example CRISPR/Cas9 (WO2014/194190).

For the purposes of the invention, "recombinant" means with regard to, for example, a nucleic acid sequence, a nucleic acid molecule, an expression cassette or a vector construct comprising nucleic acid brought about by man by genetechnological methods in which either

(a) the sequences of the nucleic acids or a part thereof, or
(b) genetic control sequence(s) which are operably linked with the nucleic acid sequences according to the invention, for example a promoter, or
(c) a) and b)
are not located in their natural genetic environment within the genome of the wildtype plant or have been modified by man by genetechnological methods. The modification may take the form of, for example, a substitution, addition, deletion, inversion or insertion of one or more nucleotide residues. The natural genetic environment is understood as meaning the natural genomic or chromosomal locus in the original plant or the presence in a genomic library or the combination with the natural promoter.

For instance, a naturally occurring expression cassette - for example the naturally occurring combination of the natural promoter of the nucleic acid sequences with the corresponding nucleic acid sequence encoding a protein useful in the methods of the present invention, as defined above - becomes a recombinant expression cassette when this expression cassette is modified by man by non-natural, synthetic ("artificial") methods such as, for example, mutagenic treatment. Suitable methods are described, for example, in US 5,565,350, WO 00/15815 or US200405323. Furthermore, a naturally occurring expression cassette - for example the naturally occurring combination of the natural promoter of the nucleic acid sequences with the corresponding nucleic acid sequence encoding a protein useful in the methods of the present invention, as defined above - becomes a recombinant expression cassette when this expression cassette is not integrated in the natural genetic environment but in a different genetic environment.

The term "isolated nucleic acid" or "isolated protein" refers to a nucleic acid or protein that is not located in its natural environment, in particular its natural cellular environment. Thus, an isolated nucleic acid or isolated protein is essentially separated from other components of its natural environment. However, the skilled person in the art is aware that preparations of an isolated nucleic acid or an isolated protein can display a certain degree of impurity depending on the isolation procedure used. Methods for purifying nucleic acids and proteins are well known in the art. The isolated gene may be isolated from an organism or may be manmade, for example by chemical synthesis. In this regard, a recombinant nucleic acid may also be in an isolated form.

As used herein, the term "transgenic" refers to an organism, e.g., a plant, plant part, plant cell, callus, or propagation material that exogenously contains the nucleic acid, recombinant construct, vector or expression cassette described herein or a part thereof which is preferably introduced by non-essentially biological processes, preferably by Agrobacteria transformation. The recombinant construct or a part thereof is stably integrated into a chromosome, so that it is passed on to successive generations by clonal propagation, vegetative propagation or sexual propagation. Preferred successive generations are transgenic too. Essentially biological processes may be crossing of plants and/or natural recombination.

A transgenic plant, plants cell or tissue for the purposes of the invention is thus understood as meaning that an exogenous nucleic acid as defined for the purpose of this inventionoptionally in combination with one or more nucleic acids as defined for the purpose of this invention is integrated into the genome by means of genetechnology.

A "wild type" plant, "wild type plant part", or "wild type plant cell", or "wild type plant propagation material" means that said plant, plant part, plant cell or plant propagation material does not express exogenous nucleic acids and exogenous and proteins.

Natural locus means the location on a specific chromosome and/or the location between certain genes and/or the same sequence background as in the original plant which is transformed.

Preferably, the transgenic plant, plant cell or tissue thereof expresses the nucleic as defined for the purpose of this invention. Preferably, the transgenic plant, plant cell or tissue thereof is transformed with recombinant vector constructs comprising nucleic acids as defined for the purpose of this invention.

The term "expression" or "gene expression" means the transcription of a specific gene or specific genes or specific genetic vector construct. The term "expression" or "gene expression" in particular means the transcription of a gene or genes or genetic vector construct into structural RNA (rRNA, tRNA), or mRNA with or without subsequent translation of the latter into a protein. The process includes transcription of DNA and processing of the resulting RNA product. The term "expression" or "gene expression" can also include the translation of the mRNA and therewith the synthesis of the encoded protein, i.e., protein expression.

The term "increased expression" or "enhanced expression" or "overexpression" or "increase of content" as used herein means any form of expression that is additional to the original wild-type expression level. For the purposes of this invention, the original wild-type expression level might also be zero (absence of expression).

Methods for increasing expression of genes or gene products are well documented in the art and include, for example, overexpression driven by appropriate promoters, the use of transcription enhancers or translation enhancers, or RNAa (Li et al 2006, PNAS 103(46) 17337-42). Isolated nucleic acids which serve as promoter or enhancer elements may be introduced in an appropriate position (typically upstream) of a non-heterologous form of a polynucleotide so as to upregulate expression of a nucleic acid encoding the protein of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

The term "functional fragment" refers to any nucleic acid or protein according to the present invention which comprises merely a part of the fulllength nucleic acid or fulllength protein, respectively, but still provides the essentially same or similar function, e.g., increased fungal resistance and/or the same, essentially the same or similar biological activity when expressed in a plant. Preferably, the fragment comprises at least 70%, at least 80 %, at least 90 % at least 95%, at least 98 %, at least 99% of the original sequence. Preferably, the functional fragment comprises contiguous nucleic acids or amino acids as in the original nucleic acid or original protein, respectively.

The term "splice variant" as used herein encompasses variants of a nucleic acid sequence in which selected introns and/or exons or parts thereof have been excised, replaced, displaced or added, or in which introns have been shortened or lengthened. Thus, a splice variant can have one or more or even all introns removed or added or partially removed or partially added. According to this definition, a cDNA is considered as a splice variant of the respective introncontaining genomic sequence and vice versa. Such splice variants may be found in nature or may be manmade. Methods for predicting and isolating such splice variants are well known in the art (see for example Foissac and Schiex (2005) BMC Bioinformatics 6: 25).

The wildtype plant may express the respective endogenous nucleic acids according to the present invention. As far as overexpression of exogenous nucleic acids according to the present invention is concerned, for the purposes of this invention, the original wild-type expression level of the corresponding endogenous nucleic acids might also be zero (absence of expression).

With respect to a vector construct and/or the recombinant nucleic acid molecules, the term "operatively linked" is intended to mean that the nucleic acid to be expressed is linked to the regulatory sequence, including promoters, terminators, enhancers and/or other expression control elements (e.g. polyadenylation signals), in a manner which allows for expression of the nucleic acid (e.g. in a host plant cell when the vector is introduced into the host plant cell).

Such regulatory sequences are described, for example, in Goeddel, Gene Expression Technology: Methods in Enzymology 185, Academic Press, San Diego, CA (1990) and Gruber and Crosby, in: Methods in Plant Molecular Biology and Biotechnology, Eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the vector can depend on such factors as the choice of the host cell to be transformed, the level of expression of nucleic acid desired, and the like.

The term "introduction" or "transformation" as referred to herein encompass the transfer of an exogenous polynucleotide into a host cell, irrespective of the method used for transfer. Plant tissue capable of subsequent clonal propagation, whether by organogenesis or embryogenesis, may be transformed with a vector construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The host genome includes the nucleic acid contained in the nucleus as well as the nucleic acid contained in the plastids, e.g., chloroplasts, and / or mitochondria. The resulting transformed plant cell may then be used to regenerate a transformed plant in a manner known to persons skilled in the art.

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Nucleic acids and proteins for the purpose of the present invention:

The nucleic acid according to the present invention to be overexpressed in order to control fungal infection and/or increase the plant health is for example a nucleic acid selected from SEQ ID No. SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97 and 99. The nucleic acid according to the present invention to be overexpressed in order to achieve control fungal infection and/or increase the plant health is for example a nucleic acid encoding a protein selected from SEQ ID No. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98 and 100.

The nucleic acid and proteins having SEQ-ID-no. 1 to 100 to be overexpressed for the purpose of the present in order to achieve increased resistance to fungal pathogens are listed in the table 2 below as lead genes (LEAD) together with examples for corresponding homologes (HOM).

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 1 | DNA | Nicotiana tabacum | LEAD | - BI-1 | - |
| 2 | PRT | Nicotiana tabacum | LEAD | - BI-1 | 100 |
| 3 | DNA | Arabidopsis thaliana | LEAD | CL1a | - |
| 4 | PRT | Arabidopsis thaliana | LEAD | CL1a | 100 |
| 5 | DNA | Arabidopsis thaliana | LEAD | CL1b | - |
| 6 | PRT | Arabidopsis thaliana | LEAD | CL1b | 100 |
| 7 | DNA | Arabidopsis thaliana | LEAD | - ADR1 | - |
| 8 | PRT | Arabidopsis thaliana | LEAD | - ADR1 | 100 |
| 9 | DNA | Arabidopsis thaliana | LEAD | CL10 | - |
| 10 | PRT | Arabidopsis thaliana | LEAD | CL10 | 100 |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 11 | DNA | Arabidopsis thaliana | LEAD | CL11 | - |
| 12 | PRT | Arabidopsis thaliana | LEAD | CL11 | 100 |
| 13 | DNA | Solanum lycopersicum | LEAD | - PTI5-V84I | - |
| 14 | PRT | Solanum lycopersicum | LEAD | - PTI5-V84I | 100 |
| 15 | DNA | Arabidopsis thaliana | LEAD | CL12 | - |
| 16 | PRT | Arabidopsis thaliana | LEAD | CL12 | 100 |
| 17 | DNA | Arabidopsis thaliana | LEAD | CL13 | - |
| 18 | PRT | Arabidopsis thaliana | LEAD | CL13 | 100 |
| 19 | DNA | Pseudomonas sp | LEAD | -ACD | - |
| 20 | PRT | Pseudomonas sp | LEAD | -ACD | 100 |
| 21 | DNA | Arabidopsis thaliana | LEAD | CL14 | - |
| 22 | PRT | Arabidopsis thaliana | LEAD | CL14 | 100 |
| 23 | DNA | Arabidopsis thaliana | LEAD | CL15 | - |
| 24 | PRT | Arabidopsis thaliana | LEAD | CL15 | 100 |
| 25 | DNA | Arabidopsis thaliana | LEAD | - OCP3 | - |
| 26 | PRT | Arabidopsis thaliana | LEAD | - OCP3 | 100 |
| 27 | DNA | Arabidopsis thaliana | LEAD | CL16 | - |
| 28 | PRT | Arabidopsis thaliana | LEAD | CL16 | 100 |
| 29 | DNA | Arabidopsis thaliana | LEAD | CL17 | - |
| 30 | PRT | Arabidopsis thaliana | LEAD | CL17 | 100 |
| 31 | DNA | Arabidopsis thaliana | LEAD | - RLK2 | - |
| 32 | PRT | Arabidopsis thaliana | LEAD | - RLK2 | 100 |
| 33 | DNA | Arabidopsis thaliana | LEAD | CL18 | - |
| 34 | PRT | Arabidopsis thaliana | LEAD | CL18 | 100 |
| 35 | DNA | Arabidopsis thaliana | LEAD | CL19 | - |
| 36 | PRT | Arabidopsis thaliana | LEAD | CL19 | 100 |
| 37 | DNA | Capsicum annuum | LEAD | - CaSAR | - |
| 38 | PRT | Capsicum annuum | LEAD | - CaSAR | 100 |
| 39 | DNA | Arabidopsis thaliana | LEAD | CL2 | - |
| 40 | PRT | Arabidopsis thaliana | LEAD | CL2 | 100 |
| 41 | DNA | Arabidopsis thaliana | LEAD | CL20 | - |
| 42 | PRT | Arabidopsis thaliana | LEAD | CL20 | 100 |
| 43 | DNA | Arabidopsis thaliana | LEAD | - HCP6 | - |
| 44 | PRT | Arabidopsis thaliana | LEAD | - HCP6 | 100 |
| 45 | DNA | Arabidopsis thaliana | LEAD | CL21 | - |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 46 | PRT | Arabidopsis thaliana | LEAD | CL21 | 100 |
| 47 | DNA | Arabidopsis thaliana | LEAD | - HCP7 | - |
| 48 | PRT | Arabidopsis thaliana | LEAD | - HCP7 | 100 |
| 49 | DNA | Arabidopsis thaliana | LEAD | CL22a | - |
| 50 | PRT | Arabidopsis thaliana | LEAD | CL22a | 100 |
| 51 | DNA | Arabidopsis thaliana | LEAD | CL22b | - |
| 52 | PRT | Arabidopsis thaliana | LEAD | CL22b | 100 |
| 53 | DNA | Aspergillus nidulans | LEAD | - Hydrophobin | Hydrophobin - |
| 54 | PRT | Aspergillus nidulans | LEAD | - Hydrophobin | 100 |
| 55 | DNA | Arabidopsis thaliana | LEAD | CL23 | - |
| 56 | PRT | Arabidopsis thaliana | LEAD | CL23 | 100 |
| 57 | DNA | Arabidopsis thaliana | LEAD | CL24 | - |
| 58 | PRT | Arabidopsis thaliana | LEAD | CL24 | 100 |
| 59 | DNA | Arabidopsis thaliana | LEAD | F6H1 | - |
| 60 | PRT | Arabidopsis thaliana | LEAD | F6H1 | 100 |
| 61 | DNA | Arabidopsis thaliana | LEAD | CL25 | - |
| 62 | PRT | Arabidopsis thaliana | LEAD | CL25 | 100 |
| 63 | DNA | Arabidopsis thaliana | LEAD | CL3 | - |
| 64 | PRT | Arabidopsis thaliana | LEAD | CL3 | 100 |
| 65 | DNA | Arabidopsis thaliana | LEAD | CCoAMT | - |
| 66 | PRT | Arabidopsis thaliana | LEAD | CCoAMT | 100 |
| 67 | DNA | Arabidopsis thaliana | LEAD | CL4 | - |
| 68 | PRT | Arabidopsis thaliana | LEAD | CL4 | 100 |
| 69 | DNA | Arabidopsis thaliana | LEAD | ABC-transporter | - |
| 70 | PRT | Arabidopsis thaliana | LEAD | ABC-transporter | 100 |
| 71 | DNA | Arabidopsis thaliana | LEAD | CL5a | - |
| 72 | PRT | Arabidopsis thaliana | LEAD | CL5a | 100 |
| 73 | DNA | Arabidopsis thaliana | LEAD | CL5b | - |
| 74 | PRT | Arabidopsis thaliana | LEAD | CL5b | 100 |
| 75 | DNA | Arabidopsis thaliana | LEAD | - EIN2cterm (genomic) | - |
| 76 | PRT | Arabidopsis thaliana | LEAD | - EIN2cterm (genomic) | 100 |
| 77 | DNA | Arabidopsis thaliana | LEAD | CL6 | - |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 78 | PRT | Arabidopsis thaliana | LEAD | CL6 | 100 |
| 79 | DNA | Arabidopsis thaliana | LEAD | CL7 | - |
| 80 | PRT | Arabidopsis thaliana | LEAD | CL7 | 100 |
| 81 | DNA | Arabidopsis thaliana | LEAD | - HCP4 (genomic) | - |
| 82 | PRT | Arabidopsis thaliana | LEAD | - HCP4 (genomic) | 100 |
| 83 | DNA | Arabidopsis thaliana | LEAD | CL8 | - |
| 84 | PRT | Arabidopsis thaliana | LEAD | CL8 | 100 |
| 85 | DNA | Arabidopsis thaliana | LEAD | - RLK1 | - |
| 86 | PRT | Arabidopsis thaliana | LEAD | - RLK1 | 100 |
| 87 | DNA | Arabidopsis thaliana | LEAD | - HCP5 (genomic) | - |
| 88 | PRT | Arabidopsis thaliana | LEAD | - HCP5 (genomic) | 100 |
| 89 | DNA | Arabidopsis thaliana | LEAD | CL9 | - |
| 90 | PRT | Arabidopsis thaliana | LEAD | CL9 | 100 |
| 91 | DNA | Arabidopsis thaliana | LEAD | - MYB (genomic) | - |
| 92 | PRT | Arabidopsis thaliana | LEAD | - MYB (genomic) | 100 |
| 93 | DNA | Arabidopsis thaliana | LEAD | - MYB (genomic) | - |
| 94 | PRT | Arabidopsis thaliana | LEAD | - MYB (genomic) | 100 |
| 95 | DNA | Arabidopsis thaliana | LEAD | - ERF1 | - |
| 96 | PRT | Arabidopsis thaliana | LEAD | - ERF1 | 100 |
| 97 | DNA | Arabidopsis thaliana | LEAD | GLP9 | - |
| 98 | PRT | Arabidopsis thaliana | LEAD | GLP9 | 100 |
| 99 | DNA | Arabidopsis thaliana | LEAD | HCP7 | - |
| 100 | PRT | Arabidopsis thaliana | LEAD | HCP7 | 100 |
| 101 | DNA | Populus trichocarpa | HOM | - BI-1 | - |
| 102 | PRT | Populus trichocarpa | HOM | - BI-1 | 82.3 |
| 103 | DNA | Arabidopsis lyrata subsp. Lyrata | HOM | - BI-1 | - |
| 104 | PRT | Arabidopsis lyrata subsp. Lyrata | HOM | - BI-1 | 75.2 |
| 105 | DNA | Populus trichocarpa | HOM | - BI-1 | - |
| 106 | PRT | Populus trichocarpa | HOM | - BI-1 | 80.7 |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 107 | DNA | Arabidopsis lyrata subsp. Lyrata | HOM | - BI-1 | - |
| 108 | PRT | Arabidopsis lyrata subsp. Lyrata | HOM | - BI-1 | 75.5 |
| 109 | DNA | Arabidopsis thaliana | HOM | CL1a | - |
| 110 | PRT | Arabidopsis thaliana | HOM | CL1a | 98.1 |
| 111 | DNA | Arabidopsis thaliana | HOM | CL1b | - |
| 112 | PRT | Arabidopsis thaliana | HOM | CL1b | 98.1 |
| 113 | DNA | Arabidopsis lyrata subsp. lyrata | HOM | - ADR1 | - |
| 114 | PRT | Arabidopsis lyrata subsp. lyrata | HOM | - ADR1 | 91.1 |
| 115 | DNA | Arabidopsis lyrata subsp. lyrata | HOM | CL10 | - |
| 116 | PRT | Arabidopsis lyrata subsp. lyrata | HOM | CL10 | 97.2 |
| 117 | DNA | Arabidopsis lyrata subsp. lyrata | HOM | CL11 | - |
| 118 | PRT | Arabidopsis lyrata subsp. lyrata | HOM | CL11 | 83 |
| 119 | DNA | Arabidopsis thaliana | HOM | CL12 | - |
| 120 | PRT | Arabidopsis thaliana | HOM | CL12 | 88.5 |
| 121 | DNA | Arabidopsis lyrata subsp. lyrata | HOM | CL13 | - |
| 122 | PRT | Arabidopsis lyrata subsp. lyrata | HOM | CL13 | 93.3 |
| 123 | DNA | Pseudomonas syringae | HOM | -ACD | - |
| 124 | PRT | Pseudomonas syringae | HOM | -ACD | 89.1 |
| 125 | DNA | Pseudomonas syringae | HOM | -ACD | - |
| 126 | PRT | Pseudomonas syringae | HOM | -ACD | 89.6 |
| 127 | DNA | Pseudomonas putida | HOM | -ACD | - |
| 128 | PRT | Pseudomonas putida | HOM | -ACD | 98.8 |
| 129 | DNA | Pseudomonas syringae pv. tomato str. DC3000 | HOM | -ACD | - |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 130 | PRT | Pseudomonas syringae pv. tomato str. DC3000 | HOM | -ACD | 88.5 |
| 131 | DNA | Arabidopsis lyrata subsp. lyrata | HOM | CL14 | - |
| 132 | PRT | Arabidopsis lyrata subsp. lyrata | HOM | CL14 | 95.5 |
| 133 | DNA | Eutrema salsugineum | HOM | CL14 | - |
| 134 | PRT | Eutrema salsugineum | HOM | CL14 | 89.8 |
| 135 | DNA | Arabidopsis lyrata subsp. lyrata | HOM | CL15 | - |
| 136 | PRT | Arabidopsis lyrata subsp. lyrata | HOM | CL15 | 94.6 |
| 137 | DNA | Eutrema salsugineum | HOM | CL15 | - |
| 138 | PRT | Eutrema salsugineum | HOM | CL15 | 84.3 |
| 139 | DNA | Arabidopsis lyrata subsp. lyrata | HOM | - OCP3 | - |
| 140 | PRT | Arabidopsis lyrata subsp. lyrata | HOM | - OCP3 | 90.4 |
| 141 | DNA | Eutrema salsugineum | HOM | - OCP3 | - |
| 142 | PRT | Eutrema salsugineum | HOM | - OCP3 | 80.2 |
| 143 | DNA | Arabidopsis lyrata subsp. lyrata | HOM | CL16 | - |
| 144 | PRT | Arabidopsis lyrata subsp. lyrata | HOM | CL16 | 96.6 |
| 145 | DNA | Eutrema salsugineum | HOM | CL16 | - |
| 146 | PRT | Eutrema salsugineum | HOM | CL16 | 82.1 |
| 147 | DNA | Eutrema salsugineum | HOM | CL17 | - |
| 148 | PRT | Eutrema salsugineum | HOM | CL17 | 91.7 |
| 149 | DNA | Populus trichocarpa | HOM | CL18 | - |
| 150 | PRT | Populus trichocarpa | HOM | CL18 | 86.1 |
| 151 | DNA | Arabidopsis lyrata subsp. Lyrata | HOM | CL18 | - |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 152 | PRT | Arabidopsis lyrata subsp. Lyrata | HOM | CL18 | 89.7 |
| 153 | DNA | Populus trichocarpa | HOM | CL18 | - |
| 154 | PRT | Populus trichocarpa | HOM | CL18 | 84.8 |
| 155 | DNA | Capsicum annuum | HOM | - CaSAR | - |
| 156 | PRT | Capsicum annuum | HOM | - CaSAR | 98.8 |
| 157 | DNA | Capsicum annuum | HOM | - CaSAR | - |
| 158 | PRT | Capsicum annuum | HOM | - CaSAR | 97.7 |
| 159 | DNA | Capsicum annuum | HOM | - CaSAR | - |
| 160 | PRT | Capsicum annuum | HOM | - CaSAR | 95.3 |
| 161 | DNA | Capsicum annuum | HOM | - CaSAR | - |
| 162 | PRT | Capsicum annuum | HOM | - CaSAR | 94.2 |
| 163 | DNA | Arabidopsis lyrata subsp. lyrata | HOM | CL2 | - |
| 164 | PRT | Arabidopsis lyrata subsp. lyrata | HOM | CL2 | 92.1 |
| 165 | DNA | Capsicum annuum | HOM | CL20 | - |
| 166 | PRT | Capsicum annuum | HOM | CL20 | 85.9 |
| 167 | DNA | Populus trichocarpa | HOM | CL20 | - |
| 168 | PRT | Populus trichocarpa | HOM | CL20 | 85.5 |
| 169 | DNA | Arabidopsis lyrata subsp. Lyrata | HOM | CL20 | - |
| 170 | PRT | Arabidopsis lyrata subsp. Lyrata | HOM | CL20 | 97.8 |
| 171 | DNA | Fragaria vesca subsp. Vesca | HOM | CL20 | - |
| 172 | PRT | Fragaria vesca subsp. Vesca | HOM | CL20 | 85.5 |
| 173 | DNA | Arabidopsis lyrata subsp. lyrata | HOM | CL22b | - |
| 174 | PRT | Arabidopsis lyrata subsp. lyrata | HOM | CL22b | 92.9 |
| 175 | DNA | Arabidopsis lyrata subsp. lyrata | HOM | CL24 | - |
| 176 | PRT | Arabidopsis lyrata subsp. lyrata | HOM | CL24 | 96.7 |
| 177 | DNA | Eutrema salsugineum | HOM | CL24 | - |
| 178 | PRT | Eutrema salsugineum | HOM | CL24 | 86.1 |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 179 | DNA | Arabidopsis lyrata subsp. lyrata | HOM | F6H1 | - |
| 180 | PRT | Arabidopsis lyrata subsp. lyrata | HOM | F6H1 | 96.4 |
| 181 | DNA | Arabidopsis lyrata subsp. lyrata | HOM | F6H1 | - |
| 182 | PRT | Arabidopsis lyrata subsp. lyrata | HOM | F6H1 | 95.3 |
| 183 | DNA | Eutrema salsugineum | HOM | F6H1 | - |
| 184 | PRT | Eutrema salsugineum | HOM | F6H1 | 91.1 |
| 185 | DNA | Eutrema salsugineum | HOM | CL25 | - |
| 186 | PRT | Eutrema salsugineum | HOM | CL25 | 87.8 |
| 187 | DNA | Eutrema salsugineum | HOM | CL3 | - |
| 188 | PRT | Eutrema salsugineum | HOM | CL3 | 85.9 |
| 189 | DNA | Populus tremuloides | HOM | CCoAMT | - |
| 190 | PRT | Populus tremuloides | HOM | CCoAMT | 85.3 |
| 191 | DNA | Fragaria vesca subsp. Vesca | HOM | CCoAMT | - |
| 192 | PRT | Fragaria vesca subsp. Vesca | HOM | CCoAMT | 84.6 |
| 193 | DNA | Capsicum annuum | HOM | CCoAMT | - |
| 194 | PRT | Capsicum annuum | HOM | CCoAMT | 83.8 |
| 195 | DNA | Populus trichocarpa | HOM | CCoAMT | - |
| 196 | PRT | Populus trichocarpa | HOM | CCoAMT | 85.3 |
| 197 | DNA | Eutrema salsugineum | HOM | CL4 | - |
| 198 | PRT | Eutrema salsugineum | HOM | CL4 | 84.4 |
| 199 | DNA | Eutrema salsugineum | HOM | ABC-transporter | - |
| 200 | PRT | Eutrema salsugineum | HOM | ABC-transporter | 89.8 |
| 201 | DNA | Eutrema salsugineum | HOM | CL5a | - |
| 202 | PRT | Eutrema salsugineum | HOM | CL5a | 91.9 |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 203 | DNA | Arabidopsis lyrata subsp. lyrata | HOM | CL6 | - |
| 204 | PRT | Arabidopsis lyrata subsp. lyrata | HOM | CL6 | 82.2 |
| 205 | DNA | Camelina sativa | HOM | CL7 | - |
| 206 | PRT | Camelina sativa | HOM | CL7 | 80.6 |
| 207 | DNA | Eutrema salsugineum | HOM | CL8 | - |
| 208 | PRT | Eutrema salsugineum | HOM | CL8 | 82.3 |
| 209 | DNA | Arabidopsis lyrata subsp. lyrata | HOM | - RLK1 | - |
| 210 | PRT | Arabidopsis lyrata subsp. lyrata | HOM | - RLK1 | 92.6 |
| 211 | DNA | Populus trichocarpa | HOM | CL9 | - |
| 212 | PRT | Populus trichocarpa | HOM | CL9 | 90.7 |
| 213 | DNA | Populus trichocarpa | HOM | CL9 | - |
| 214 | PRT | Populus trichocarpa | HOM | CL9 | 90.4 |
| 215 | DNA | Populus trichocarpa | HOM | CL9 | - |
| 216 | PRT | Populus trichocarpa | HOM | CL9 | 91.6 |
| 217 | DNA | Physcomitrella patens | HOM | CL9 | - |
| 218 | PRT | Physcomitrella patens | HOM | CL9 | 88.7 |
| 219 | DNA | Arabidopsis lyrata subsp. lyrata | HOM | - MYB (genomic) | - |
| 220 | PRT | Arabidopsis lyrata subsp. lyrata | HOM | - MYB (genomic) | 90.9 |
| 221 | DNA | Eutrema salsugineum | HOM | - MYB (genomic) | - |
| 222 | PRT | Eutrema salsugineum | HOM | - MYB (genomic) | 81 |
| 223 | DNA | Arabidopsis lyrata subsp. lyrata | HOM | - ERF1 | - |
| 224 | PRT | Arabidopsis lyrata subsp. lyrata | HOM | - ERF1 | 91.3 |
| 225 | DNA | Eutrema salsugineum | HOM | - ERF1 | - |
| 226 | PRT | Eutrema salsugineum | HOM | - ERF1 | 83.3 |

(continued)

| Table 2SEQ ID NO: | Molecule type | Organism | sequence is ... | sequence belongs to LEAD | global Identity% to LEAD PROTEIN (needle; parameters: -gapopen 10.0-gapextend 0.5; matrix EBLOSUM62) |
|---|---|---|---|---|---|
| 227 | DNA | Arabidopsis lyrata subsp. lyrata | HOM | GLP9 | - |
| 228 | PRT | Arabidopsis lyrata subsp. lyrata | HOM | GLP9 | 94.1 |
| 229 | DNA | Eutrema salsugineum | HOM | GLP9 | - |
| 230 | PRT | Eutrema salsugineum | HOM | GLP9 | 86 |
| 231 | DNA | Arabidopsis lyrata subsp. lyrata | HOM | HCP7 | - |
| 232 | PRT | Arabidopsis lyrata subsp. lyrata | HOM | HCP7 | 92.1 |
| 233 | DNA | Camelina sativa | HOM | HCP7 | - |
| 234 | PRT | Camelina sativa | HOM | HCP7 | 81.1 |
| 235 | DNA | artificial | vector construct (fig 2) | - | - |
| 236 | DNA | artificial | primer | - | - |
| 237 | DNA | artificial | primer | - | - |

All the nucleic acid sequences mentioned herein (single-stranded and double-stranded DNA and RNA sequences, for example cDNA and mRNA) can be produced in a known way by chemical synthesis from the nucleotide building blocks, e.g. by fragment condensation of individual overlapping, complementary nucleic acid building blocks of the double helix. The sequences are obtained from different databases. Chemical synthesis of oligonucleotides can, for example, be performed in a known way, by the phosphoamidite method (Voet, Voet, 2nd edition, Wiley Press, New York, pages 896-897). The accumulation of synthetic oligonucleotides and filling of gaps by means of the Klenow fragment of DNA polymerase and ligation reactions as well as general cloning techniques are described in Sambrook et al. (1989), see below.

Promoters according to the present invention may be constitutive, inducible, in particular pathogen-inducible, developmental stage-preferred, cell type-preferred, tissue-preferred or organ-preferred. Examples for suitable promoters and terminators are:

p-PcUbi::gene::t-ocs
p-SUPER::CCoAOMT1::t-nos
p-Glyma14g06680::gene:: t-StCATHD
p-SUPER::gene::t-nos

The PcUbi promoter regulates constitutive expression of the ubi4-2 gene (accession number X64345) of Petroselinum crispum (Kawalleck, P., Somssich, I. E., Feldbrügge, M., Hahlbrock, K., & Weisshaar, B. (1993). Polyubiquitin gene expression and structural properties of the ubi4-2 gene in Petroselinum crispum. Plant molecular biology, 21(4), 673-684. The p-Super promoter consists of three identical Octapine Synthase Enhancers followed by a MAS promoter (Lee et al., 2007 Plant Physiology Vol145 Issue 4 1294-1300). The p-Glyma14g06680 promoter has been identified in a screen for genes that are predominantly expressed in the leaf of soybean. The promoter regulates the expression of the gene Glyma14g06680, which is most likely a water channel protein (WO12127373)
T-ocs and t-NOS terminators are both dervived from Agrobacterium (Gielen, J., et al. "The complete nucleotide sequence of the TL-DNA of the Agrobacterium tumefaciens plasmid pTiAch5." The EMBO journal 3.4 (1984): 835. T-ocs is the terminator of the octopine synthase gene and t-NOS is the terminator of the nopaline synthase gene of Agrobacterium tumefaciens The StCATHD-pA is the terminator of the cathepsin D inhibitor gene from Solanum tuberosum (t-StCat) (Herbers et al. 1994)

One type of recombinant vector construct is a "plasmid," which refers to a circular double stranded DNA loop into which additional DNA segments can be ligated. Another type of vector is a viral vector, wherein additional DNA segments can be ligated into the viral genome. Certain vector constructs are capable of autonomous replication in a host plant cell into which they are introduced. Other vector constructs are integrated into the genome of a host plant cell upon introduction into the host cell, and thereby are replicated along with the host genome. In particular the vector construct is capable of directing the expression of gene to which the vectors is operatively linked. However, the invention is intended to include such other forms of expression vector constructs, such as viral vectors (e.g., potato virus X, tobacco rattle virus, and/or Gemini virus), which serve equivalent functions.

Suitable methods for transforming or transfecting host cells including plant cells are well known in the art of plant biotechnology. Any method may be used to transform the recombinant expression vector into plant cells to yield the transgenic plants of the invention. General methods for transforming dicotyledonous plants are disclosed, for example, in U.S. Pat. Nos. 4,940,838; 5,464,763, and the like. Methods for transforming specific dicotyledonous plants, for example, cotton, are set forth in U.S. Pat. Nos. 5,004,863; 5,159,135; and 5,846,797. Soy transformation methods are set forth in U.S. Pat. Nos. 4,992,375; 5,416,011; 5,569,834; 5,824,877; 6,384,301 and in EP 0301749B1 may be used. Transformation methods may include direct and indirect methods of transformation. Suitable direct methods include polyethylene glycol induced DNA uptake, liposome-mediated transformation (US 4,536,475), biolistic methods using the gene gun (Fromm ME et al., Bio/Technology. 8(9):833-9, 1990; Gordon-Kamm et al. Plant Cell 2:603, 1990), electroporation, incubation of dry embryos in DNA-comprising solution, and microinjection. In the case of these direct transformation methods, the plasmids used need not meet any particular requirements. Simple plasmids, such as those of the pUC series, pBR322, M13mp series, pACYC184 and the like can be used. If intact plants are to be regenerated from the transformed cells, an additional selectable marker gene is preferably located on the plasmid. The direct transformation techniques are equally suitable for dicotyledonous and monocotyledonous plants.

Transformation can also be carried out by bacterial infection by means of Agrobacterium (for example EP 0 116 718), viral infection by means of viral vectors (EP 0 067 553; US 4,407,956; WO 95/34668; WO 93/03161) or by means of pollen (EP 0 270 356; WO 85/01856; US 4,684,611). *Agrobacterium* based transformation techniques (especially for dicotyledonous plants) are well known in the art. The Agrobacterium strain *(e.g., Agrobacterium tumefaciens* or *Agrobacterium rhizogenes)* comprises a plasmid (Ti or Ri plasmid) and a T-DNA element which is transferred to the plant following infection with *Agrobacterium.* The T-DNA (transferred DNA) is integrated into the genome of the plant cell. The T-DNA may be localized on the Ri- or Tiplasmid or is separately comprised in a so-called binary vector. Methods for the *Agrobacterium*-mediated transformation are described, for example, in Horsch RB et al. (1985) Science 225:1229. The *Agrobacterium-mediated* transformation is best suited to dicotyledonous plants but has also been adapted to monocotyledonous plants. The transformation of plants by Agrobacteria is described in, for example, White FF, Vectors for Gene Transfer in Higher Plants, Transgenic Plants, Vol. 1, Engineering and Utilization, edited by S.D. Kung and R. Wu, Academic Press, 1993, pp. 15 - 38; Jenes B et al. Techniques for Gene Transfer, Transgenic Plants, Vol. 1, Engineering and Utilization, edited by S.D. Kung and R. Wu, Academic Press, 1993, pp. 128-143; Potrykus (1991) Annu Rev Plant Physiol Plant Molec Biol 42:205- 225. Transformation may result in transient or stable transformation and expression. Although a nucleotide sequence of the present invention can be inserted into any plant and plant cell falling within these broad classes, it is particularly useful in crop plant cells.

The genetically modified plant cells can be regenerated via all methods with which the skilled worker is familiar. Suitable methods can be found in the above mentioned publications by S.D. Kung and R. Wu, Potrykus or Höfgen and Willmitzer.

After transformation, plant cells or cell groupings may be selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above. The transformed plants may also be directly selected by screening for the presence of the exogenous nucleic acid(s) and/or protein(s) as defined pursuant to the present invention.

Following DNA transfer and regeneration, putatively transformed plants may also be evaluated, for instance using Southern analysis and/or quantitative PCR, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques or crossed with appropriate tester lines to generate hybrids. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed root-stock grafted to an untransformed scion).
Preferably, constructs or vectors or expression cassettes are not present in the genome of the original plant or are present in the genome of the transgenic plant not at their natural locus of the genome of the original plant.

The term "locus of growth" may be understood as field, greenhouse, growth chamber, soil or any other substrate which is used for growing plants. The locus where they (plants) are to grow may be understood as field, greenhouse, growth chamber, soil or any other substrate which is later used for growing plants.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring. Preferably, the term plant propagation material denotes seeds.

The term "plant part" is to be understood to denote all parts of the plant such as cells, leaves, stem, bulbs, roots, fruits, tubersrhizomes, shoots, sprouts and other parts of the plant.

Preferred plants, from which "transgenic plants" can be derived can be selected from the group consisting of cereals such as wheat, corn (maize), barley, rye, sorghum, and oat, banana, cabbage, canola (rapeseed), cotton, peanut, potato, rice, soybean, sugar beet, sugarcane, sunflower, sweet corn, tobacco, tomato, more preferably from the group consisting of canola (rapeseed), cotton, corn (maize), potato, rice, soybean, sugar beet, sugar cane, most preferably from corn (maize), soybean and rice.

The present invention relates to a method for controlling fungal infection and/or increasing plant health in transgenic plant, transgenic plant parts, transgenic plant cells and/or transgenic plant propagation materials comprising the application of a fungicide to transgenic plant, transgenic plant parts, transgenic plant cells, transgenic plant propagation materials, and/or at their locus of growth and/or at their locus there are they to grow, wherein the fungicide is selected from the group consisting of

a) Respiration inhibitors,
b) Sterol biosynthesis inhibitors,
c) Nucleic acid synthesis inhibitors,
d) Inhibitors of cell division and cytoskeleton,
e) Inhibitors of amino acid and protein synthesis,
f) Signal transduction inhibitors,
g) Lipid and membrane synthesis inhibitors,
h) Inhibitors with Multi Site Action,
i) Cell wall synthesis inhibitors,
j) Plant defence inducers, and
k) Unknown mode of action
and combinations thereof,
wherein the transgenic plant is overexpressing exogenous protein(s),

(a) wherein said exogenous protein(s) is(are) encoded by

(i) nucleic acid(s) having at least 70% identity, at least 80% identity, at least 90% identity, at least 95% identity with sequence(s) selected from the group consisting of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97 and 99 or functional fragment(s) thereof, or splice variant(s) thereof;
(ii) nucleic acid(s) coding for protein(s) having at least 70% identity, at least 80% identity, at least 90% identity, at least 95% identity with sequence(s) selected from the group consisting of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98 and 100 or functional fragment(s) thereof;
(iii) exogenous nucleic acid(s) capable of hybridizing under stringent conditions with complementary sequence(s) of any of the nucleic acid(s) according to (i) or (ii); or
(iv) nucleic acid(s) encoding the same protein(s) as the nucleic acid(s) of (i) to (iii) above, but differing from the nucleic acid(s) of (i) to (iii) above due to the degeneracy of the genetic code;

operably linked with a promoter and a transcription termination sequence.

In one embodiment of the present invention the fungicides are selected from the group consisting of :

A) Respiration inhibitors

- Inhibitors of complex III at Qo site (e. g. strobilurins): azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-N-[2-[(1,4-dimethyl-5-phenyl-pyrazol-3-yl)oxylmethyl]phenyl]-N-methoxy-carbamate (A.1.22), 1-[3-chloro-2-[[1-(4-chlorophenyl)-1 H-pyrazol-3-yl]oxymethyl]phenyl]-4-methyl-tetrazol-5-one (A.1.23), 1-[3-bromo-2-[[1-(4-chlorophenyl)pyrazol-3-yl]oxymethyl]phenyl]-4-methyltetrazol-5-one (A.1.24), 1-[2-[[1-(4-chlorophenyl)pyrazol-3-yl]oxymethyl]-3-methylphenyl]-4-methyl-tetrazol-5-one (A.1.25), 1-[2-[[1-(4-chlorophenyl)pyrazol-3-yl]oxymethyl]-3-fluoro-phenyl]-4-methyl-tetrazol-5-one (A.1.26), 1-[2-[[1-(2,4-dichlorophenyl)pyrazol-3-yl]oxymethyl]-3-fluoro-phenyl]-4-methyl-tetrazol-5-one (A. 1.27), 1-[2-[[4-(4-chlorophenyl)thiazol-2-yl]oxymethyl]-3-methyl-phenyl]-4-methyl-tetrazol-5-one (A.1.28), 1-[3-chloro-2-[[4-(p-tolyl)thiazol-2-yl]oxymethyl]phenyl]-4-methyl-tetrazol-5-one (A.1.29), 1-[3-cyclopropyl-2-[[2-methyl-4-(1-methylpyrazol-3-yl)phenoxy]methyl]phenyl]-4-methyl-tetrazol-5-one (A.1.30), 1-[3-(difluoromethoxy)-2-[[2-methyl-4-(1-methylpyrazol-3-yl)phenoxy]methyl]phenyl]-4-methyl-tetrazol-5-one (A.1.31), 1-methyl-4-[3-methyl-2-[[2-methyl-4-(1-methylpyrazol-3-yl)phenoxy]methyl]phenyl]tetrazol-5-one (A.1.32), 1-methyl-4-[3-methyl-2-[[1-[3-(trifluoromethyl)phenyl]-ethylideneamino]oxymethyl]phenyl]tetrazol-5-one (A.1.33), (Z,2E)-5-[1-(2,4-dichlorophenyl)pyrazol-3-yl]-oxy-2-methoxyimino-N,3-dimethyl-pent-3-enamide (A.1.34), (Z,2E)-5-[1-(4-chlorophenyl)pyrazol-3-yl]oxy-2-methoxyimino-N,3-dimethyl-pent-3-enamide (A.1.35), (Z,2E)-5-[1-(4-chloro-2-fluoro-phenyl)pyrazol-3-yl]oxy-2-methoxyimino-N,3-dimethyl-pent-3-enamide (A.1.36),
- inhibitors of complex III at Qi site: cyazofamid (A.2.1), amisulbrom (A.2.2), [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate (A.2.3), [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate (A.2.4), [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate (A.2.5), [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate (A.2.6); (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate (A.2.7), (3S,6S,7R,8R)-8-benzyl-3-[3-[(isobutyryloxy)methoxy]-4-methoxypicolinamido]-6-methyl-4,9-dioxo-1,5-dioxonan-7-yl isobutyrate (A.2.8);
- inhibitors of complex II (e. g. carboxamides): benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.14), penthiopyrad (A.3.15), sedaxane (A.3.16), tecloftalam (A.3.17), thifluzamide (A.3.18), N-(4'-trifluoromethylthiobiphenyl-2-yl)-3-difluoromethyl-1-methyl-1 H-pyrazole-4-carboxamide (A.3.19), N-(2-(1,3,3-trimethylbutyl)-phenyl)-1,3-dimethyl-5-fluoro-1 H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethylindan-4-yl)pyrazole-4-carboxamide (A.3.22), 1,3-dimethyl-N-(1,1,3-trimethylindan-4-yl)pyrazole-4-carboxamide (A.3.23), 3-(trifluoromethyl)-1,5-dimethyl-N-(1,1,3-trimethylindan-4-yl)pyrazole-4-carboxamide (A.3.24), 1,3,5-tri-methyl-N-(1,1,3-trimethylindan-4-yl)pyrazole-4-carboxamide (A.3.25), N-(7-fluoro-1,1,3-trimethyl-indan-4-yl)-1,3-dimethyl-pyrazole-4-carboxamide (A.3.26), N-[2-(2,4-dichlorophenyl)-2-methoxy-1-methyl-ethyl]-3-(difluoromethyl)-1-methyl-pyrazole-4-carboxamide (A.3.27);
- other respiration inhibitors (e. g. complex I, uncouplers): diflumetorim (A.4.1), (5,8-difluoroquinazolin-4-yl)-{2-[2-fluoro-4-(4-trifluoromethylpyridin-2-yloxy)-phenyl]-ethyl}-amine (A.4.2); nitrophenyl derivates: binapacryl (A.4.3), dinobuton (A.4.4), dinocap (A.4.5), fluazinam (A.4.6); ferimzone (A.4.7); organometal compounds: fentin salts, such as fentin-acetate (A.4.8), fentin chloride (A.4.9) or fentin hydroxide (A.4.10); ametoctradin (A.4.11); and silthiofam (A.4.12);

B) Sterol biosynthesis inhibitors (SBI fungicides)

- C14 demethylase inhibitors (DMI fungicides): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[rel-(2S;3R)-3-(2-chlorophenyl)-2-(2,4-difluorophenyl)-oxiranylmethyl]-5-thiocyanato-1 H-[1,2,4]triazolo (B.1.31), 2-[rel-(2S;3R)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chloro-phenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.51); imidazoles: imazalil (B.1.42), pefurazoate (B.1.43), prochloraz (B.1.44), triflumizol (B.1.45); pyrimidines, pyridines and piperazines: fenarimol (B.1.46), nuarimol (B.1.47), pyrifenox (B.1.48), triforine (B.1.49), [3-(4-chloro-2-fluoro-phenyl)-5-(2,4-difluorophenyl)isoxazol-4-yl]-(3-pyridyl)methanol (B.1.50);
- Delta14-reductase inhibitors: aldimorph (B.2.1), dodemorph (B.2.2), dodemorphacetate (B.2.3), fenpropimorph (B.2.4), tridemorph (B.2.5), fenpropidin (B.2.6), piperalin (B.2.7), spiroxamine (B.2.8);
- Inhibitors of 3-keto reductase: fenhexamid (B.3.1);

C) Nucleic acid synthesis inhibitors

- phenylamides or acyl amino acid fungicides: benalaxyl (C.1.1), benalaxyl-M (C.1.2), kiralaxyl (C.1.3), metalaxyl (C.1.4), metalaxyl-M (mefenoxam, C.1.5), ofurace (C.1.6), oxadixyl (C.1.7);
- others: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7);

D) Inhibitors of cell division and cytoskeleton

- tubulin inhibitors, such as benzimidazoles, thiophanates: benomyl (D1.1), carbendazim (D1.2), fuberidazole (D1.3), thiabendazole (D1.4), thiophanate-methyl (D1.5); triazolopyrimidines: 5-chloro-7-(4-methylpiperidin-1-yl)-6-(2,4,6-trifluorophenyl)-[1,2,4]triazolo[1,5-a]pyrimidine (D1.6);
- other cell division inhibitors: diethofencarb (D2.1), ethaboxam (D2.2), pencycuron (D2.3), fluopicolide (D2.4), zoxamide (D2.5), metrafenone (D2.6), pyriofenone (D2.7);

E) Inhibitors of amino acid and protein synthesis

- methionine synthesis inhibitors (anilino-pyrimidines): cyprodinil (E.1.1), mepanipyrim (E.1.2), pyrimethanil (E.1.3);
- protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6), polyoxine (E.2.7), validamycin A (E.2.8);

F) Signal transduction inhibitors

- MAP / histidine kinase inhibitors: fluoroimid (F.1.1), iprodione (F.1.2), procymidone (F.1.3), vinclozolin (F.1.4), fenpiclonil (F.1.5), fludioxonil (F.1.6);
- G protein inhibitors: quinoxyfen (F.2.1);

G) Lipid and membrane synthesis inhibitors

- Phospholipid biosynthesis inhibitors: edifenphos (G.1.1), iprobenfos (G.1.2), pyrazophos (G.1.3), isoprothiolane (G.1.4);

- lipid peroxidation: dicloran (G.2.1), quintozene (G.2.2), tecnazene (G.2.3), tolclofosmethyl (G.2.4), biphenyl (G.2.5), chloroneb (G.2.6), etridiazole (G.2.7);
- phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7) and N-(1-(1-(4-cyano-phenyl)ethanesulfonyl)-but-2-yl) carbamic acid-(4-fluorophenyl) ester (G.3.8);
- compounds affecting cell membrane permeability and fatty acides: propamocarb (G.4.1 );
- fatty acid amide hydrolase inhibitors: oxathiapiprolin (G.5.1), 2-{3-[2-(1-{[3,5-bis(difluoromethyl-1 H-pyrazol-1-yl]acetyl}piperidin-4-yl)-1,3-thiazol-4-yl]-4,5-dihydro-1,2-oxazol-5-yl}phenyl methanesulfonate (G.5.2), 2-{3-[2-(1-{[3,5-bis(difluoromethyl)-1 H-pyrazol-1-yl]acetyl}piperidin-4-yl) 1,3-thiazol-4-yl]-4,5-dihydro-1,2-oxazol-5-yl}-3-chlorophenyl methanesulfonate (G.5.3);

H) Inhibitors with Multi Site Action

- inorganic active substances: Bordeaux mixture (H.1.1), copper acetate (H.1.2), copper hydroxide (H.1.3), copper oxychloride (H.1.4), basic copper sulfate (H.1.5), sulfur (H.1.6);
- thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
- organochlorine compounds (e. g. phthalimides, sulfamides, chloronitriles): anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11), N-(4-chloro-2-nitro-phenyl)-N-ethyl-4-methyl-benzenesulfonamide (H.3.12);

- guanidines and others: guanidine (H.4.1), dodine (H.4.2), dodine free base (H.4.3), guazatine (H.4.4), guazatine-acetate (H.4.5), iminoctadine (H.4.6), iminoctadinetriacetate (H.4.7), iminoctadine-tris(albesilate) (H.4.8), dithianon (H.4.9), 2,6-dimethyl-1 H,5H-[1,4]dithiino[2,3-c:5,6-c']dipyrrole-1,3,5,7(2H,6H)-tetraone (H.4.10);

I) Cell wall synthesis inhibitors

- inhibitors of glucan synthesis: validamycin (I.1.1), polyoxin B (I.1.2);
- melanin synthesis inhibitors: pyroquilon (I.2.1), tricyclazole (I.2.2), carpropamid (I.2.3), dicyclomet (I.2.4), fenoxanil (I.2.5);

J) Plant defence inducers

- acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);

K) Unknown mode of action

- bronopol (K.1.1), chinomethionat (K.1.2), cyflufenamid (K.1.3), cymoxanil (K.1.4), dazomet (K.1.5), debacarb (K.1.6), diclomezine (K.1.7), difenzoquat (K.1.8), difenzoquat-methylsulfate (K.1.9), diphenylamin (K.1.10), fenpyrazamine (K.1.11), flumetover (K.1.12), flusulfamide (K.1.13), flutianil (K.1.14), methasulfocarb (K.1.15), nitrapyrin (K.1.16), nitrothal-isopropyl (K.1.18), oxathiapiprolin (K.1.19), tolprocarb (K.1.20), oxin-copper (K.1.21), proquinazid (K.1.22), tebufloquin (K.1.23), tecloftalam (K.1.24), triazoxide (K.1.25), 2-butoxy-6-iodo-3-propylchromen-4-one (K.1.26), 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone (K.1.27), 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone (K.1.28), 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone (K.1.29), N-(cyclopropylmethoxyimino-(6-difluoro-methoxy-2,3-difluorophenyl)-methyl)-2-phenyl acetamide (K.1.30), N'-(4-(4-chloro-3-trifluoromethyl-phenoxy)-2,5-dimethyl-phenyl)-N-ethyl-N-methyl formamidine (K.1.31), N'-(4-(4-fluoro-3-trifluoromethyl-phenoxy)-2,5-dimethyl-phenyl)-N-ethyl-N-methyl formamidine (K.1.32), N'-(2-methyl-5-trifluoromethyl-4-(3-trimethylsilanyl-propoxy)-phenyl)-N-ethyl-N-methyl formamidine (K.1.33), N'-(5-difluoromethyl-2-methyl-4-(3-tri-methylsilanyl-propoxy)-phenyl)-N-ethyl-N-methyl formamidine (K.1.34), methoxyacetic acid 6-tert-butyl-8-fluoro-2,3-dimethyl-quinolin-4-yl ester (K.1.35), 3-[5-(4-methylphenyl)-2,3-dimethyl-isoxazolidin-3-yl]-pyridine (K.1.36), 3-[5-(4-chlorophenyl)-2,3-dimethyl-isoxazolidin-3-yl]-pyridine (pyrisoxazole) (K.1.37), N-(6-methoxy-pyridin-3-yl) cyclopropanecarboxylic acid amide (K.1.38), 5-chloro-1-(4,6-di-methoxy-pyrimidin-2-yl)-2-methyl-1 H-benzoimidazole (K.1.39), 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluorophenyl]propan-2-ol (K.1.43), 2-[2-fluoro-6-[(8-fluoro-2-methyl-3-quinolyl)oxy]phenyl]propan-2-ol (K.1.44), 3-(5-fluoro-3,3,4,4-tetramethyl-3,4-dihydroisoquinolin-1-yl)-quinoline (K.1.45), 3-(4,4-difluoro-3,3-dimethyl-3,4-dihydroisoquinolin-1-yl)quinoline (K.1.46), 3-(4,4,5-trifluoro-3,3-dimethyl-3,4-dihydroisoquinolin-1-yl)quinoline (K.1.47), 9-fluoro-2,2-dimethyl-5-(3-quinolyl)-3H-1,4-benzoxazepine (K.1.48)

and combinations thereof.

In regard to the instant invention the term fungi includes, but is not limited to the following genera and species:

*Alternaria* spp. (Alternaria leaf spot) on rice, soybeans, potatoes (e. g. *A. solani* or *A. alternata*), and wheat; *Ascochyta* spp. on cereals, e. g. *A. tritici* (anthracnose) on wheat and *A. hordei* on barley; *Bipolaris* and *Drechslera* spp. (teleomorph: *Cochliobolus* spp.), e. g. Southern leaf blight *(D. maydis)* or Northern leaf blight (*B. zeicola*) on corn, e. g. spot blotch (*B. sorokiniana*) on cereals and e. g. *B. oryzae* on rice; *Blumeria* (formerly *Erysiphe*) *graminis* (powdery mildew) on cereals (e. g. on wheat or barley); *Cercospora* spp. (Cercospora leaf spots) on corn (e. g. Gray leaf spot: *C. zeae-maydis*) on soybeans (e. g. *C. sojina* or *C. kikuchii*) and rice; *Cladosporium* spp. on cereals, e. g. *C. herbarum* (black ear) on wheat; *Claviceps purpurea* (ergot) on cereals; *Cochliobolus* (anamorph: *Helminthosporium* of *Bipolaris)* spp. (leaf spots) on corn *(C. carbonum),* cereals (e. g. *C. sativus*, anamorph: *B. sorokiniana*) and rice (e. g. *C. miyabeanus*, anamorph: *H. oryzae*); *Colletotrichum* (teleomorph: *Glomerella*) spp. (anthracnose) on corn (e. g. *C. graminicola:* Anthracnose stalk rot) and soybeans (e. g. *C. truncatum* or *C. gloeosporioides*); *Corticium* spp., e. g. *C. sasakii* (sheath blight) on rice; *Corynespora cassiicola* (leaf spots) on soybeans; *Dematophora* (teleomorph: *Rosellinia*) *necatrix* (root and stem rot) on soybeans; *Diaporthe* spp., e. g. *D. phaseolorum* (damping off) on soybeans; *Drechslera* (syn. *Helminthosporium,* teleomorph: *Pyrenophora)* spp. on corn, cereals, such as barley (e. g. *D. teres*, net blotch) and wheat (e. g. *D. tritici-repentis*: tan spot) and rice; *Entyloma oryzae* (leaf smut) on rice; *Epicoccum* spp. (black mold) on wheat; *Exserohilum* (syn. *Helminthosporium*) spp. on corn (e. g. *E. turcicum*); *Fusarium* (teleomorph: *Gibberella)* spp. (wilt, root or stem rot) on various plants, such as *F. graminearum* or *F. culmorum* (root rot, scab or head blight) on cereals (e. g. wheat or barley), *F. solani* (f. sp. *glycines* now syn. *F. virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae)* and rice (e. g. *G. fujikuroi*: Bakanae disease); Grainstaining complex on rice; *Helminthosporium* spp. (syn. *Drechslera*, teleomorph: *Cochliobolus*) on corn, cereals and rice; *Macrophomina phaseolina* (syn. *phaseoli*) (root and stem rot) on soybeans; *Microdochium* (syn. *Fusarium) nivale* (pink snow mold) on cereals (e. g. wheat or barley); *Microsphaera diffusa* (powdery mildew) on soybeans; *Mycosphaerella* spp. on cereals, such as e. g. *M. graminicola* (anamorph: *Septoria tritici*, Septoria blotch) on wheat; *Peronospora* spp. (downy mildew) soybeans (e. g. *P. manshurica); Phakopsora pachyrhizi* and *P. meibomiae* (soybean rust) on soybeans; *Phialophora* spp. on soybeans (e. g. *P. gregata*: stem rot); *Phomopsis* spp. on soybeans (e. g. stem rot: *P. phaseoli*, teleomorph: *Diaporthe phaseolorum*); *Physoderma maydis* (brown spots) on corn; *Phytophthora* spp. soybeans (e. g. *P. megasperma*, syn. *P. sojae*;; *Polymyxa* spp., e. g. on cereals, such as barley and wheat *(P. graminis*); *Pseudocercosporella herpotrichoides* (eyespot, teleomorph: *Tapesia yallundae*) on cereals, e. g. wheat or barley; *Puccinia* spp. (rusts) on cereals, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea,* rice blast) on rice and *P. grisea* on cereals; *Pythium* spp. (damping-off) on rice, corn, wheat, soybeans, (e. g. *P. ultimum* or *P. aphanidermatum*); *Ramularia* spp., e. g. *R. collo-cygni* (Ramularia leaf spots, Physiological leaf spots) on barley; *Rhizoctonia* spp. on rice and corn, e. g. *R. solani* (root and stem rot) on soybeans, *R. solani* (sheath blight) on rice or *R. cerealis* (Rhizoctonia spring blight) on wheat or barley; *Rhynchosporium secalis* (scald) on barley, rye and triticale; *Sarocladium oryzae* and *S. attenuatum* (sheath rot) on rice; *Sclerotinia* spp. (stem rot or white mold) on field crops, such as soybeans (e. g. *S. rolfsii* or *S. sclerotiorum*); *Septoria* spp. on various plants, e. g. *S. glycines* (brown spot) on soybeans, *S. tritici* (Septoria blotch) on wheat and *S.* (syn. *Stagonospora*) nodorum (Stagonospora blotch) on cereals; *Setospaeria* spp. (leaf blight) on corn (e. g. *S. turcicum,* syn. *Helminthosporium turcicum*); *Sphacelotheca* spp. (smut) on corn, (e. g. *S. reiliana*: head smut), sorghum; *Stagonospora* spp. on cereals, e. g. *S. nodorum* (Stagonospora blotch, teleomorph: *Leptosphaeria* [syn. *Phaeosphaeria] nodorum)* on wheat; *Thielaviopsis* spp. (black root rot) on soybeans, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye;; *Ustilago* spp. (loose smut) on cereals (e. g. *U. nuda* and *U. avaenae*), corn (e.

g. *U. maydis*: corn smut).

In one embodiment of the present invention the term fungal infection is rust fungus, downy mildew, powdery mildew, leaf spot, late blight, fusarium and/or septoria. The rust fungus infection may be Phakopsora meibomiae, Phakopsora pachyrhizi, Fusarium graminearum and/or Fusarium verticolloides.

In one embodiment of the present invention the transgenic plant is selected from the group consisting of soy, rice, wheat, barley, rye, and corn, wherein the plant is preferably soy or corn.

In one embodiment of the present invention the plant is soy and the fungicide is selected from the group consisting of:

Pyraclostrobin, Azoxystrobin, Trifloxystrobin, Picoxystrobin, (Z,2E)-5-[1-(2,4-dichlorophenyl)pyrazol-3-yl]-oxy-2-methoxyimino-N,3-dimethyl-pent-3-enamide, (Z,2E)-5-[1-(4-chlorophenyl)pyrazol-3-yl]oxy-2-methoxyimino-N,3-dimethyl-pent-3-enamide, 1-[3-chloro-2-[[1-(4-chlorophenyl)-1 H-pyrazol-3-yl]oxymethyl]phenyl]-4-methyl-tetrazol-5-one, 1-[3-bromo-2-[[1-(4-chlorophenyl)pyrazol-3-yl]oxymethyl]phenyl]-4-methyl-tetrazol-5-one, 1-[2-[[1-(4-chlorophenyl)pyrazol-3-yl]oxymethyl]-3-methyl-phenyl]-4-methyl-tetrazol-5-one, 1-[2-[[1-(4-chlorophenyl)pyrazol-3-yl]oxymethyl]-3-fluoro-phenyl]-4-methyl-tetrazol-5-one, 1-[2-[[1-(2,4-dichlorophenyl)pyrazol-3-yl]oxymethyl]-3-fluoro-phenyl]-4-methyl-tetrazol-5-one, 1-[2-[[4-(4-chlorophenyl)thiazol-2-yl]oxymethyl]-3-methyl-phenyl]-4-methyl-tetrazol-5-one, 1-[3-chloro-2-[[4-(p-tolyl)thiazol-2-yl]oxymethyl]phenyl]-4-methyltetrazol-5-one, 1-[3-cyclopropyl-2-[[2-methyl-4-(1-methylpyrazol-3-yl)phenoxy]methyl]phenyl]-4 methyl-tetrazol-5-one, 1-[3-(difluoromethoxy)-2-[[2-methyl-4-(1 methylpyrazol-3 yl)phenoxy]methyl]phenyl]-4-methyl-tetrazol-5-one, 1-methyl-4-[3-methyl-2 [[2 methyl-4-(1-methylpyrazol-3-yl)phenoxy]methyl]phenyl]tetrazol-5-one, 1-methyl-4-[3-methyl-2-[[1-[3-(trifluoromethyl)phenyl]-ethylideneamino]oxymethyl]phenyl]tetrazol-5 one, Fluxapyroxad, N-[9-(dichloromethylene)-1,2,3,4-tetrahydro-1,4-me¬thanonaphthalen-5-yl]-3-(difluoromethyl)-1-methyl-1 H-pyrazole-4-carboxamide, Sedaxane, Penflufen, 3-(difluoromethyl)-1-methyl-N-(1,1,3-trimethylindan-4-yl)pyrazole-4-carboxamide; 3-(trifluoromethyl)-1-methyl-N-(1,1,3-trimethylindan-4-yl)pyrazole-4-carboxamide; 1,3-dimethyl-N-(1,1,3-trimethylindan-4-yl)pyrazole-4-carboxamide; 3-(trifluoromethyl)-1,5-dimethyl-N-(1,1,3-trimethylindan-4-yl)pyrazole-4-carboxamide; 3-(difluoromethyl)-1,5-dimethyl-N-(1,1,3-trimethylindan-4-yl)pyrazole-4-carboxamide; 1,3,5-trimethyl-N-(1,1,3-trimethylindan-4-yl)pyrazole-4-carboxamide, Carboxin, Fluazinam, Fentin salts (fentin acetate, fentin chloride, fentin hydroxide), epoxiconazole, prothioconazole, difenoconazole, tebuconazole, 1-[rel-(2S;3R)-3-(2-chlorophenyl)-2-(2,4-difluorophenyl)-oxiranylmethyl]-5 thiocyanato-1 H-[1,2,4]triazole, 2-[rel-(2S;3R)-3-(2-chlorophenyl)-2-(2,4-difluorophenyl)-oxiranylmethyl]-2H [1,2,4]triazole-3-thiol, 2-[2-chloro-4-(4-chlorophenoxy)phenyl]-1 (1,2,4-triazol-1-yl)pentan-2-ol, 1-[4-(4-chlorophenoxy)-2-(trifluoromethyl)phenyl]-1 cyclopropyl-2-(1,2,4-triazol-1-yl)ethanol, 2-[4-(4-chlorophenoxy)-2-(trifluoromethyl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chlorophenoxy)-2-(trifluoromethyl)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)pentan-2-ol, 2-[4-(4-fluorophenoxy)-2-(trifluoromethyl)phenyl]-1-(1,2,4-triazol-1-yl)propan-2-ol, 2-[2-chloro-4-(4-chlorophenoxy)phenyl]-1-(1,2,4-triazol-1-yl)pent-3-yn-2-ol, cyproconazole, flutriafol, myclobutanil, Metalaxyl, Thiophanate-methyl, Carbendazim, Fludioxonil, thiram, chlorothalonil and combinations thereof.

In one embodiment of the present invention the plant is corn and the fungicide is selected from the group consisting of:

Pyraclostrobin, Azoxystrobin, Trifloxystrobin. Picoxystrobin, Fluoxastrobin, Fluxapyroxad, Carboxin, epoxiconazole, metconazole, prothioconazole, difenoconazole, propiconazole, tebuconazole, cyproconazole, flutriafol, myclobutanil, diniconazole, ipconazole, triadimefon, captan, Metalaxyl, Carbendazim, Thiabendazole, Fludioxonil, mancozeb, thiram and combinations thereof.

In a further specific embodiment, the present invention relates to a method of controlling fungi and/or increasing the health of plants by treating transgenic plants, transgenic plant parts, transgenic plant cells, transgenic plant propagation materials, preferably seeds of transgenic plants, and/or their locus of growth and/or at the locus where they are to grow with a fungicide, wherein the transgenic plant is soy or corn and is expressing exogenous proteins

(a) wherein said exogenous protein(s) is(are) encoded by

(i) nucleic acid(s) having at least 70% identity, at least 80% identity, at least 90% identity, at least 95% identity with any sequence(s) as defined in column 2 of Table 3 or functional fragment(s) thereof, or splice variant(s) thereof;

(ii) nucleic acid(s) coding for protein(s) having at least 70% identity, at least 80% identity, at least 90% identity, at least 95% identity with any sequence(s) as defined in column 3 of Table 3 or functional fragment(s) thereof;
(iii) exogenous nucleic acid(s) capable of hybridizing under stringent conditions with complementary sequence(s) of any of the nucleic acid(s) according to (i) or (ii); or
(iv) nucleic acid(s) encoding the same protein(s) as the nucleic acid(s) of (i) to (iii) above, but differing from the nucleic acid(s) of (i) to (iii) above due to the degeneracy of the genetic code;

and any fungicide is as defined as in table T4.

Table T3

| No | DNA | protein | plant |
|---|---|---|---|
| T3-1 | SEQ-ID-No.1 | SEQ-ID-No.2 | Soy or corn |
| T3-2 | SEQ-ID-No.3 | SEQ-ID-No.4 | Soy or corn |
| T3-3 | SEQ-ID-No.5 | SEQ-ID-No.6 | Soy or corn |
| T3-4 | SEQ-ID-No.7 | SEQ-ID-No.8 | Soy or corn |
| T3-5 | SEQ-ID-No.9 | SEQ-ID-No.10 | Soy or corn |
| T3-6 | SEQ-ID-No.11 | SEQ-ID-No.12 | Soy or corn |
| T3-7 | SEQ-ID-No.13 | SEQ-ID-No.14 | Soy or corn |
| T3-8 | SEQ-ID-No.15 | SEQ-ID-No.16 | Soy or corn |
| T3-9 | SEQ-ID-No.17 | SEQ-ID-No.18 | Soy or corn |
| T3-10 | SEQ-ID-No.19 | SEQ-ID-No.20 | Soy or corn |
| T3-11 | SEQ-ID-No.21 | SEQ-ID-No.22 | Soy or corn |
| T3-12 | SEQ-ID-No.23 | SEQ-ID-No.24 | Soy or corn |
| T3-13 | SEQ-ID-No.25 | SEQ-ID-No.26 | Soy or corn |
| T3-14 | SEQ-ID-No.27 | SEQ-ID-No.28 | Soy or corn |
| T3-15 | SEQ-ID-No.29 | SEQ-ID-No.30 | Soy or corn |
| T3-16 | SEQ-ID-No.31 | SEQ-ID-No.32 | Soy or corn |
| T3-17 | SEQ-ID-No.33 | SEQ-ID-No.34 | Soy or corn |
| T3-18 | SEQ-ID-No.35 | SEQ-ID-No.36 | Soy or corn |
| T3-19 | SEQ-ID-No.37 | SEQ-ID-No.38 | Soy or corn |
| T3-20 | SEQ-ID-No.39 | SEQ-ID-No.40 | Soy or corn |
| T3-21 | SEQ-ID-No.41 | SEQ-ID-No.42 | Soy or corn |
| T3-22 | SEQ-ID-No.43 | SEQ-ID-No.44 | Soy or corn |
| T3-23 | SEQ-ID-No.45 | SEQ-ID-No.46 | Soy or corn |
| T3-24 | SEQ-ID-No.47 | SEQ-ID-No.48 | Soy or corn |
| T3-25 | SEQ-ID-No.49 | SEQ-ID-No.50 | Soy or corn |
| T3-26 | SEQ-ID-No.51 | SEQ-ID-No.52 | Soy or corn |
| T3-27 | SEQ-ID-No.53 | SEQ-ID-No.54 | Soy or corn |
| T3-28 | SEQ-ID-No.55 | SEQ-ID-No.56 | Soy or corn |
| T3-29 | SEQ-ID-No.57 | SEQ-ID-No.58 | Soy or corn |
| T3-30 | SEQ-ID-No.59 | SEQ-ID-No.60 | Soy or corn |
| T3-31 | SEQ-ID-No.61 | SEQ-ID-No.62 | Soy or corn |
| T3-32 | SEQ-ID-No.63 | SEQ-ID-No.64 | Soy or corn |

(continued)

| No | DNA | protein | plant |
|---|---|---|---|
| T3-33 | SEQ-ID-No.65 | SEQ-ID-No.66 | Soy or corn |
| T3-34 | SEQ-ID-No.67 | SEQ-ID-No.68 | Soy or corn |
| T3-35 | SEQ-ID-No.69 | SEQ-ID-No.70 | Soy or corn |
| T3-36 | SEQ-ID-No.71 | SEQ-ID-No.72 | Soy or corn |
| T3-37 | SEQ-ID-No.73 | SEQ-ID-No.74 | Soy or corn |
| T3-38 | SEQ-ID-No.75 | SEQ-ID-No.76 | Soy or corn |
| T3-39 | SEQ-ID-No.77 | SEQ-ID-No.78 | Soy or corn |
| T3-40 | SEQ-ID-No.79 | SEQ-ID-No.80 | Soy or corn |
| T3-41 | SEQ-ID-No.81 | SEQ-ID-No.82 | Soy or corn |
| T3-42 | SEQ-ID-No.83 | SEQ-ID-No.84 | Soy or corn |
| T3-43 | SEQ-ID-No.85 | SEQ-ID-No.86 | Soy or corn |
| T3-44 | SEQ-ID-No.87 | SEQ-ID-No.88 | Soy or corn |
| T3-45 | SEQ-ID-No.89 | SEQ-ID-No.90 | Soy or corn |
| T3-46 | SEQ-ID-No.91 | SEQ-ID-No.92 | Soy or corn |
| T3-47 | SEQ-ID-No.93 | SEQ-ID-No.94 | Soy or corn |
| T3-48 | SEQ-ID-No.95 | SEQ-ID-No.96 | Soy or corn |
| T3-49 | SEQ-ID-No.97 | SEQ-ID-No.98 | Soy or corn |
| T3-50 | SEQ-ID-No.99 | SEQ-ID-No.100 | Soy or corn |

Tabelle T4:

| No. | Fungicide |
|---|---|
| T4-1 | Pyraclostrobin |
| T4-2 | Azoxystrobin |
| T4-3 | Trifloxystrobin |
| T4-4 | Picoxystrobin |
| T4-5 | Fluoxastrobin |
| T4-6 | Fluxapyroxad, |
| T4-7 | Carboxin, |
| T4-8 | epoxiconazole, |
| T4-9 | metconazole, |
| T4-10 | prothioconazole |
| T4-11 | Difenoconazole |
| T4-12 | propiconazole, |
| T4-13 | tebuconazole |
| T4-14 | cyproconazole |
| T4-15 | flutriafol |
| T4-16 | myclobutanil |

(continued)

| No. | Fungicide |
|---|---|
| T4-17 | Diniconazole |
| T4-18 | ipconazole, |
| T4-19 | triadimefon, |
| T4-20 | captan |
| T4-21 | Metalaxyl |
| T4-22 | Carbendazim |
| T4-23 | Thiabendazole |
| T4-24 | Fludioxonil |
| T4-25 | mancozeb |
| T4-26 | thiram |
| T4-27 | (Z,2E)-5-[1-(2,4-dichlorophenyl)pyrazol-3-yl]-oxy-2-methoxyimino-N,3-dimethyl-pent-3-enamide |
| T4-28 | (Z,2E)-5-[1-(4-chlorophenyl)pyrazol-3-yl]oxy-2-methoxyimino-N,3-dimethyl-pent-3-enamide |
| T4-29 | 1-[3-chloro-2-[[1-(4-chlorophenyl)-1 H-pyrazol-3-yl]oxymethyl]phenyl]-4-methyl-tetrazol-5-one |
| T4-30 | 1-[3-bromo-2-[[1-(4-chlorophenyl)pyrazol-3-yl]oxymethyl]phenyl]-4-methyl-tetrazol-5-one |
| T4-31 | 1-[2-[[1-(4-chlorophenyl)pyrazol-3-yl]oxymethyl]-3-methyl-phenyl]-4-methyl-tetrazol-5-one |
| T4-32 | 1-[2-[[1-(4-chlorophenyl)pyrazol-3-yl]oxymethyl]-3-fluoro-phenyl]-4-methyl-tetrazol-5-one |
| T4-33 | 1-[2-[[1-(2,4-dichlorophenyl)pyrazol-3-yl]oxymethyl]-3-fluoro-phenyl]-4-methyl-tetrazol-5-one |
| T4-34 | 1-[2-[[4-(4-chlorophenyl)thiazol-2-yl]oxymethyl]-3-methyl-phenyl]-4-methyl-tetrazol-5-one |
| T4-35 | 1-[3-chloro-2-[[4-(p-tolyl)thiazol-2-yl]oxymethyl]phenyl]-4-methyl-tetrazol-5-one |
| T4-36 | 1-[3-cyclopropyl-2-[[2-methyl-4-(1-methylpyrazol-3-yl)phenoxy]methyl]phenyl]-4 methyl-tetrazol-5-one |
| T4-37 | 1-[3-(difluoromethoxy)-2-[[2-methyl-4-(1 methylpyrazol-3 yl)phenoxy]methyl]phenyl]-4-methyl-tetrazol-5-one |
| T4-38 | 1-methyl-4-[3-methyl-2 [[2 methyl-4-(1-methylpyrazol-3-yl)phenoxy]methyl]phenyl]tetrazol-5-one |
| T4-39 | 1-methyl-4-[3-methyl-2-[[1-[3-(trifluoromethyl)phenyl]-ethylideneamino]oxymethyl]phenyl]tetrazol-5 one |
| T4-40 | N-[9-(dichloromethylene)-1,2,3,4-tetrahydro-1,4-me¬thanonaphthalen-5-yl]-3-(difluoromethyl)-1-methyl-1 H-pyrazole-4-carboxamide |
| T4-41 | Sedaxane |
| T4-42 | Penflufen |
| T4-43 | 3-(difluoromethyl)-1-methyl-N-(1,1,3-trimethylindan-4-yl)pyrazole-4-carboxamide |
| T4-44 | 3-(trifluoromethyl)-1-methyl-N-(1,1,3-trimethylindan-4-yl)pyrazole-4-carboxamide |
| T4-45 | 1,3-dimethyl-N-(1,1,3-trimethylindan-4-yl)pyrazole-4-carboxamide |
| T4-46 | 3-(trifluoromethyl)-1,5-dimethyl-N-(1,1,3-trimethylindan-4-yl)pyrazole-4-carboxamide |
| T4-47 | 3-(difluoromethyl)-1,5-dimethyl-N-(1,1,3-trimethylindan-4-yl)pyrazole-4-carboxamide |
| T4-48 | 1,3,5-trimethyl-N-(1,1,3-trimethylindan-4-yl)pyrazole-4-carboxamide |
| T4-49 | Fluazinam |
| T4-50 | Fentin salts |
| T4-51 | 1-[rel-(2S;3R)-3-(2-chlorophenyl)-2-(2,4-difluorophenyl)-oxiranylmethyl]-5 thiocyanato-1 H-[1,2,4]triazole |
| T4-52 | 2-[rel-(2S;3R)-3-(2-chlorophenyl)-2-(2,4-difluorophenyl)-oxiranylmethyl]-2H [1,2,4]triazole-3-thiol |

(continued)

| No. | Fungicide |
|---|---|
| T4-53 | 2-[2-chloro-4-(4-chlorophenoxy)phenyl]-1(1,2,4-triazol-1-yl)pentan-2-ol |
| T4-54 | 1-[4-(4-chlorophenoxy)-2-(trifluoromethyl)phenyl]-1 cyclopropyl-2-(1,2,4-triazol-1-yl)ethanol |
| T4-55 | 2-[4-(4-chlorophenoxy)-2-(trifluoromethyl)phenyl]-1-(1,2,4-triazol-1-yl)butan-2-ol |
| T4-56 | 2-[2-chloro-4-(4-chlorophenoxy)phenyl]-1-(1,2,4-triazol-1-yl)butan-2-ol |
| T4-57 | 2-[4-(4-chlorophenoxy)-2-(trifluoromethyl)phenyl]-3-methyl-1-(1,2,4-triazol-1-yl)butan-2-ol |
| T4-58 | 2-[4-(4-chlorophenoxy)-2-(trifluoromethyl)phenyl]-1-(1,2,4-triazol-1-yl)propan-2-ol |
| T4-59 | 2-[2-chloro-4-(4-chlorophenoxy)phenyl]-3-methyl-1-(1,2,4-triazol-1-yl)butan-2-ol |
| T4-60 | 2-[4-(4-chlorophenoxy)-2-(trifluoromethyl)phenyl]-1-(1,2,4-triazol-1-yl)pentan-2-ol |
| T4-61 | -[4-(4-fluorophenoxy)-2-(trifluoromethyl)phenyl]-1-(1,2,4-triazol-1-yl)propan-2-ol |
| T4-62 | 2, 2-[2-chloro-4-(4-chlorophenoxy)phenyl]-1-(1,2,4-triazol-1-yl)pent-3-yn-2-ol |
| T4-63 | Thiophanate-methyl |
| T4-64 | Chlorothalonil |

In a preferred embodiment, the present invention relates to a method of controlling fungal infection and/or increasing the health of transgenic soy or corn plants, wherein
preferred combinations of exogenous DNA and/or proteins as defined in Table 3 and fungicides as defined in Table 4 are compiled in Table T5, wherein each row corresponds to one embodiment according to the present invention.

Table 5:

| T3_No | T4_No |
|---|---|
| T3-1 | T4-1 |
| T3-1 | T4-10 |
| T3-1 | T4-11 |
| T3-1 | T4-12 |
| T3-1 | T4-13 |
| T3-1 | T4-14 |
| T3-1 | T4-15 |
| T3-1 | T4-16 |
| T3-1 | T4-17 |
| T3-1 | T4-18 |
| T3-1 | T4-19 |
| T3-1 | T4-2 |
| T3-1 | T4-20 |
| T3-1 | T4-21 |
| T3-1 | T4-22 |
| T3-1 | T4-23 |
| T3-1 | T4-24 |
| T3-1 | T4-25 |
| T3-1 | T4-26 |
| T3-1 | T4-27 |
| T3-1 | T4-28 |
| T3-1 | T4-29 |
| T3-1 | T4-3 |
| T3-1 | T4-30 |
| T3-1 | T4-31 |
| T3-1 | T4-32 |
| T3-1 | T4-33 |
| T3-1 | T4-34 |
| T3-1 | T4-35 |
| T3-1 | T4-36 |
| T3-1 | T4-37 |
| T3-1 | T4-38 |
| T3-1 | T4-39 |
| T3-1 | T4-4 |
| T3-1 | T4-40 |
| T3-1 | T4-41 |
| T3-1 | T4-42 |
| T3-1 | T4-43 |
| T3-1 | T4-44 |

| T3-1 | T4-45 |
|---|---|
| T3-1 | T4-46 |
| T3-1 | T4-47 |
| T3-1 | T4-48 |
| T3-1 | T4-49 |
| T3-1 | T4-5 |
| T3-1 | T4-50 |
| T3-1 | T4-51 |
| T3-1 | T4-52 |
| T3-1 | T4-53 |
| T3-1 | T4-54 |
| T3-1 | T4-55 |
| T3-1 | T4-56 |
| T3-1 | T4-57 |
| T3-1 | T4-58 |
| T3-1 | T4-59 |
| T3-1 | T4-6 |
| T3-1 | T4-60 |
| T3-1 | T4-61 |
| T3-1 | T4-62 |
| T3-1 | T4-63 |
| T3-1 | T4-64 |
| T3-1 | T4-7 |
| T3-1 | T4-8 |
| T3-1 | T4-9 |
| T3-10 | T4-1 |
| T3-10 | T4-10 |
| T3-10 | T4-11 |
| T3-10 | T4-12 |
| T3-10 | T4-13 |
| T3-10 | T4-14 |
| T3-10 | T4-15 |
| T3-10 | T4-16 |
| T3-10 | T4-17 |
| T3-10 | T4-18 |
| T3-10 | T4-19 |
| T3-10 | T4-2 |
| T3-10 | T4-20 |
| T3-10 | T4-21 |
| T3-10 | T4-22 |
| T3-10 | T4-23 |
| T3-10 | T4-24 |
| T3-10 | T4-25 |
| T3-10 | T4-26 |
| T3-10 | T4-27 |

| T3-10 | T4-28 |
|---|---|
| T3-10 | T4-29 |
| T3-10 | T4-3 |
| T3-10 | T4-30 |
| T3-10 | T4-31 |
| T3-10 | T4-32 |
| T3-10 | T4-33 |
| T3-10 | T4-34 |
| T3-10 | T4-35 |
| T3-10 | T4-36 |
| T3-10 | T4-37 |
| T3-10 | T4-38 |
| T3-10 | T4-39 |
| T3-10 | T4-4 |
| T3-10 | T4-40 |
| T3-10 | T4-41 |
| T3-10 | T4-42 |
| T3-10 | T4-43 |
| T3-10 | T4-44 |
| T3-10 | T4-45 |
| T3-10 | T4-46 |
| T3-10 | T4-47 |
| T3-10 | T4-48 |
| T3-10 | T4-49 |
| T3-10 | T4-5 |
| T3-10 | T4-50 |
| T3-10 | T4-51 |
| T3-10 | T4-52 |
| T3-10 | T4-53 |
| T3-10 | T4-54 |
| T3-10 | T4-55 |
| T3-10 | T4-56 |
| T3-10 | T4-57 |
| T3-10 | T4-58 |
| T3-10 | T4-59 |
| T3-10 | T4-6 |
| T3-10 | T4-60 |
| T3-10 | T4-61 |
| T3-10 | T4-62 |
| T3-10 | T4-63 |
| T3-10 | T4-64 |
| T3-10 | T4-7 |
| T3-10 | T4-8 |
| T3-10 | T4-9 |
| T3-11 | T4-1 |

| T3-11 | T4-10 |
|---|---|
| T3-11 | T4-11 |
| T3-11 | T4-12 |
| T3-11 | T4-13 |
| T3-11 | T4-14 |
| T3-11 | T4-15 |
| T3-11 | T4-16 |
| T3-11 | T4-17 |
| T3-11 | T4-18 |
| T3-11 | T4-19 |
| T3-11 | T4-2 |
| T3-11 | T4-20 |
| T3-11 | T4-21 |
| T3-11 | T4-22 |
| T3-11 | T4-23 |
| T3-11 | T4-24 |
| T3-11 | T4-25 |
| T3-11 | T4-26 |
| T3-11 | T4-27 |
| T3-11 | T4-28 |
| T3-11 | T4-29 |
| T3-11 | T4-3 |
| T3-11 | T4-30 |
| T3-11 | T4-31 |
| T3-11 | T4-32 |
| T3-11 | T4-33 |
| T3-11 | T4-34 |
| T3-11 | T4-35 |
| T3-11 | T4-36 |
| T3-11 | T4-37 |
| T3-11 | T4-38 |
| T3-11 | T4-39 |
| T3-11 | T4-4 |
| T3-11 | T4-40 |
| T3-11 | T4-41 |
| T3-11 | T4-42 |
| T3-11 | T4-43 |
| T3-11 | T4-44 |
| T3-11 | T4-45 |
| T3-11 | T4-46 |
| T3-11 | T4-47 |
| T3-11 | T4-48 |
| T3-11 | T4-49 |
| T3-11 | T4-5 |
| T3-11 | T4-50 |

| T3-11 | T4-51 |
|---|---|
| T3-11 | T4-52 |
| T3-11 | T4-53 |
| T3-11 | T4-54 |
| T3-11 | T4-55 |
| T3-11 | T4-56 |
| T3-11 | T4-57 |
| T3-11 | T4-58 |
| T3-11 | T4-59 |
| T3-11 | T4-6 |
| T3-11 | T4-60 |
| T3-11 | T4-61 |
| T3-11 | T4-62 |
| T3-11 | T4-63 |
| T3-11 | T4-64 |
| T3-11 | T4-7 |
| T3-11 | T4-8 |
| T3-11 | T4-9 |
| T3-12 | T4-1 |
| T3-12 | T4-10 |
| T3-12 | T4-11 |
| T3-12 | T4-12 |
| T3-12 | T4-13 |
| T3-12 | T4-14 |
| T3-12 | T4-15 |
| T3-12 | T4-16 |
| T3-12 | T4-17 |
| T3-12 | T4-18 |
| T3-12 | T4-19 |
| T3-12 | T4-2 |
| T3-12 | T4-20 |
| T3-12 | T4-21 |
| T3-12 | T4-22 |
| T3-12 | T4-23 |
| T3-12 | T4-24 |
| T3-12 | T4-25 |
| T3-12 | T4-26 |
| T3-12 | T4-27 |
| T3-12 | T4-28 |
| T3-12 | T4-29 |
| T3-12 | T4-3 |
| T3-12 | T4-30 |
| T3-12 | T4-31 |
| T3-12 | T4-32 |
| T3-12 | T4-33 |

| | |
|---|---|
| T3-12 | T4-34 |
| T3-12 | T4-35 |
| T3-12 | T4-36 |
| T3-12 | T4-37 |
| T3-12 | T4-38 |
| T3-12 | T4-39 |
| T3-12 | T4-4 |
| T3-12 | T4-40 |
| T3-12 | T4-41 |
| T3-12 | T4-42 |
| T3-12 | T4-43 |
| T3-12 | T4-44 |
| T3-12 | T4-45 |
| T3-12 | T4-46 |
| T3-12 | T4-47 |
| T3-12 | T4-48 |
| T3-12 | T4-49 |
| T3-12 | T4-5 |
| T3-12 | T4-50 |
| T3-12 | T4-51 |
| T3-12 | T4-52 |
| T3-12 | T4-53 |
| T3-12 | T4-54 |
| T3-12 | T4-55 |
| T3-12 | T4-56 |
| T3-12 | T4-57 |
| T3-12 | T4-58 |
| T3-12 | T4-59 |
| T3-12 | T4-6 |
| T3-12 | T4-60 |
| T3-12 | T4-61 |
| T3-12 | T4-62 |
| T3-12 | T4-63 |
| T3-12 | T4-64 |
| T3-12 | T4-7 |
| T3-12 | T4-8 |
| T3-12 | T4-9 |
| T3-13 | T4-1 |
| T3-13 | T4-10 |
| T3-13 | T4-11 |
| T3-13 | T4-12 |
| T3-13 | T4-13 |
| T3-13 | T4-14 |
| T3-13 | T4-15 |
| T3-13 | T4-16 |

| | |
|---|---|
| T3-13 | T4-17 |
| T3-13 | T4-18 |
| T3-13 | T4-19 |
| T3-13 | T4-2 |
| T3-13 | T4-20 |
| T3-13 | T4-21 |
| T3-13 | T4-22 |
| T3-13 | T4-23 |
| T3-13 | T4-24 |
| T3-13 | T4-25 |
| T3-13 | T4-26 |
| T3-13 | T4-27 |
| T3-13 | T4-28 |
| T3-13 | T4-29 |
| T3-13 | T4-3 |
| T3-13 | T4-30 |
| T3-13 | T4-31 |
| T3-13 | T4-32 |
| T3-13 | T4-33 |
| T3-13 | T4-34 |
| T3-13 | T4-35 |
| T3-13 | T4-36 |
| T3-13 | T4-37 |
| T3-13 | T4-38 |
| T3-13 | T4-39 |
| T3-13 | T4-4 |
| T3-13 | T4-40 |
| T3-13 | T4-41 |
| T3-13 | T4-42 |
| T3-13 | T4-43 |
| T3-13 | T4-44 |
| T3-13 | T4-45 |
| T3-13 | T4-46 |
| T3-13 | T4-47 |
| T3-13 | T4-48 |
| T3-13 | T4-49 |
| T3-13 | T4-5 |
| T3-13 | T4-50 |
| T3-13 | T4-51 |
| T3-13 | T4-52 |
| T3-13 | T4-53 |
| T3-13 | T4-54 |
| T3-13 | T4-55 |
| T3-13 | T4-56 |
| T3-13 | T4-57 |

| | |
|---|---|
| T3-13 | T4-58 |
| T3-13 | T4-59 |
| T3-13 | T4-6 |
| T3-13 | T4-60 |
| T3-13 | T4-61 |
| T3-13 | T4-62 |
| T3-13 | T4-63 |
| T3-13 | T4-64 |
| T3-13 | T4-7 |
| T3-13 | T4-8 |
| T3-13 | T4-9 |
| T3-14 | T4-1 |
| T3-14 | T4-10 |
| T3-14 | T4-11 |
| T3-14 | T4-12 |
| T3-14 | T4-13 |
| T3-14 | T4-14 |
| T3-14 | T4-15 |
| T3-14 | T4-16 |
| T3-14 | T4-17 |
| T3-14 | T4-18 |
| T3-14 | T4-19 |
| T3-14 | T4-2 |
| T3-14 | T4-20 |
| T3-14 | T4-21 |
| T3-14 | T4-22 |
| T3-14 | T4-23 |
| T3-14 | T4-24 |
| T3-14 | T4-25 |
| T3-14 | T4-26 |
| T3-14 | T4-27 |
| T3-14 | T4-28 |
| T3-14 | T4-29 |
| T3-14 | T4-3 |
| T3-14 | T4-30 |
| T3-14 | T4-31 |
| T3-14 | T4-32 |
| T3-14 | T4-33 |
| T3-14 | T4-34 |
| T3-14 | T4-35 |
| T3-14 | T4-36 |
| T3-14 | T4-37 |
| T3-14 | T4-38 |
| T3-14 | T4-39 |
| T3-14 | T4-4 |

| | |
|---|---|
| T3-14 | T4-40 |
| T3-14 | T4-41 |
| T3-14 | T4-42 |
| T3-14 | T4-43 |
| T3-14 | T4-44 |
| T3-14 | T4-45 |
| T3-14 | T4-46 |
| T3-14 | T4-47 |
| T3-14 | T4-48 |
| T3-14 | T4-49 |
| T3-14 | T4-5 |
| T3-14 | T4-50 |
| T3-14 | T4-51 |
| T3-14 | T4-52 |
| T3-14 | T4-53 |
| T3-14 | T4-54 |
| T3-14 | T4-55 |
| T3-14 | T4-56 |
| T3-14 | T4-57 |
| T3-14 | T4-58 |
| T3-14 | T4-59 |
| T3-14 | T4-6 |
| T3-14 | T4-60 |
| T3-14 | T4-61 |
| T3-14 | T4-62 |
| T3-14 | T4-63 |
| T3-14 | T4-64 |
| T3-14 | T4-7 |
| T3-14 | T4-8 |
| T3-14 | T4-9 |
| T3-15 | T4-1 |
| T3-15 | T4-10 |
| T3-15 | T4-11 |
| T3-15 | T4-12 |
| T3-15 | T4-13 |
| T3-15 | T4-14 |
| T3-15 | T4-15 |
| T3-15 | T4-16 |
| T3-15 | T4-17 |
| T3-15 | T4-18 |
| T3-15 | T4-19 |
| T3-15 | T4-2 |
| T3-15 | T4-20 |
| T3-15 | T4-21 |
| T3-15 | T4-22 |

| | |
|---|---|
| T3-15 | T4-23 |
| T3-15 | T4-24 |
| T3-15 | T4-25 |
| T3-15 | T4-26 |
| T3-15 | T4-27 |
| T3-15 | T4-28 |
| T3-15 | T4-29 |
| T3-15 | T4-3 |
| T3-15 | T4-30 |
| T3-15 | T4-31 |
| T3-15 | T4-32 |
| T3-15 | T4-33 |
| T3-15 | T4-34 |
| T3-15 | T4-35 |
| T3-15 | T4-36 |
| T3-15 | T4-37 |
| T3-15 | T4-38 |
| T3-15 | T4-39 |
| T3-15 | T4-4 |
| T3-15 | T4-40 |
| T3-15 | T4-41 |
| T3-15 | T4-42 |
| T3-15 | T4-43 |
| T3-15 | T4-44 |
| T3-15 | T4-45 |
| T3-15 | T4-46 |
| T3-15 | T4-47 |
| T3-15 | T4-48 |
| T3-15 | T4-49 |
| T3-15 | T4-5 |
| T3-15 | T4-50 |
| T3-15 | T4-51 |
| T3-15 | T4-52 |
| T3-15 | T4-53 |
| T3-15 | T4-54 |
| T3-15 | T4-55 |
| T3-15 | T4-56 |
| T3-15 | T4-57 |
| T3-15 | T4-58 |
| T3-15 | T4-59 |
| T3-15 | T4-6 |
| T3-15 | T4-60 |
| T3-15 | T4-61 |
| T3-15 | T4-62 |
| T3-15 | T4-63 |

| | |
|---|---|
| T3-15 | T4-64 |
| T3-15 | T4-7 |
| T3-15 | T4-8 |
| T3-15 | T4-9 |
| T3-16 | T4-1 |
| T3-16 | T4-10 |
| T3-16 | T4-11 |
| T3-16 | T4-12 |
| T3-16 | T4-13 |
| T3-16 | T4-14 |
| T3-16 | T4-15 |
| T3-16 | T4-16 |
| T3-16 | T4-17 |
| T3-16 | T4-18 |
| T3-16 | T4-19 |
| T3-16 | T4-2 |
| T3-16 | T4-20 |
| T3-16 | T4-21 |
| T3-16 | T4-22 |
| T3-16 | T4-23 |
| T3-16 | T4-24 |
| T3-16 | T4-25 |
| T3-16 | T4-26 |
| T3-16 | T4-27 |
| T3-16 | T4-28 |
| T3-16 | T4-29 |
| T3-16 | T4-3 |
| T3-16 | T4-30 |
| T3-16 | T4-31 |
| T3-16 | T4-32 |
| T3-16 | T4-33 |
| T3-16 | T4-34 |
| T3-16 | T4-35 |
| T3-16 | T4-36 |
| T3-16 | T4-37 |
| T3-16 | T4-38 |
| T3-16 | T4-39 |
| T3-16 | T4-4 |
| T3-16 | T4-40 |
| T3-16 | T4-41 |
| T3-16 | T4-42 |
| T3-16 | T4-43 |
| T3-16 | T4-44 |
| T3-16 | T4-45 |
| T3-16 | T4-46 |

| | |
|---|---|
| T3-16 | T4-47 |
| T3-16 | T4-48 |
| T3-16 | T4-49 |
| T3-16 | T4-5 |
| T3-16 | T4-50 |
| T3-16 | T4-51 |
| T3-16 | T4-52 |
| T3-16 | T4-53 |
| T3-16 | T4-54 |
| T3-16 | T4-55 |
| T3-16 | T4-56 |
| T3-16 | T4-57 |
| T3-16 | T4-58 |
| T3-16 | T4-59 |
| T3-16 | T4-6 |
| T3-16 | T4-60 |
| T3-16 | T4-61 |
| T3-16 | T4-62 |
| T3-16 | T4-63 |
| T3-16 | T4-64 |
| T3-16 | T4-7 |
| T3-16 | T4-8 |
| T3-16 | T4-9 |
| T3-17 | T4-1 |
| T3-17 | T4-10 |
| T3-17 | T4-11 |
| T3-17 | T4-12 |
| T3-17 | T4-13 |
| T3-17 | T4-14 |
| T3-17 | T4-15 |
| T3-17 | T4-16 |
| T3-17 | T4-17 |
| T3-17 | T4-18 |
| T3-17 | T4-19 |
| T3-17 | T4-2 |
| T3-17 | T4-20 |
| T3-17 | T4-21 |
| T3-17 | T4-22 |
| T3-17 | T4-23 |
| T3-17 | T4-24 |
| T3-17 | T4-25 |
| T3-17 | T4-26 |
| T3-17 | T4-27 |
| T3-17 | T4-28 |
| T3-17 | T4-29 |

| | |
|---|---|
| T3-17 | T4-3 |
| T3-17 | T4-30 |
| T3-17 | T4-31 |
| T3-17 | T4-32 |
| T3-17 | T4-33 |
| T3-17 | T4-34 |
| T3-17 | T4-35 |
| T3-17 | T4-36 |
| T3-17 | T4-37 |
| T3-17 | T4-38 |
| T3-17 | T4-39 |
| T3-17 | T4-4 |
| T3-17 | T4-40 |
| T3-17 | T4-41 |
| T3-17 | T4-42 |
| T3-17 | T4-43 |
| T3-17 | T4-44 |
| T3-17 | T4-45 |
| T3-17 | T4-46 |
| T3-17 | T4-47 |
| T3-17 | T4-48 |
| T3-17 | T4-49 |
| T3-17 | T4-5 |
| T3-17 | T4-50 |
| T3-17 | T4-51 |
| T3-17 | T4-52 |
| T3-17 | T4-53 |
| T3-17 | T4-54 |
| T3-17 | T4-55 |
| T3-17 | T4-56 |
| T3-17 | T4-57 |
| T3-17 | T4-58 |
| T3-17 | T4-59 |
| T3-17 | T4-6 |
| T3-17 | T4-60 |
| T3-17 | T4-61 |
| T3-17 | T4-62 |
| T3-17 | T4-63 |
| T3-17 | T4-64 |
| T3-17 | T4-7 |
| T3-17 | T4-8 |
| T3-17 | T4-9 |
| T3-18 | T4-1 |
| T3-18 | T4-10 |
| T3-18 | T4-11 |

| T3-18 | T4-12 |
|---|---|
| T3-18 | T4-13 |
| T3-18 | T4-14 |
| T3-18 | T4-15 |
| T3-18 | T4-16 |
| T3-18 | T4-17 |
| T3-18 | T4-18 |
| T3-18 | T4-19 |
| T3-18 | T4-2 |
| T3-18 | T4-20 |
| T3-18 | T4-21 |
| T3-18 | T4-22 |
| T3-18 | T4-23 |
| T3-18 | T4-24 |
| T3-18 | T4-25 |
| T3-18 | T4-26 |
| T3-18 | T4-27 |
| T3-18 | T4-28 |
| T3-18 | T4-29 |
| T3-18 | T4-3 |
| T3-18 | T4-30 |
| T3-18 | T4-31 |
| T3-18 | T4-32 |
| T3-18 | T4-33 |
| T3-18 | T4-34 |
| T3-18 | T4-35 |
| T3-18 | T4-36 |
| T3-18 | T4-37 |
| T3-18 | T4-38 |
| T3-18 | T4-39 |
| T3-18 | T4-4 |
| T3-18 | T4-40 |
| T3-18 | T4-41 |
| T3-18 | T4-42 |
| T3-18 | T4-43 |
| T3-18 | T4-44 |
| T3-18 | T4-45 |
| T3-18 | T4-46 |
| T3-18 | T4-47 |
| T3-18 | T4-48 |
| T3-18 | T4-49 |
| T3-18 | T4-5 |
| T3-18 | T4-50 |
| T3-18 | T4-51 |
| T3-18 | T4-52 |

| T3-18 | T4-53 |
|---|---|
| T3-18 | T4-54 |
| T3-18 | T4-55 |
| T3-18 | T4-56 |
| T3-18 | T4-57 |
| T3-18 | T4-58 |
| T3-18 | T4-59 |
| T3-18 | T4-6 |
| T3-18 | T4-60 |
| T3-18 | T4-61 |
| T3-18 | T4-62 |
| T3-18 | T4-63 |
| T3-18 | T4-64 |
| T3-18 | T4-7 |
| T3-18 | T4-8 |
| T3-18 | T4-9 |
| T3-19 | T4-1 |
| T3-19 | T4-10 |
| T3-19 | T4-11 |
| T3-19 | T4-12 |
| T3-19 | T4-13 |
| T3-19 | T4-14 |
| T3-19 | T4-15 |
| T3-19 | T4-16 |
| T3-19 | T4-17 |
| T3-19 | T4-18 |
| T3-19 | T4-19 |
| T3-19 | T4-2 |
| T3-19 | T4-20 |
| T3-19 | T4-21 |
| T3-19 | T4-22 |
| T3-19 | T4-23 |
| T3-19 | T4-24 |
| T3-19 | T4-25 |
| T3-19 | T4-26 |
| T3-19 | T4-27 |
| T3-19 | T4-28 |
| T3-19 | T4-29 |
| T3-19 | T4-3 |
| T3-19 | T4-30 |
| T3-19 | T4-31 |
| T3-19 | T4-32 |
| T3-19 | T4-33 |
| T3-19 | T4-34 |
| T3-19 | T4-35 |

| T3-19 | T4-36 |
|---|---|
| T3-19 | T4-37 |
| T3-19 | T4-38 |
| T3-19 | T4-39 |
| T3-19 | T4-4 |
| T3-19 | T4-40 |
| T3-19 | T4-41 |
| T3-19 | T4-42 |
| T3-19 | T4-43 |
| T3-19 | T4-44 |
| T3-19 | T4-45 |
| T3-19 | T4-46 |
| T3-19 | T4-47 |
| T3-19 | T4-48 |
| T3-19 | T4-49 |
| T3-19 | T4-5 |
| T3-19 | T4-50 |
| T3-19 | T4-51 |
| T3-19 | T4-52 |
| T3-19 | T4-53 |
| T3-19 | T4-54 |
| T3-19 | T4-55 |
| T3-19 | T4-56 |
| T3-19 | T4-57 |
| T3-19 | T4-58 |
| T3-19 | T4-59 |
| T3-19 | T4-6 |
| T3-19 | T4-60 |
| T3-19 | T4-61 |
| T3-19 | T4-62 |
| T3-19 | T4-63 |
| T3-19 | T4-64 |
| T3-19 | T4-7 |
| T3-19 | T4-8 |
| T3-19 | T4-9 |
| T3-2 | T4-1 |
| T3-2 | T4-10 |
| T3-2 | T4-11 |
| T3-2 | T4-12 |
| T3-2 | T4-13 |
| T3-2 | T4-14 |
| T3-2 | T4-15 |
| T3-2 | T4-16 |
| T3-2 | T4-17 |
| T3-2 | T4-18 |

| T3-2 | T4-19 |
|---|---|
| T3-2 | T4-2 |
| T3-2 | T4-20 |
| T3-2 | T4-21 |
| T3-2 | T4-22 |
| T3-2 | T4-23 |
| T3-2 | T4-24 |
| T3-2 | T4-25 |
| T3-2 | T4-26 |
| T3-2 | T4-27 |
| T3-2 | T4-28 |
| T3-2 | T4-29 |
| T3-2 | T4-3 |
| T3-2 | T4-30 |
| T3-2 | T4-31 |
| T3-2 | T4-32 |
| T3-2 | T4-33 |
| T3-2 | T4-34 |
| T3-2 | T4-35 |
| T3-2 | T4-36 |
| T3-2 | T4-37 |
| T3-2 | T4-38 |
| T3-2 | T4-39 |
| T3-2 | T4-4 |
| T3-2 | T4-40 |
| T3-2 | T4-41 |
| T3-2 | T4-42 |
| T3-2 | T4-43 |
| T3-2 | T4-44 |
| T3-2 | T4-45 |
| T3-2 | T4-46 |
| T3-2 | T4-47 |
| T3-2 | T4-48 |
| T3-2 | T4-49 |
| T3-2 | T4-5 |
| T3-2 | T4-50 |
| T3-2 | T4-51 |
| T3-2 | T4-52 |
| T3-2 | T4-53 |
| T3-2 | T4-54 |
| T3-2 | T4-55 |
| T3-2 | T4-56 |
| T3-2 | T4-57 |
| T3-2 | T4-58 |
| T3-2 | T4-59 |

| | |
|---|---|
| T3-2 | T4-6 |
| T3-2 | T4-60 |
| T3-2 | T4-61 |
| T3-2 | T4-62 |
| T3-2 | T4-63 |
| T3-2 | T4-64 |
| T3-2 | T4-7 |
| T3-2 | T4-8 |
| T3-2 | T4-9 |
| T3-20 | T4-1 |
| T3-20 | T4-10 |
| T3-20 | T4-11 |
| T3-20 | T4-12 |
| T3-20 | T4-13 |
| T3-20 | T4-14 |
| T3-20 | T4-15 |
| T3-20 | T4-16 |
| T3-20 | T4-17 |
| T3-20 | T4-18 |
| T3-20 | T4-19 |
| T3-20 | T4-2 |
| T3-20 | T4-20 |
| T3-20 | T4-21 |
| T3-20 | T4-22 |
| T3-20 | T4-23 |
| T3-20 | T4-24 |
| T3-20 | T4-25 |
| T3-20 | T4-26 |
| T3-20 | T4-27 |
| T3-20 | T4-28 |
| T3-20 | T4-29 |
| T3-20 | T4-3 |
| T3-20 | T4-30 |
| T3-20 | T4-31 |
| T3-20 | T4-32 |
| T3-20 | T4-33 |
| T3-20 | T4-34 |
| T3-20 | T4-35 |
| T3-20 | T4-36 |
| T3-20 | T4-37 |
| T3-20 | T4-38 |
| T3-20 | T4-39 |
| T3-20 | T4-4 |
| T3-20 | T4-40 |
| T3-20 | T4-41 |

| | |
|---|---|
| T3-20 | T4-42 |
| T3-20 | T4-43 |
| T3-20 | T4-44 |
| T3-20 | T4-45 |
| T3-20 | T4-46 |
| T3-20 | T4-47 |
| T3-20 | T4-48 |
| T3-20 | T4-49 |
| T3-20 | T4-5 |
| T3-20 | T4-50 |
| T3-20 | T4-51 |
| T3-20 | T4-52 |
| T3-20 | T4-53 |
| T3-20 | T4-54 |
| T3-20 | T4-55 |
| T3-20 | T4-56 |
| T3-20 | T4-57 |
| T3-20 | T4-58 |
| T3-20 | T4-59 |
| T3-20 | T4-6 |
| T3-20 | T4-60 |
| T3-20 | T4-61 |
| T3-20 | T4-62 |
| T3-20 | T4-63 |
| T3-20 | T4-64 |
| T3-20 | T4-7 |
| T3-20 | T4-8 |
| T3-20 | T4-9 |
| T3-21 | T4-1 |
| T3-21 | T4-10 |
| T3-21 | T4-11 |
| T3-21 | T4-12 |
| T3-21 | T4-13 |
| T3-21 | T4-14 |
| T3-21 | T4-15 |
| T3-21 | T4-16 |
| T3-21 | T4-17 |
| T3-21 | T4-18 |
| T3-21 | T4-19 |
| T3-21 | T4-2 |
| T3-21 | T4-20 |
| T3-21 | T4-21 |
| T3-21 | T4-22 |
| T3-21 | T4-23 |
| T3-21 | T4-24 |

| | |
|---|---|
| T3-21 | T4-25 |
| T3-21 | T4-26 |
| T3-21 | T4-27 |
| T3-21 | T4-28 |
| T3-21 | T4-29 |
| T3-21 | T4-3 |
| T3-21 | T4-30 |
| T3-21 | T4-31 |
| T3-21 | T4-32 |
| T3-21 | T4-33 |
| T3-21 | T4-34 |
| T3-21 | T4-35 |
| T3-21 | T4-36 |
| T3-21 | T4-37 |
| T3-21 | T4-38 |
| T3-21 | T4-39 |
| T3-21 | T4-4 |
| T3-21 | T4-40 |
| T3-21 | T4-41 |
| T3-21 | T4-42 |
| T3-21 | T4-43 |
| T3-21 | T4-44 |
| T3-21 | T4-45 |
| T3-21 | T4-46 |
| T3-21 | T4-47 |
| T3-21 | T4-48 |
| T3-21 | T4-49 |
| T3-21 | T4-5 |
| T3-21 | T4-50 |
| T3-21 | T4-51 |
| T3-21 | T4-52 |
| T3-21 | T4-53 |
| T3-21 | T4-54 |
| T3-21 | T4-55 |
| T3-21 | T4-56 |
| T3-21 | T4-57 |
| T3-21 | T4-58 |
| T3-21 | T4-59 |
| T3-21 | T4-6 |
| T3-21 | T4-60 |
| T3-21 | T4-61 |
| T3-21 | T4-62 |
| T3-21 | T4-63 |
| T3-21 | T4-64 |
| T3-21 | T4-7 |

| | |
|---|---|
| T3-21 | T4-8 |
| T3-21 | T4-9 |
| T3-22 | T4-1 |
| T3-22 | T4-10 |
| T3-22 | T4-11 |
| T3-22 | T4-12 |
| T3-22 | T4-13 |
| T3-22 | T4-14 |
| T3-22 | T4-15 |
| T3-22 | T4-16 |
| T3-22 | T4-17 |
| T3-22 | T4-18 |
| T3-22 | T4-19 |
| T3-22 | T4-2 |
| T3-22 | T4-20 |
| T3-22 | T4-21 |
| T3-22 | T4-22 |
| T3-22 | T4-23 |
| T3-22 | T4-24 |
| T3-22 | T4-25 |
| T3-22 | T4-26 |
| T3-22 | T4-27 |
| T3-22 | T4-28 |
| T3-22 | T4-29 |
| T3-22 | T4-3 |
| T3-22 | T4-30 |
| T3-22 | T4-31 |
| T3-22 | T4-32 |
| T3-22 | T4-33 |
| T3-22 | T4-34 |
| T3-22 | T4-35 |
| T3-22 | T4-36 |
| T3-22 | T4-37 |
| T3-22 | T4-38 |
| T3-22 | T4-39 |
| T3-22 | T4-4 |
| T3-22 | T4-40 |
| T3-22 | T4-41 |
| T3-22 | T4-42 |
| T3-22 | T4-43 |
| T3-22 | T4-44 |
| T3-22 | T4-45 |
| T3-22 | T4-46 |
| T3-22 | T4-47 |
| T3-22 | T4-48 |

| | |
|---|---|
| T3-22 | T4-49 |
| T3-22 | T4-5 |
| T3-22 | T4-50 |
| T3-22 | T4-51 |
| T3-22 | T4-52 |
| T3-22 | T4-53 |
| T3-22 | T4-54 |
| T3-22 | T4-55 |
| T3-22 | T4-56 |
| T3-22 | T4-57 |
| T3-22 | T4-58 |
| T3-22 | T4-59 |
| T3-22 | T4-6 |
| T3-22 | T4-60 |
| T3-22 | T4-61 |
| T3-22 | T4-62 |
| T3-22 | T4-63 |
| T3-22 | T4-64 |
| T3-22 | T4-7 |
| T3-22 | T4-8 |
| T3-22 | T4-9 |
| T3-23 | T4-1 |
| T3-23 | T4-10 |
| T3-23 | T4-11 |
| T3-23 | T4-12 |
| T3-23 | T4-13 |
| T3-23 | T4-14 |
| T3-23 | T4-15 |
| T3-23 | T4-16 |
| T3-23 | T4-17 |
| T3-23 | T4-18 |
| T3-23 | T4-19 |
| T3-23 | T4-2 |
| T3-23 | T4-20 |
| T3-23 | T4-21 |
| T3-23 | T4-22 |
| T3-23 | T4-23 |
| T3-23 | T4-24 |
| T3-23 | T4-25 |
| T3-23 | T4-26 |
| T3-23 | T4-27 |
| T3-23 | T4-28 |
| T3-23 | T4-29 |
| T3-23 | T4-3 |
| T3-23 | T4-30 |

| | |
|---|---|
| T3-23 | T4-31 |
| T3-23 | T4-32 |
| T3-23 | T4-33 |
| T3-23 | T4-34 |
| T3-23 | T4-35 |
| T3-23 | T4-36 |
| T3-23 | T4-37 |
| T3-23 | T4-38 |
| T3-23 | T4-39 |
| T3-23 | T4-4 |
| T3-23 | T4-40 |
| T3-23 | T4-41 |
| T3-23 | T4-42 |
| T3-23 | T4-43 |
| T3-23 | T4-44 |
| T3-23 | T4-45 |
| T3-23 | T4-46 |
| T3-23 | T4-47 |
| T3-23 | T4-48 |
| T3-23 | T4-49 |
| T3-23 | T4-5 |
| T3-23 | T4-50 |
| T3-23 | T4-51 |
| T3-23 | T4-52 |
| T3-23 | T4-53 |
| T3-23 | T4-54 |
| T3-23 | T4-55 |
| T3-23 | T4-56 |
| T3-23 | T4-57 |
| T3-23 | T4-58 |
| T3-23 | T4-59 |
| T3-23 | T4-6 |
| T3-23 | T4-60 |
| T3-23 | T4-61 |
| T3-23 | T4-62 |
| T3-23 | T4-63 |
| T3-23 | T4-64 |
| T3-23 | T4-7 |
| T3-23 | T4-8 |
| T3-23 | T4-9 |
| T3-24 | T4-1 |
| T3-24 | T4-10 |
| T3-24 | T4-11 |
| T3-24 | T4-12 |
| T3-24 | T4-13 |

| | |
|---|---|
| T3-24 | T4-14 |
| T3-24 | T4-15 |
| T3-24 | T4-16 |
| T3-24 | T4-17 |
| T3-24 | T4-18 |
| T3-24 | T4-19 |
| T3-24 | T4-2 |
| T3-24 | T4-20 |
| T3-24 | T4-21 |
| T3-24 | T4-22 |
| T3-24 | T4-23 |
| T3-24 | T4-24 |
| T3-24 | T4-25 |
| T3-24 | T4-26 |
| T3-24 | T4-27 |
| T3-24 | T4-28 |
| T3-24 | T4-29 |
| T3-24 | T4-3 |
| T3-24 | T4-30 |
| T3-24 | T4-31 |
| T3-24 | T4-32 |
| T3-24 | T4-33 |
| T3-24 | T4-34 |
| T3-24 | T4-35 |
| T3-24 | T4-36 |
| T3-24 | T4-37 |
| T3-24 | T4-38 |
| T3-24 | T4-39 |
| T3-24 | T4-4 |
| T3-24 | T4-40 |
| T3-24 | T4-41 |
| T3-24 | T4-42 |
| T3-24 | T4-43 |
| T3-24 | T4-44 |
| T3-24 | T4-45 |
| T3-24 | T4-46 |
| T3-24 | T4-47 |
| T3-24 | T4-48 |
| T3-24 | T4-49 |
| T3-24 | T4-5 |
| T3-24 | T4-50 |
| T3-24 | T4-51 |
| T3-24 | T4-52 |
| T3-24 | T4-53 |
| T3-24 | T4-54 |

| | |
|---|---|
| T3-24 | T4-55 |
| T3-24 | T4-56 |
| T3-24 | T4-57 |
| T3-24 | T4-58 |
| T3-24 | T4-59 |
| T3-24 | T4-6 |
| T3-24 | T4-60 |
| T3-24 | T4-61 |
| T3-24 | T4-62 |
| T3-24 | T4-63 |
| T3-24 | T4-64 |
| T3-24 | T4-7 |
| T3-24 | T4-8 |
| T3-24 | T4-9 |
| T3-25 | T4-1 |
| T3-25 | T4-10 |
| T3-25 | T4-11 |
| T3-25 | T4-12 |
| T3-25 | T4-13 |
| T3-25 | T4-14 |
| T3-25 | T4-15 |
| T3-25 | T4-16 |
| T3-25 | T4-17 |
| T3-25 | T4-18 |
| T3-25 | T4-19 |
| T3-25 | T4-2 |
| T3-25 | T4-20 |
| T3-25 | T4-21 |
| T3-25 | T4-22 |
| T3-25 | T4-23 |
| T3-25 | T4-24 |
| T3-25 | T4-25 |
| T3-25 | T4-26 |
| T3-25 | T4-27 |
| T3-25 | T4-28 |
| T3-25 | T4-29 |
| T3-25 | T4-3 |
| T3-25 | T4-30 |
| T3-25 | T4-31 |
| T3-25 | T4-32 |
| T3-25 | T4-33 |
| T3-25 | T4-34 |
| T3-25 | T4-35 |
| T3-25 | T4-36 |
| T3-25 | T4-37 |

| | |
|---|---|
| T3-25 | T4-38 |
| T3-25 | T4-39 |
| T3-25 | T4-4 |
| T3-25 | T4-40 |
| T3-25 | T4-41 |
| T3-25 | T4-42 |
| T3-25 | T4-43 |
| T3-25 | T4-44 |
| T3-25 | T4-45 |
| T3-25 | T4-46 |
| T3-25 | T4-47 |
| T3-25 | T4-48 |
| T3-25 | T4-49 |
| T3-25 | T4-5 |
| T3-25 | T4-50 |
| T3-25 | T4-51 |
| T3-25 | T4-52 |
| T3-25 | T4-53 |
| T3-25 | T4-54 |
| T3-25 | T4-55 |
| T3-25 | T4-56 |
| T3-25 | T4-57 |
| T3-25 | T4-58 |
| T3-25 | T4-59 |
| T3-25 | T4-6 |
| T3-25 | T4-60 |
| T3-25 | T4-61 |
| T3-25 | T4-62 |
| T3-25 | T4-63 |
| T3-25 | T4-64 |
| T3-25 | T4-7 |
| T3-25 | T4-8 |
| T3-25 | T4-9 |
| T3-26 | T4-1 |
| T3-26 | T4-10 |
| T3-26 | T4-11 |
| T3-26 | T4-12 |
| T3-26 | T4-13 |
| T3-26 | T4-14 |
| T3-26 | T4-15 |
| T3-26 | T4-16 |
| T3-26 | T4-17 |
| T3-26 | T4-18 |
| T3-26 | T4-19 |
| T3-26 | T4-2 |

| | |
|---|---|
| T3-26 | T4-20 |
| T3-26 | T4-21 |
| T3-26 | T4-22 |
| T3-26 | T4-23 |
| T3-26 | T4-24 |
| T3-26 | T4-25 |
| T3-26 | T4-26 |
| T3-26 | T4-27 |
| T3-26 | T4-28 |
| T3-26 | T4-29 |
| T3-26 | T4-3 |
| T3-26 | T4-30 |
| T3-26 | T4-31 |
| T3-26 | T4-32 |
| T3-26 | T4-33 |
| T3-26 | T4-34 |
| T3-26 | T4-35 |
| T3-26 | T4-36 |
| T3-26 | T4-37 |
| T3-26 | T4-38 |
| T3-26 | T4-39 |
| T3-26 | T4-4 |
| T3-26 | T4-40 |
| T3-26 | T4-41 |
| T3-26 | T4-42 |
| T3-26 | T4-43 |
| T3-26 | T4-44 |
| T3-26 | T4-45 |
| T3-26 | T4-46 |
| T3-26 | T4-47 |
| T3-26 | T4-48 |
| T3-26 | T4-49 |
| T3-26 | T4-5 |
| T3-26 | T4-50 |
| T3-26 | T4-51 |
| T3-26 | T4-52 |
| T3-26 | T4-53 |
| T3-26 | T4-54 |
| T3-26 | T4-55 |
| T3-26 | T4-56 |
| T3-26 | T4-57 |
| T3-26 | T4-58 |
| T3-26 | T4-59 |
| T3-26 | T4-6 |
| T3-26 | T4-60 |

| | |
|---|---|
| T3-26 | T4-61 |
| T3-26 | T4-62 |
| T3-26 | T4-63 |
| T3-26 | T4-64 |
| T3-26 | T4-7 |
| T3-26 | T4-8 |
| T3-26 | T4-9 |
| T3-27 | T4-1 |
| T3-27 | T4-10 |
| T3-27 | T4-11 |
| T3-27 | T4-12 |
| T3-27 | T4-13 |
| T3-27 | T4-14 |
| T3-27 | T4-15 |
| T3-27 | T4-16 |
| T3-27 | T4-17 |
| T3-27 | T4-18 |
| T3-27 | T4-19 |
| T3-27 | T4-2 |
| T3-27 | T4-20 |
| T3-27 | T4-21 |
| T3-27 | T4-22 |
| T3-27 | T4-23 |
| T3-27 | T4-24 |
| T3-27 | T4-25 |
| T3-27 | T4-26 |
| T3-27 | T4-27 |
| T3-27 | T4-28 |
| T3-27 | T4-29 |
| T3-27 | T4-3 |
| T3-27 | T4-30 |
| T3-27 | T4-31 |
| T3-27 | T4-32 |
| T3-27 | T4-33 |
| T3-27 | T4-34 |
| T3-27 | T4-35 |
| T3-27 | T4-36 |
| T3-27 | T4-37 |
| T3-27 | T4-38 |
| T3-27 | T4-39 |
| T3-27 | T4-4 |
| T3-27 | T4-40 |
| T3-27 | T4-41 |
| T3-27 | T4-42 |
| T3-27 | T4-43 |

| | |
|---|---|
| T3-27 | T4-44 |
| T3-27 | T4-45 |
| T3-27 | T4-46 |
| T3-27 | T4-47 |
| T3-27 | T4-48 |
| T3-27 | T4-49 |
| T3-27 | T4-5 |
| T3-27 | T4-50 |
| T3-27 | T4-51 |
| T3-27 | T4-52 |
| T3-27 | T4-53 |
| T3-27 | T4-54 |
| T3-27 | T4-55 |
| T3-27 | T4-56 |
| T3-27 | T4-57 |
| T3-27 | T4-58 |
| T3-27 | T4-59 |
| T3-27 | T4-6 |
| T3-27 | T4-60 |
| T3-27 | T4-61 |
| T3-27 | T4-62 |
| T3-27 | T4-63 |
| T3-27 | T4-64 |
| T3-27 | T4-7 |
| T3-27 | T4-8 |
| T3-27 | T4-9 |
| T3-28 | T4-1 |
| T3-28 | T4-10 |
| T3-28 | T4-11 |
| T3-28 | T4-12 |
| T3-28 | T4-13 |
| T3-28 | T4-14 |
| T3-28 | T4-15 |
| T3-28 | T4-16 |
| T3-28 | T4-17 |
| T3-28 | T4-18 |
| T3-28 | T4-19 |
| T3-28 | T4-2 |
| T3-28 | T4-20 |
| T3-28 | T4-21 |
| T3-28 | T4-22 |
| T3-28 | T4-23 |
| T3-28 | T4-24 |
| T3-28 | T4-25 |
| T3-28 | T4-26 |

| | |
|---|---|
| T3-28 | T4-27 |
| T3-28 | T4-28 |
| T3-28 | T4-29 |
| T3-28 | T4-3 |
| T3-28 | T4-30 |
| T3-28 | T4-31 |
| T3-28 | T4-32 |
| T3-28 | T4-33 |
| T3-28 | T4-34 |
| T3-28 | T4-35 |
| T3-28 | T4-36 |
| T3-28 | T4-37 |
| T3-28 | T4-38 |
| T3-28 | T4-39 |
| T3-28 | T4-4 |
| T3-28 | T4-40 |
| T3-28 | T4-41 |
| T3-28 | T4-42 |
| T3-28 | T4-43 |
| T3-28 | T4-44 |
| T3-28 | T4-45 |
| T3-28 | T4-46 |
| T3-28 | T4-47 |
| T3-28 | T4-48 |
| T3-28 | T4-49 |
| T3-28 | T4-5 |
| T3-28 | T4-50 |
| T3-28 | T4-51 |
| T3-28 | T4-52 |
| T3-28 | T4-53 |
| T3-28 | T4-54 |
| T3-28 | T4-55 |
| T3-28 | T4-56 |
| T3-28 | T4-57 |
| T3-28 | T4-58 |
| T3-28 | T4-59 |
| T3-28 | T4-6 |
| T3-28 | T4-60 |
| T3-28 | T4-61 |
| T3-28 | T4-62 |
| T3-28 | T4-63 |
| T3-28 | T4-64 |
| T3-28 | T4-7 |
| T3-28 | T4-8 |
| T3-28 | T4-9 |

| | |
|---|---|
| T3-29 | T4-1 |
| T3-29 | T4-10 |
| T3-29 | T4-11 |
| T3-29 | T4-12 |
| T3-29 | T4-13 |
| T3-29 | T4-14 |
| T3-29 | T4-15 |
| T3-29 | T4-16 |
| T3-29 | T4-17 |
| T3-29 | T4-18 |
| T3-29 | T4-19 |
| T3-29 | T4-2 |
| T3-29 | T4-20 |
| T3-29 | T4-21 |
| T3-29 | T4-22 |
| T3-29 | T4-23 |
| T3-29 | T4-24 |
| T3-29 | T4-25 |
| T3-29 | T4-26 |
| T3-29 | T4-27 |
| T3-29 | T4-28 |
| T3-29 | T4-29 |
| T3-29 | T4-3 |
| T3-29 | T4-30 |
| T3-29 | T4-31 |
| T3-29 | T4-32 |
| T3-29 | T4-33 |
| T3-29 | T4-34 |
| T3-29 | T4-35 |
| T3-29 | T4-36 |
| T3-29 | T4-37 |
| T3-29 | T4-38 |
| T3-29 | T4-39 |
| T3-29 | T4-4 |
| T3-29 | T4-40 |
| T3-29 | T4-41 |
| T3-29 | T4-42 |
| T3-29 | T4-43 |
| T3-29 | T4-44 |
| T3-29 | T4-45 |
| T3-29 | T4-46 |
| T3-29 | T4-47 |
| T3-29 | T4-48 |
| T3-29 | T4-49 |
| T3-29 | T4-5 |

| | |
|---|---|
| T3-29 | T4-50 |
| T3-29 | T4-51 |
| T3-29 | T4-52 |
| T3-29 | T4-53 |
| T3-29 | T4-54 |
| T3-29 | T4-55 |
| T3-29 | T4-56 |
| T3-29 | T4-57 |
| T3-29 | T4-58 |
| T3-29 | T4-59 |
| T3-29 | T4-6 |
| T3-29 | T4-60 |
| T3-29 | T4-61 |
| T3-29 | T4-62 |
| T3-29 | T4-63 |
| T3-29 | T4-64 |
| T3-29 | T4-7 |
| T3-29 | T4-8 |
| T3-29 | T4-9 |
| T3-3 | T4-1 |
| T3-3 | T4-10 |
| T3-3 | T4-11 |
| T3-3 | T4-12 |
| T3-3 | T4-13 |
| T3-3 | T4-14 |
| T3-3 | T4-15 |
| T3-3 | T4-16 |
| T3-3 | T4-17 |
| T3-3 | T4-18 |
| T3-3 | T4-19 |
| T3-3 | T4-2 |
| T3-3 | T4-20 |
| T3-3 | T4-21 |
| T3-3 | T4-22 |
| T3-3 | T4-23 |
| T3-3 | T4-24 |
| T3-3 | T4-25 |
| T3-3 | T4-26 |
| T3-3 | T4-27 |
| T3-3 | T4-28 |
| T3-3 | T4-29 |
| T3-3 | T4-3 |
| T3-3 | T4-30 |
| T3-3 | T4-31 |
| T3-3 | T4-32 |

| | |
|---|---|
| T3-3 | T4-33 |
| T3-3 | T4-34 |
| T3-3 | T4-35 |
| T3-3 | T4-36 |
| T3-3 | T4-37 |
| T3-3 | T4-38 |
| T3-3 | T4-39 |
| T3-3 | T4-4 |
| T3-3 | T4-40 |
| T3-3 | T4-41 |
| T3-3 | T4-42 |
| T3-3 | T4-43 |
| T3-3 | T4-44 |
| T3-3 | T4-45 |
| T3-3 | T4-46 |
| T3-3 | T4-47 |
| T3-3 | T4-48 |
| T3-3 | T4-49 |
| T3-3 | T4-5 |
| T3-3 | T4-50 |
| T3-3 | T4-51 |
| T3-3 | T4-52 |
| T3-3 | T4-53 |
| T3-3 | T4-54 |
| T3-3 | T4-55 |
| T3-3 | T4-56 |
| T3-3 | T4-57 |
| T3-3 | T4-58 |
| T3-3 | T4-59 |
| T3-3 | T4-6 |
| T3-3 | T4-60 |
| T3-3 | T4-61 |
| T3-3 | T4-62 |
| T3-3 | T4-63 |
| T3-3 | T4-64 |
| T3-3 | T4-7 |
| T3-3 | T4-8 |
| T3-3 | T4-9 |
| T3-30 | T4-1 |
| T3-30 | T4-10 |
| T3-30 | T4-11 |
| T3-30 | T4-12 |
| T3-30 | T4-13 |
| T3-30 | T4-14 |
| T3-30 | T4-15 |

| | |
|---|---|
| T3-30 | T4-16 |
| T3-30 | T4-17 |
| T3-30 | T4-18 |
| T3-30 | T4-19 |
| T3-30 | T4-2 |
| T3-30 | T4-20 |
| T3-30 | T4-21 |
| T3-30 | T4-22 |
| T3-30 | T4-23 |
| T3-30 | T4-24 |
| T3-30 | T4-25 |
| T3-30 | T4-26 |
| T3-30 | T4-27 |
| T3-30 | T4-28 |
| T3-30 | T4-29 |
| T3-30 | T4-3 |
| T3-30 | T4-30 |
| T3-30 | T4-31 |
| T3-30 | T4-32 |
| T3-30 | T4-33 |
| T3-30 | T4-34 |
| T3-30 | T4-35 |
| T3-30 | T4-36 |
| T3-30 | T4-37 |
| T3-30 | T4-38 |
| T3-30 | T4-39 |
| T3-30 | T4-4 |
| T3-30 | T4-40 |
| T3-30 | T4-41 |
| T3-30 | T4-42 |
| T3-30 | T4-43 |
| T3-30 | T4-44 |
| T3-30 | T4-45 |
| T3-30 | T4-46 |
| T3-30 | T4-47 |
| T3-30 | T4-48 |
| T3-30 | T4-49 |
| T3-30 | T4-5 |
| T3-30 | T4-50 |
| T3-30 | T4-51 |
| T3-30 | T4-52 |
| T3-30 | T4-53 |
| T3-30 | T4-54 |
| T3-30 | T4-55 |
| T3-30 | T4-56 |

| | |
|---|---|
| T3-30 | T4-57 |
| T3-30 | T4-58 |
| T3-30 | T4-59 |
| T3-30 | T4-6 |
| T3-30 | T4-60 |
| T3-30 | T4-61 |
| T3-30 | T4-62 |
| T3-30 | T4-63 |
| T3-30 | T4-64 |
| T3-30 | T4-7 |
| T3-30 | T4-8 |
| T3-30 | T4-9 |
| T3-31 | T4-1 |
| T3-31 | T4-10 |
| T3-31 | T4-11 |
| T3-31 | T4-12 |
| T3-31 | T4-13 |
| T3-31 | T4-14 |
| T3-31 | T4-15 |
| T3-31 | T4-16 |
| T3-31 | T4-17 |
| T3-31 | T4-18 |
| T3-31 | T4-19 |
| T3-31 | T4-2 |
| T3-31 | T4-20 |
| T3-31 | T4-21 |
| T3-31 | T4-22 |
| T3-31 | T4-23 |
| T3-31 | T4-24 |
| T3-31 | T4-25 |
| T3-31 | T4-26 |
| T3-31 | T4-27 |
| T3-31 | T4-28 |
| T3-31 | T4-29 |
| T3-31 | T4-3 |
| T3-31 | T4-30 |
| T3-31 | T4-31 |
| T3-31 | T4-32 |
| T3-31 | T4-33 |
| T3-31 | T4-34 |
| T3-31 | T4-35 |
| T3-31 | T4-36 |
| T3-31 | T4-37 |
| T3-31 | T4-38 |
| T3-31 | T4-39 |

| | |
|---|---|
| T3-31 | T4-4 |
| T3-31 | T4-40 |
| T3-31 | T4-41 |
| T3-31 | T4-42 |
| T3-31 | T4-43 |
| T3-31 | T4-44 |
| T3-31 | T4-45 |
| T3-31 | T4-46 |
| T3-31 | T4-47 |
| T3-31 | T4-48 |
| T3-31 | T4-49 |
| T3-31 | T4-5 |
| T3-31 | T4-50 |
| T3-31 | T4-51 |
| T3-31 | T4-52 |
| T3-31 | T4-53 |
| T3-31 | T4-54 |
| T3-31 | T4-55 |
| T3-31 | T4-56 |
| T3-31 | T4-57 |
| T3-31 | T4-58 |
| T3-31 | T4-59 |
| T3-31 | T4-6 |
| T3-31 | T4-60 |
| T3-31 | T4-61 |
| T3-31 | T4-62 |
| T3-31 | T4-63 |
| T3-31 | T4-64 |
| T3-31 | T4-7 |
| T3-31 | T4-8 |
| T3-31 | T4-9 |
| T3-32 | T4-1 |
| T3-32 | T4-10 |
| T3-32 | T4-11 |
| T3-32 | T4-12 |
| T3-32 | T4-13 |
| T3-32 | T4-14 |
| T3-32 | T4-15 |
| T3-32 | T4-16 |
| T3-32 | T4-17 |
| T3-32 | T4-18 |
| T3-32 | T4-19 |
| T3-32 | T4-2 |
| T3-32 | T4-20 |
| T3-32 | T4-21 |

| | |
|---|---|
| T3-32 | T4-22 |
| T3-32 | T4-23 |
| T3-32 | T4-24 |
| T3-32 | T4-25 |
| T3-32 | T4-26 |
| T3-32 | T4-27 |
| T3-32 | T4-28 |
| T3-32 | T4-29 |
| T3-32 | T4-3 |
| T3-32 | T4-30 |
| T3-32 | T4-31 |
| T3-32 | T4-32 |
| T3-32 | T4-33 |
| T3-32 | T4-34 |
| T3-32 | T4-35 |
| T3-32 | T4-36 |
| T3-32 | T4-37 |
| T3-32 | T4-38 |
| T3-32 | T4-39 |
| T3-32 | T4-4 |
| T3-32 | T4-40 |
| T3-32 | T4-41 |
| T3-32 | T4-42 |
| T3-32 | T4-43 |
| T3-32 | T4-44 |
| T3-32 | T4-45 |
| T3-32 | T4-46 |
| T3-32 | T4-47 |
| T3-32 | T4-48 |
| T3-32 | T4-49 |
| T3-32 | T4-5 |
| T3-32 | T4-50 |
| T3-32 | T4-51 |
| T3-32 | T4-52 |
| T3-32 | T4-53 |
| T3-32 | T4-54 |
| T3-32 | T4-55 |
| T3-32 | T4-56 |
| T3-32 | T4-57 |
| T3-32 | T4-58 |
| T3-32 | T4-59 |
| T3-32 | T4-6 |
| T3-32 | T4-60 |
| T3-32 | T4-61 |
| T3-32 | T4-62 |

| | |
|---|---|
| T3-32 | T4-63 |
| T3-32 | T4-64 |
| T3-32 | T4-7 |
| T3-32 | T4-8 |
| T3-32 | T4-9 |
| T3-33 | T4-1 |
| T3-33 | T4-10 |
| T3-33 | T4-11 |
| T3-33 | T4-12 |
| T3-33 | T4-13 |
| T3-33 | T4-14 |
| T3-33 | T4-15 |
| T3-33 | T4-16 |
| T3-33 | T4-17 |
| T3-33 | T4-18 |
| T3-33 | T4-19 |
| T3-33 | T4-2 |
| T3-33 | T4-20 |
| T3-33 | T4-21 |
| T3-33 | T4-22 |
| T3-33 | T4-23 |
| T3-33 | T4-24 |
| T3-33 | T4-25 |
| T3-33 | T4-26 |
| T3-33 | T4-27 |
| T3-33 | T4-28 |
| T3-33 | T4-29 |
| T3-33 | T4-3 |
| T3-33 | T4-30 |
| T3-33 | T4-31 |
| T3-33 | T4-32 |
| T3-33 | T4-33 |
| T3-33 | T4-34 |
| T3-33 | T4-35 |
| T3-33 | T4-36 |
| T3-33 | T4-37 |
| T3-33 | T4-38 |
| T3-33 | T4-39 |
| T3-33 | T4-4 |
| T3-33 | T4-40 |
| T3-33 | T4-41 |
| T3-33 | T4-42 |
| T3-33 | T4-43 |
| T3-33 | T4-44 |
| T3-33 | T4-45 |

| | |
|---|---|
| T3-33 | T4-46 |
| T3-33 | T4-47 |
| T3-33 | T4-48 |
| T3-33 | T4-49 |
| T3-33 | T4-5 |
| T3-33 | T4-50 |
| T3-33 | T4-51 |
| T3-33 | T4-52 |
| T3-33 | T4-53 |
| T3-33 | T4-54 |
| T3-33 | T4-55 |
| T3-33 | T4-56 |
| T3-33 | T4-57 |
| T3-33 | T4-58 |
| T3-33 | T4-59 |
| T3-33 | T4-6 |
| T3-33 | T4-60 |
| T3-33 | T4-61 |
| T3-33 | T4-62 |
| T3-33 | T4-63 |
| T3-33 | T4-64 |
| T3-33 | T4-7 |
| T3-33 | T4-8 |
| T3-33 | T4-9 |
| T3-34 | T4-1 |
| T3-34 | T4-10 |
| T3-34 | T4-11 |
| T3-34 | T4-12 |
| T3-34 | T4-13 |
| T3-34 | T4-14 |
| T3-34 | T4-15 |
| T3-34 | T4-16 |
| T3-34 | T4-17 |
| T3-34 | T4-18 |
| T3-34 | T4-19 |
| T3-34 | T4-2 |
| T3-34 | T4-20 |
| T3-34 | T4-21 |
| T3-34 | T4-22 |
| T3-34 | T4-23 |
| T3-34 | T4-24 |
| T3-34 | T4-25 |
| T3-34 | T4-26 |
| T3-34 | T4-27 |
| T3-34 | T4-28 |

| | |
|---|---|
| T3-34 | T4-29 |
| T3-34 | T4-3 |
| T3-34 | T4-30 |
| T3-34 | T4-31 |
| T3-34 | T4-32 |
| T3-34 | T4-33 |
| T3-34 | T4-34 |
| T3-34 | T4-35 |
| T3-34 | T4-36 |
| T3-34 | T4-37 |
| T3-34 | T4-38 |
| T3-34 | T4-39 |
| T3-34 | T4-4 |
| T3-34 | T4-40 |
| T3-34 | T4-41 |
| T3-34 | T4-42 |
| T3-34 | T4-43 |
| T3-34 | T4-44 |
| T3-34 | T4-45 |
| T3-34 | T4-46 |
| T3-34 | T4-47 |
| T3-34 | T4-48 |
| T3-34 | T4-49 |
| T3-34 | T4-5 |
| T3-34 | T4-50 |
| T3-34 | T4-51 |
| T3-34 | T4-52 |
| T3-34 | T4-53 |
| T3-34 | T4-54 |
| T3-34 | T4-55 |
| T3-34 | T4-56 |
| T3-34 | T4-57 |
| T3-34 | T4-58 |
| T3-34 | T4-59 |
| T3-34 | T4-6 |
| T3-34 | T4-60 |
| T3-34 | T4-61 |
| T3-34 | T4-62 |
| T3-34 | T4-63 |
| T3-34 | T4-64 |
| T3-34 | T4-7 |
| T3-34 | T4-8 |
| T3-34 | T4-9 |
| T3-35 | T4-1 |
| T3-35 | T4-10 |

| | |
|---|---|
| T3-35 | T4-11 |
| T3-35 | T4-12 |
| T3-35 | T4-13 |
| T3-35 | T4-14 |
| T3-35 | T4-15 |
| T3-35 | T4-16 |
| T3-35 | T4-17 |
| T3-35 | T4-18 |
| T3-35 | T4-19 |
| T3-35 | T4-2 |
| T3-35 | T4-20 |
| T3-35 | T4-21 |
| T3-35 | T4-22 |
| T3-35 | T4-23 |
| T3-35 | T4-24 |
| T3-35 | T4-25 |
| T3-35 | T4-26 |
| T3-35 | T4-27 |
| T3-35 | T4-28 |
| T3-35 | T4-29 |
| T3-35 | T4-3 |
| T3-35 | T4-30 |
| T3-35 | T4-31 |
| T3-35 | T4-32 |
| T3-35 | T4-33 |
| T3-35 | T4-34 |
| T3-35 | T4-35 |
| T3-35 | T4-36 |
| T3-35 | T4-37 |
| T3-35 | T4-38 |
| T3-35 | T4-39 |
| T3-35 | T4-4 |
| T3-35 | T4-40 |
| T3-35 | T4-41 |
| T3-35 | T4-42 |
| T3-35 | T4-43 |
| T3-35 | T4-44 |
| T3-35 | T4-45 |
| T3-35 | T4-46 |
| T3-35 | T4-47 |
| T3-35 | T4-48 |
| T3-35 | T4-49 |
| T3-35 | T4-5 |
| T3-35 | T4-50 |
| T3-35 | T4-51 |

| T3-35 | T4-52 |
|---|---|
| T3-35 | T4-53 |
| T3-35 | T4-54 |
| T3-35 | T4-55 |
| T3-35 | T4-56 |
| T3-35 | T4-57 |
| T3-35 | T4-58 |
| T3-35 | T4-59 |
| T3-35 | T4-6 |
| T3-35 | T4-60 |
| T3-35 | T4-61 |
| T3-35 | T4-62 |
| T3-35 | T4-63 |
| T3-35 | T4-64 |
| T3-35 | T4-7 |
| T3-35 | T4-8 |
| T3-35 | T4-9 |
| T3-36 | T4-1 |
| T3-36 | T4-10 |
| T3-36 | T4-11 |
| T3-36 | T4-12 |
| T3-36 | T4-13 |
| T3-36 | T4-14 |
| T3-36 | T4-15 |
| T3-36 | T4-16 |
| T3-36 | T4-17 |
| T3-36 | T4-18 |
| T3-36 | T4-19 |
| T3-36 | T4-2 |
| T3-36 | T4-20 |
| T3-36 | T4-21 |
| T3-36 | T4-22 |
| T3-36 | T4-23 |
| T3-36 | T4-24 |
| T3-36 | T4-25 |
| T3-36 | T4-26 |
| T3-36 | T4-27 |
| T3-36 | T4-28 |
| T3-36 | T4-29 |
| T3-36 | T4-3 |
| T3-36 | T4-30 |
| T3-36 | T4-31 |
| T3-36 | T4-32 |
| T3-36 | T4-33 |
| T3-36 | T4-34 |

| T3-36 | T4-35 |
|---|---|
| T3-36 | T4-36 |
| T3-36 | T4-37 |
| T3-36 | T4-38 |
| T3-36 | T4-39 |
| T3-36 | T4-4 |
| T3-36 | T4-40 |
| T3-36 | T4-41 |
| T3-36 | T4-42 |
| T3-36 | T4-43 |
| T3-36 | T4-44 |
| T3-36 | T4-45 |
| T3-36 | T4-46 |
| T3-36 | T4-47 |
| T3-36 | T4-48 |
| T3-36 | T4-49 |
| T3-36 | T4-5 |
| T3-36 | T4-50 |
| T3-36 | T4-51 |
| T3-36 | T4-52 |
| T3-36 | T4-53 |
| T3-36 | T4-54 |
| T3-36 | T4-55 |
| T3-36 | T4-56 |
| T3-36 | T4-57 |
| T3-36 | T4-58 |
| T3-36 | T4-59 |
| T3-36 | T4-6 |
| T3-36 | T4-60 |
| T3-36 | T4-61 |
| T3-36 | T4-62 |
| T3-36 | T4-63 |
| T3-36 | T4-64 |
| T3-36 | T4-7 |
| T3-36 | T4-8 |
| T3-36 | T4-9 |
| T3-37 | T4-1 |
| T3-37 | T4-10 |
| T3-37 | T4-11 |
| T3-37 | T4-12 |
| T3-37 | T4-13 |
| T3-37 | T4-14 |
| T3-37 | T4-15 |
| T3-37 | T4-16 |
| T3-37 | T4-17 |

| T3-37 | T4-18 |
|---|---|
| T3-37 | T4-19 |
| T3-37 | T4-2 |
| T3-37 | T4-20 |
| T3-37 | T4-21 |
| T3-37 | T4-22 |
| T3-37 | T4-23 |
| T3-37 | T4-24 |
| T3-37 | T4-25 |
| T3-37 | T4-26 |
| T3-37 | T4-27 |
| T3-37 | T4-28 |
| T3-37 | T4-29 |
| T3-37 | T4-3 |
| T3-37 | T4-30 |
| T3-37 | T4-31 |
| T3-37 | T4-32 |
| T3-37 | T4-33 |
| T3-37 | T4-34 |
| T3-37 | T4-35 |
| T3-37 | T4-36 |
| T3-37 | T4-37 |
| T3-37 | T4-38 |
| T3-37 | T4-39 |
| T3-37 | T4-4 |
| T3-37 | T4-40 |
| T3-37 | T4-41 |
| T3-37 | T4-42 |
| T3-37 | T4-43 |
| T3-37 | T4-44 |
| T3-37 | T4-45 |
| T3-37 | T4-46 |
| T3-37 | T4-47 |
| T3-37 | T4-48 |
| T3-37 | T4-49 |
| T3-37 | T4-5 |
| T3-37 | T4-50 |
| T3-37 | T4-51 |
| T3-37 | T4-52 |
| T3-37 | T4-53 |
| T3-37 | T4-54 |
| T3-37 | T4-55 |
| T3-37 | T4-56 |
| T3-37 | T4-57 |
| T3-37 | T4-58 |

| T3-37 | T4-59 |
|---|---|
| T3-37 | T4-6 |
| T3-37 | T4-60 |
| T3-37 | T4-61 |
| T3-37 | T4-62 |
| T3-37 | T4-63 |
| T3-37 | T4-64 |
| T3-37 | T4-7 |
| T3-37 | T4-8 |
| T3-37 | T4-9 |
| T3-38 | T4-1 |
| T3-38 | T4-10 |
| T3-38 | T4-11 |
| T3-38 | T4-12 |
| T3-38 | T4-13 |
| T3-38 | T4-14 |
| T3-38 | T4-15 |
| T3-38 | T4-16 |
| T3-38 | T4-17 |
| T3-38 | T4-18 |
| T3-38 | T4-19 |
| T3-38 | T4-2 |
| T3-38 | T4-20 |
| T3-38 | T4-21 |
| T3-38 | T4-22 |
| T3-38 | T4-23 |
| T3-38 | T4-24 |
| T3-38 | T4-25 |
| T3-38 | T4-26 |
| T3-38 | T4-27 |
| T3-38 | T4-28 |
| T3-38 | T4-29 |
| T3-38 | T4-3 |
| T3-38 | T4-30 |
| T3-38 | T4-31 |
| T3-38 | T4-32 |
| T3-38 | T4-33 |
| T3-38 | T4-34 |
| T3-38 | T4-35 |
| T3-38 | T4-36 |
| T3-38 | T4-37 |
| T3-38 | T4-38 |
| T3-38 | T4-39 |
| T3-38 | T4-4 |
| T3-38 | T4-40 |

| | |
|---|---|
| T3-38 | T4-41 |
| T3-38 | T4-42 |
| T3-38 | T4-43 |
| T3-38 | T4-44 |
| T3-38 | T4-45 |
| T3-38 | T4-46 |
| T3-38 | T4-47 |
| T3-38 | T4-48 |
| T3-38 | T4-49 |
| T3-38 | T4-5 |
| T3-38 | T4-50 |
| T3-38 | T4-51 |
| T3-38 | T4-52 |
| T3-38 | T4-53 |
| T3-38 | T4-54 |
| T3-38 | T4-55 |
| T3-38 | T4-56 |
| T3-38 | T4-57 |
| T3-38 | T4-58 |
| T3-38 | T4-59 |
| T3-38 | T4-6 |
| T3-38 | T4-60 |
| T3-38 | T4-61 |
| T3-38 | T4-62 |
| T3-38 | T4-63 |
| T3-38 | T4-64 |
| T3-38 | T4-7 |
| T3-38 | T4-8 |
| T3-38 | T4-9 |
| T3-39 | T4-1 |
| T3-39 | T4-10 |
| T3-39 | T4-11 |
| T3-39 | T4-12 |
| T3-39 | T4-13 |
| T3-39 | T4-14 |
| T3-39 | T4-15 |
| T3-39 | T4-16 |
| T3-39 | T4-17 |
| T3-39 | T4-18 |
| T3-39 | T4-19 |
| T3-39 | T4-2 |
| T3-39 | T4-20 |
| T3-39 | T4-21 |
| T3-39 | T4-22 |
| T3-39 | T4-23 |

| | |
|---|---|
| T3-39 | T4-24 |
| T3-39 | T4-25 |
| T3-39 | T4-26 |
| T3-39 | T4-27 |
| T3-39 | T4-28 |
| T3-39 | T4-29 |
| T3-39 | T4-3 |
| T3-39 | T4-30 |
| T3-39 | T4-31 |
| T3-39 | T4-32 |
| T3-39 | T4-33 |
| T3-39 | T4-34 |
| T3-39 | T4-35 |
| T3-39 | T4-36 |
| T3-39 | T4-37 |
| T3-39 | T4-38 |
| T3-39 | T4-39 |
| T3-39 | T4-4 |
| T3-39 | T4-40 |
| T3-39 | T4-41 |
| T3-39 | T4-42 |
| T3-39 | T4-43 |
| T3-39 | T4-44 |
| T3-39 | T4-45 |
| T3-39 | T4-46 |
| T3-39 | T4-47 |
| T3-39 | T4-48 |
| T3-39 | T4-49 |
| T3-39 | T4-5 |
| T3-39 | T4-50 |
| T3-39 | T4-51 |
| T3-39 | T4-52 |
| T3-39 | T4-53 |
| T3-39 | T4-54 |
| T3-39 | T4-55 |
| T3-39 | T4-56 |
| T3-39 | T4-57 |
| T3-39 | T4-58 |
| T3-39 | T4-59 |
| T3-39 | T4-6 |
| T3-39 | T4-60 |
| T3-39 | T4-61 |
| T3-39 | T4-62 |
| T3-39 | T4-63 |
| T3-39 | T4-64 |

| | |
|---|---|
| T3-39 | T4-7 |
| T3-39 | T4-8 |
| T3-39 | T4-9 |
| T3-4 | T4-1 |
| T3-4 | T4-10 |
| T3-4 | T4-11 |
| T3-4 | T4-12 |
| T3-4 | T4-13 |
| T3-4 | T4-14 |
| T3-4 | T4-15 |
| T3-4 | T4-16 |
| T3-4 | T4-17 |
| T3-4 | T4-18 |
| T3-4 | T4-19 |
| T3-4 | T4-2 |
| T3-4 | T4-20 |
| T3-4 | T4-21 |
| T3-4 | T4-22 |
| T3-4 | T4-23 |
| T3-4 | T4-24 |
| T3-4 | T4-25 |
| T3-4 | T4-26 |
| T3-4 | T4-27 |
| T3-4 | T4-28 |
| T3-4 | T4-29 |
| T3-4 | T4-3 |
| T3-4 | T4-30 |
| T3-4 | T4-31 |
| T3-4 | T4-32 |
| T3-4 | T4-33 |
| T3-4 | T4-34 |
| T3-4 | T4-35 |
| T3-4 | T4-36 |
| T3-4 | T4-37 |
| T3-4 | T4-38 |
| T3-4 | T4-39 |
| T3-4 | T4-4 |
| T3-4 | T4-40 |
| T3-4 | T4-41 |
| T3-4 | T4-42 |
| T3-4 | T4-43 |
| T3-4 | T4-44 |
| T3-4 | T4-45 |
| T3-4 | T4-46 |
| T3-4 | T4-47 |

| | |
|---|---|
| T3-4 | T4-48 |
| T3-4 | T4-49 |
| T3-4 | T4-5 |
| T3-4 | T4-50 |
| T3-4 | T4-51 |
| T3-4 | T4-52 |
| T3-4 | T4-53 |
| T3-4 | T4-54 |
| T3-4 | T4-55 |
| T3-4 | T4-56 |
| T3-4 | T4-57 |
| T3-4 | T4-58 |
| T3-4 | T4-59 |
| T3-4 | T4-6 |
| T3-4 | T4-60 |
| T3-4 | T4-61 |
| T3-4 | T4-62 |
| T3-4 | T4-63 |
| T3-4 | T4-64 |
| T3-4 | T4-7 |
| T3-4 | T4-8 |
| T3-4 | T4-9 |
| T3-40 | T4-1 |
| T3-40 | T4-10 |
| T3-40 | T4-11 |
| T3-40 | T4-12 |
| T3-40 | T4-13 |
| T3-40 | T4-14 |
| T3-40 | T4-15 |
| T3-40 | T4-16 |
| T3-40 | T4-17 |
| T3-40 | T4-18 |
| T3-40 | T4-19 |
| T3-40 | T4-2 |
| T3-40 | T4-20 |
| T3-40 | T4-21 |
| T3-40 | T4-22 |
| T3-40 | T4-23 |
| T3-40 | T4-24 |
| T3-40 | T4-25 |
| T3-40 | T4-26 |
| T3-40 | T4-27 |
| T3-40 | T4-28 |
| T3-40 | T4-29 |
| T3-40 | T4-3 |

| | |
|---|---|
| T3-40 | T4-30 |
| T3-40 | T4-31 |
| T3-40 | T4-32 |
| T3-40 | T4-33 |
| T3-40 | T4-34 |
| T3-40 | T4-35 |
| T3-40 | T4-36 |
| T3-40 | T4-37 |
| T3-40 | T4-38 |
| T3-40 | T4-39 |
| T3-40 | T4-4 |
| T3-40 | T4-40 |
| T3-40 | T4-41 |
| T3-40 | T4-42 |
| T3-40 | T4-43 |
| T3-40 | T4-44 |
| T3-40 | T4-45 |
| T3-40 | T4-46 |
| T3-40 | T4-47 |
| T3-40 | T4-48 |
| T3-40 | T4-49 |
| T3-40 | T4-5 |
| T3-40 | T4-50 |
| T3-40 | T4-51 |
| T3-40 | T4-52 |
| T3-40 | T4-53 |
| T3-40 | T4-54 |
| T3-40 | T4-55 |
| T3-40 | T4-56 |
| T3-40 | T4-57 |
| T3-40 | T4-58 |
| T3-40 | T4-59 |
| T3-40 | T4-6 |
| T3-40 | T4-60 |
| T3-40 | T4-61 |
| T3-40 | T4-62 |
| T3-40 | T4-63 |
| T3-40 | T4-64 |
| T3-40 | T4-7 |
| T3-40 | T4-8 |
| T3-40 | T4-9 |
| T3-41 | T4-1 |
| T3-41 | T4-10 |
| T3-41 | T4-11 |
| T3-41 | T4-12 |

| | |
|---|---|
| T3-41 | T4-13 |
| T3-41 | T4-14 |
| T3-41 | T4-15 |
| T3-41 | T4-16 |
| T3-41 | T4-17 |
| T3-41 | T4-18 |
| T3-41 | T4-19 |
| T3-41 | T4-2 |
| T3-41 | T4-20 |
| T3-41 | T4-21 |
| T3-41 | T4-22 |
| T3-41 | T4-23 |
| T3-41 | T4-24 |
| T3-41 | T4-25 |
| T3-41 | T4-26 |
| T3-41 | T4-27 |
| T3-41 | T4-28 |
| T3-41 | T4-29 |
| T3-41 | T4-3 |
| T3-41 | T4-30 |
| T3-41 | T4-31 |
| T3-41 | T4-32 |
| T3-41 | T4-33 |
| T3-41 | T4-34 |
| T3-41 | T4-35 |
| T3-41 | T4-36 |
| T3-41 | T4-37 |
| T3-41 | T4-38 |
| T3-41 | T4-39 |
| T3-41 | T4-4 |
| T3-41 | T4-40 |
| T3-41 | T4-41 |
| T3-41 | T4-42 |
| T3-41 | T4-43 |
| T3-41 | T4-44 |
| T3-41 | T4-45 |
| T3-41 | T4-46 |
| T3-41 | T4-47 |
| T3-41 | T4-48 |
| T3-41 | T4-49 |
| T3-41 | T4-5 |
| T3-41 | T4-50 |
| T3-41 | T4-51 |
| T3-41 | T4-52 |
| T3-41 | T4-53 |

| | |
|---|---|
| T3-41 | T4-54 |
| T3-41 | T4-55 |
| T3-41 | T4-56 |
| T3-41 | T4-57 |
| T3-41 | T4-58 |
| T3-41 | T4-59 |
| T3-41 | T4-6 |
| T3-41 | T4-60 |
| T3-41 | T4-61 |
| T3-41 | T4-62 |
| T3-41 | T4-63 |
| T3-41 | T4-64 |
| T3-41 | T4-7 |
| T3-41 | T4-8 |
| T3-41 | T4-9 |
| T3-42 | T4-1 |
| T3-42 | T4-10 |
| T3-42 | T4-11 |
| T3-42 | T4-12 |
| T3-42 | T4-13 |
| T3-42 | T4-14 |
| T3-42 | T4-15 |
| T3-42 | T4-16 |
| T3-42 | T4-17 |
| T3-42 | T4-18 |
| T3-42 | T4-19 |
| T3-42 | T4-2 |
| T3-42 | T4-20 |
| T3-42 | T4-21 |
| T3-42 | T4-22 |
| T3-42 | T4-23 |
| T3-42 | T4-24 |
| T3-42 | T4-25 |
| T3-42 | T4-26 |
| T3-42 | T4-27 |
| T3-42 | T4-28 |
| T3-42 | T4-29 |
| T3-42 | T4-3 |
| T3-42 | T4-30 |
| T3-42 | T4-31 |
| T3-42 | T4-32 |
| T3-42 | T4-33 |
| T3-42 | T4-34 |
| T3-42 | T4-35 |
| T3-42 | T4-36 |

| | |
|---|---|
| T3-42 | T4-37 |
| T3-42 | T4-38 |
| T3-42 | T4-39 |
| T3-42 | T4-4 |
| T3-42 | T4-40 |
| T3-42 | T4-41 |
| T3-42 | T4-42 |
| T3-42 | T4-43 |
| T3-42 | T4-44 |
| T3-42 | T4-45 |
| T3-42 | T4-46 |
| T3-42 | T4-47 |
| T3-42 | T4-48 |
| T3-42 | T4-49 |
| T3-42 | T4-5 |
| T3-42 | T4-50 |
| T3-42 | T4-51 |
| T3-42 | T4-52 |
| T3-42 | T4-53 |
| T3-42 | T4-54 |
| T3-42 | T4-55 |
| T3-42 | T4-56 |
| T3-42 | T4-57 |
| T3-42 | T4-58 |
| T3-42 | T4-59 |
| T3-42 | T4-6 |
| T3-42 | T4-60 |
| T3-42 | T4-61 |
| T3-42 | T4-62 |
| T3-42 | T4-63 |
| T3-42 | T4-64 |
| T3-42 | T4-7 |
| T3-42 | T4-8 |
| T3-42 | T4-9 |
| T3-43 | T4-1 |
| T3-43 | T4-10 |
| T3-43 | T4-11 |
| T3-43 | T4-12 |
| T3-43 | T4-13 |
| T3-43 | T4-14 |
| T3-43 | T4-15 |
| T3-43 | T4-16 |
| T3-43 | T4-17 |
| T3-43 | T4-18 |
| T3-43 | T4-19 |

| | |
|---|---|
| T3-43 | T4-2 |
| T3-43 | T4-20 |
| T3-43 | T4-21 |
| T3-43 | T4-22 |
| T3-43 | T4-23 |
| T3-43 | T4-24 |
| T3-43 | T4-25 |
| T3-43 | T4-26 |
| T3-43 | T4-27 |
| T3-43 | T4-28 |
| T3-43 | T4-29 |
| T3-43 | T4-3 |
| T3-43 | T4-30 |
| T3-43 | T4-31 |
| T3-43 | T4-32 |
| T3-43 | T4-33 |
| T3-43 | T4-34 |
| T3-43 | T4-35 |
| T3-43 | T4-36 |
| T3-43 | T4-37 |
| T3-43 | T4-38 |
| T3-43 | T4-39 |
| T3-43 | T4-4 |
| T3-43 | T4-40 |
| T3-43 | T4-41 |
| T3-43 | T4-42 |
| T3-43 | T4-43 |
| T3-43 | T4-44 |
| T3-43 | T4-45 |
| T3-43 | T4-46 |
| T3-43 | T4-47 |
| T3-43 | T4-48 |
| T3-43 | T4-49 |
| T3-43 | T4-5 |
| T3-43 | T4-50 |
| T3-43 | T4-51 |
| T3-43 | T4-52 |
| T3-43 | T4-53 |
| T3-43 | T4-54 |
| T3-43 | T4-55 |
| T3-43 | T4-56 |
| T3-43 | T4-57 |
| T3-43 | T4-58 |
| T3-43 | T4-59 |
| T3-43 | T4-6 |

| | |
|---|---|
| T3-43 | T4-60 |
| T3-43 | T4-61 |
| T3-43 | T4-62 |
| T3-43 | T4-63 |
| T3-43 | T4-64 |
| T3-43 | T4-7 |
| T3-43 | T4-8 |
| T3-43 | T4-9 |
| T3-44 | T4-1 |
| T3-44 | T4-10 |
| T3-44 | T4-11 |
| T3-44 | T4-12 |
| T3-44 | T4-13 |
| T3-44 | T4-14 |
| T3-44 | T4-15 |
| T3-44 | T4-16 |
| T3-44 | T4-17 |
| T3-44 | T4-18 |
| T3-44 | T4-19 |
| T3-44 | T4-2 |
| T3-44 | T4-20 |
| T3-44 | T4-21 |
| T3-44 | T4-22 |
| T3-44 | T4-23 |
| T3-44 | T4-24 |
| T3-44 | T4-25 |
| T3-44 | T4-26 |
| T3-44 | T4-27 |
| T3-44 | T4-28 |
| T3-44 | T4-29 |
| T3-44 | T4-3 |
| T3-44 | T4-30 |
| T3-44 | T4-31 |
| T3-44 | T4-32 |
| T3-44 | T4-33 |
| T3-44 | T4-34 |
| T3-44 | T4-35 |
| T3-44 | T4-36 |
| T3-44 | T4-37 |
| T3-44 | T4-38 |
| T3-44 | T4-39 |
| T3-44 | T4-4 |
| T3-44 | T4-40 |
| T3-44 | T4-41 |
| T3-44 | T4-42 |

| | |
|---|---|
| T3-44 | T4-43 |
| T3-44 | T4-44 |
| T3-44 | T4-45 |
| T3-44 | T4-46 |
| T3-44 | T4-47 |
| T3-44 | T4-48 |
| T3-44 | T4-49 |
| T3-44 | T4-5 |
| T3-44 | T4-50 |
| T3-44 | T4-51 |
| T3-44 | T4-52 |
| T3-44 | T4-53 |
| T3-44 | T4-54 |
| T3-44 | T4-55 |
| T3-44 | T4-56 |
| T3-44 | T4-57 |
| T3-44 | T4-58 |
| T3-44 | T4-59 |
| T3-44 | T4-6 |
| T3-44 | T4-60 |
| T3-44 | T4-61 |
| T3-44 | T4-62 |
| T3-44 | T4-63 |
| T3-44 | T4-64 |
| T3-44 | T4-7 |
| T3-44 | T4-8 |
| T3-44 | T4-9 |
| T3-45 | T4-1 |
| T3-45 | T4-10 |
| T3-45 | T4-11 |
| T3-45 | T4-12 |
| T3-45 | T4-13 |
| T3-45 | T4-14 |
| T3-45 | T4-15 |
| T3-45 | T4-16 |
| T3-45 | T4-17 |
| T3-45 | T4-18 |
| T3-45 | T4-19 |
| T3-45 | T4-2 |
| T3-45 | T4-20 |
| T3-45 | T4-21 |
| T3-45 | T4-22 |
| T3-45 | T4-23 |
| T3-45 | T4-24 |
| T3-45 | T4-25 |

| | |
|---|---|
| T3-45 | T4-26 |
| T3-45 | T4-27 |
| T3-45 | T4-28 |
| T3-45 | T4-29 |
| T3-45 | T4-3 |
| T3-45 | T4-30 |
| T3-45 | T4-31 |
| T3-45 | T4-32 |
| T3-45 | T4-33 |
| T3-45 | T4-34 |
| T3-45 | T4-35 |
| T3-45 | T4-36 |
| T3-45 | T4-37 |
| T3-45 | T4-38 |
| T3-45 | T4-39 |
| T3-45 | T4-4 |
| T3-45 | T4-40 |
| T3-45 | T4-41 |
| T3-45 | T4-42 |
| T3-45 | T4-43 |
| T3-45 | T4-44 |
| T3-45 | T4-45 |
| T3-45 | T4-46 |
| T3-45 | T4-47 |
| T3-45 | T4-48 |
| T3-45 | T4-49 |
| T3-45 | T4-5 |
| T3-45 | T4-50 |
| T3-45 | T4-51 |
| T3-45 | T4-52 |
| T3-45 | T4-53 |
| T3-45 | T4-54 |
| T3-45 | T4-55 |
| T3-45 | T4-56 |
| T3-45 | T4-57 |
| T3-45 | T4-58 |
| T3-45 | T4-59 |
| T3-45 | T4-6 |
| T3-45 | T4-60 |
| T3-45 | T4-61 |
| T3-45 | T4-62 |
| T3-45 | T4-63 |
| T3-45 | T4-64 |
| T3-45 | T4-7 |
| T3-45 | T4-8 |

| T3-45 | T4-9 |
|---|---|
| T3-46 | T4-1 |
| T3-46 | T4-10 |
| T3-46 | T4-11 |
| T3-46 | T4-12 |
| T3-46 | T4-13 |
| T3-46 | T4-14 |
| T3-46 | T4-15 |
| T3-46 | T4-16 |
| T3-46 | T4-17 |
| T3-46 | T4-18 |
| T3-46 | T4-19 |
| T3-46 | T4-2 |
| T3-46 | T4-20 |
| T3-46 | T4-21 |
| T3-46 | T4-22 |
| T3-46 | T4-23 |
| T3-46 | T4-24 |
| T3-46 | T4-25 |
| T3-46 | T4-26 |
| T3-46 | T4-27 |
| T3-46 | T4-28 |
| T3-46 | T4-29 |
| T3-46 | T4-3 |
| T3-46 | T4-30 |
| T3-46 | T4-31 |
| T3-46 | T4-32 |
| T3-46 | T4-33 |
| T3-46 | T4-34 |
| T3-46 | T4-35 |
| T3-46 | T4-36 |
| T3-46 | T4-37 |
| T3-46 | T4-38 |
| T3-46 | T4-39 |
| T3-46 | T4-4 |
| T3-46 | T4-40 |
| T3-46 | T4-41 |
| T3-46 | T4-42 |
| T3-46 | T4-43 |
| T3-46 | T4-44 |
| T3-46 | T4-45 |
| T3-46 | T4-46 |
| T3-46 | T4-47 |
| T3-46 | T4-48 |
| T3-46 | T4-49 |

| T3-46 | T4-5 |
|---|---|
| T3-46 | T4-50 |
| T3-46 | T4-51 |
| T3-46 | T4-52 |
| T3-46 | T4-53 |
| T3-46 | T4-54 |
| T3-46 | T4-55 |
| T3-46 | T4-56 |
| T3-46 | T4-57 |
| T3-46 | T4-58 |
| T3-46 | T4-59 |
| T3-46 | T4-6 |
| T3-46 | T4-60 |
| T3-46 | T4-61 |
| T3-46 | T4-62 |
| T3-46 | T4-63 |
| T3-46 | T4-64 |
| T3-46 | T4-7 |
| T3-46 | T4-8 |
| T3-46 | T4-9 |
| T3-47 | T4-1 |
| T3-47 | T4-10 |
| T3-47 | T4-11 |
| T3-47 | T4-12 |
| T3-47 | T4-13 |
| T3-47 | T4-14 |
| T3-47 | T4-15 |
| T3-47 | T4-16 |
| T3-47 | T4-17 |
| T3-47 | T4-18 |
| T3-47 | T4-19 |
| T3-47 | T4-2 |
| T3-47 | T4-20 |
| T3-47 | T4-21 |
| T3-47 | T4-22 |
| T3-47 | T4-23 |
| T3-47 | T4-24 |
| T3-47 | T4-25 |
| T3-47 | T4-26 |
| T3-47 | T4-27 |
| T3-47 | T4-28 |
| T3-47 | T4-29 |
| T3-47 | T4-3 |
| T3-47 | T4-30 |
| T3-47 | T4-31 |

| T3-47 | T4-32 |
|---|---|
| T3-47 | T4-33 |
| T3-47 | T4-34 |
| T3-47 | T4-35 |
| T3-47 | T4-36 |
| T3-47 | T4-37 |
| T3-47 | T4-38 |
| T3-47 | T4-39 |
| T3-47 | T4-4 |
| T3-47 | T4-40 |
| T3-47 | T4-41 |
| T3-47 | T4-42 |
| T3-47 | T4-43 |
| T3-47 | T4-44 |
| T3-47 | T4-45 |
| T3-47 | T4-46 |
| T3-47 | T4-47 |
| T3-47 | T4-48 |
| T3-47 | T4-49 |
| T3-47 | T4-5 |
| T3-47 | T4-50 |
| T3-47 | T4-51 |
| T3-47 | T4-52 |
| T3-47 | T4-53 |
| T3-47 | T4-54 |
| T3-47 | T4-55 |
| T3-47 | T4-56 |
| T3-47 | T4-57 |
| T3-47 | T4-58 |
| T3-47 | T4-59 |
| T3-47 | T4-6 |
| T3-47 | T4-60 |
| T3-47 | T4-61 |
| T3-47 | T4-62 |
| T3-47 | T4-63 |
| T3-47 | T4-64 |
| T3-47 | T4-7 |
| T3-47 | T4-8 |
| T3-47 | T4-9 |
| T3-48 | T4-1 |
| T3-48 | T4-10 |
| T3-48 | T4-11 |
| T3-48 | T4-12 |
| T3-48 | T4-13 |
| T3-48 | T4-14 |

| T3-48 | T4-15 |
|---|---|
| T3-48 | T4-16 |
| T3-48 | T4-17 |
| T3-48 | T4-18 |
| T3-48 | T4-19 |
| T3-48 | T4-2 |
| T3-48 | T4-20 |
| T3-48 | T4-21 |
| T3-48 | T4-22 |
| T3-48 | T4-23 |
| T3-48 | T4-24 |
| T3-48 | T4-25 |
| T3-48 | T4-26 |
| T3-48 | T4-27 |
| T3-48 | T4-28 |
| T3-48 | T4-29 |
| T3-48 | T4-3 |
| T3-48 | T4-30 |
| T3-48 | T4-31 |
| T3-48 | T4-32 |
| T3-48 | T4-33 |
| T3-48 | T4-34 |
| T3-48 | T4-35 |
| T3-48 | T4-36 |
| T3-48 | T4-37 |
| T3-48 | T4-38 |
| T3-48 | T4-39 |
| T3-48 | T4-4 |
| T3-48 | T4-40 |
| T3-48 | T4-41 |
| T3-48 | T4-42 |
| T3-48 | T4-43 |
| T3-48 | T4-44 |
| T3-48 | T4-45 |
| T3-48 | T4-46 |
| T3-48 | T4-47 |
| T3-48 | T4-48 |
| T3-48 | T4-49 |
| T3-48 | T4-5 |
| T3-48 | T4-50 |
| T3-48 | T4-51 |
| T3-48 | T4-52 |
| T3-48 | T4-53 |
| T3-48 | T4-54 |
| T3-48 | T4-55 |

| | |
|---|---|
| T3-48 | T4-56 |
| T3-48 | T4-57 |
| T3-48 | T4-58 |
| T3-48 | T4-59 |
| T3-48 | T4-6 |
| T3-48 | T4-60 |
| T3-48 | T4-61 |
| T3-48 | T4-62 |
| T3-48 | T4-63 |
| T3-48 | T4-64 |
| T3-48 | T4-7 |
| T3-48 | T4-8 |
| T3-48 | T4-9 |
| T3-49 | T4-1 |
| T3-49 | T4-10 |
| T3-49 | T4-11 |
| T3-49 | T4-12 |
| T3-49 | T4-13 |
| T3-49 | T4-14 |
| T3-49 | T4-15 |
| T3-49 | T4-16 |
| T3-49 | T4-17 |
| T3-49 | T4-18 |
| T3-49 | T4-19 |
| T3-49 | T4-2 |
| T3-49 | T4-20 |
| T3-49 | T4-21 |
| T3-49 | T4-22 |
| T3-49 | T4-23 |
| T3-49 | T4-24 |
| T3-49 | T4-25 |
| T3-49 | T4-26 |
| T3-49 | T4-27 |
| T3-49 | T4-28 |
| T3-49 | T4-29 |
| T3-49 | T4-3 |
| T3-49 | T4-30 |
| T3-49 | T4-31 |
| T3-49 | T4-32 |
| T3-49 | T4-33 |
| T3-49 | T4-34 |
| T3-49 | T4-35 |
| T3-49 | T4-36 |
| T3-49 | T4-37 |
| T3-49 | T4-38 |

| | |
|---|---|
| T3-49 | T4-39 |
| T3-49 | T4-4 |
| T3-49 | T4-40 |
| T3-49 | T4-41 |
| T3-49 | T4-42 |
| T3-49 | T4-43 |
| T3-49 | T4-44 |
| T3-49 | T4-45 |
| T3-49 | T4-46 |
| T3-49 | T4-47 |
| T3-49 | T4-48 |
| T3-49 | T4-49 |
| T3-49 | T4-5 |
| T3-49 | T4-50 |
| T3-49 | T4-51 |
| T3-49 | T4-52 |
| T3-49 | T4-53 |
| T3-49 | T4-54 |
| T3-49 | T4-55 |
| T3-49 | T4-56 |
| T3-49 | T4-57 |
| T3-49 | T4-58 |
| T3-49 | T4-59 |
| T3-49 | T4-6 |
| T3-49 | T4-60 |
| T3-49 | T4-61 |
| T3-49 | T4-62 |
| T3-49 | T4-63 |
| T3-49 | T4-64 |
| T3-49 | T4-7 |
| T3-49 | T4-8 |
| T3-49 | T4-9 |
| T3-5 | T4-1 |
| T3-5 | T4-10 |
| T3-5 | T4-11 |
| T3-5 | T4-12 |
| T3-5 | T4-13 |
| T3-5 | T4-14 |
| T3-5 | T4-15 |
| T3-5 | T4-16 |
| T3-5 | T4-17 |
| T3-5 | T4-18 |
| T3-5 | T4-19 |
| T3-5 | T4-2 |
| T3-5 | T4-20 |

| | |
|---|---|
| T3-5 | T4-21 |
| T3-5 | T4-22 |
| T3-5 | T4-23 |
| T3-5 | T4-24 |
| T3-5 | T4-25 |
| T3-5 | T4-26 |
| T3-5 | T4-27 |
| T3-5 | T4-28 |
| T3-5 | T4-29 |
| T3-5 | T4-3 |
| T3-5 | T4-30 |
| T3-5 | T4-31 |
| T3-5 | T4-32 |
| T3-5 | T4-33 |
| T3-5 | T4-34 |
| T3-5 | T4-35 |
| T3-5 | T4-36 |
| T3-5 | T4-37 |
| T3-5 | T4-38 |
| T3-5 | T4-39 |
| T3-5 | T4-4 |
| T3-5 | T4-40 |
| T3-5 | T4-41 |
| T3-5 | T4-42 |
| T3-5 | T4-43 |
| T3-5 | T4-44 |
| T3-5 | T4-45 |
| T3-5 | T4-46 |
| T3-5 | T4-47 |
| T3-5 | T4-48 |
| T3-5 | T4-49 |
| T3-5 | T4-5 |
| T3-5 | T4-50 |
| T3-5 | T4-51 |
| T3-5 | T4-52 |
| T3-5 | T4-53 |
| T3-5 | T4-54 |
| T3-5 | T4-55 |
| T3-5 | T4-56 |
| T3-5 | T4-57 |
| T3-5 | T4-58 |
| T3-5 | T4-59 |
| T3-5 | T4-6 |
| T3-5 | T4-60 |
| T3-5 | T4-61 |

| | |
|---|---|
| T3-5 | T4-62 |
| T3-5 | T4-63 |
| T3-5 | T4-64 |
| T3-5 | T4-7 |
| T3-5 | T4-8 |
| T3-5 | T4-9 |
| T3-50 | T4-1 |
| T3-50 | T4-10 |
| T3-50 | T4-11 |
| T3-50 | T4-12 |
| T3-50 | T4-13 |
| T3-50 | T4-14 |
| T3-50 | T4-15 |
| T3-50 | T4-16 |
| T3-50 | T4-17 |
| T3-50 | T4-18 |
| T3-50 | T4-19 |
| T3-50 | T4-2 |
| T3-50 | T4-20 |
| T3-50 | T4-21 |
| T3-50 | T4-22 |
| T3-50 | T4-23 |
| T3-50 | T4-24 |
| T3-50 | T4-25 |
| T3-50 | T4-26 |
| T3-50 | T4-27 |
| T3-50 | T4-28 |
| T3-50 | T4-29 |
| T3-50 | T4-3 |
| T3-50 | T4-30 |
| T3-50 | T4-31 |
| T3-50 | T4-32 |
| T3-50 | T4-33 |
| T3-50 | T4-34 |
| T3-50 | T4-35 |
| T3-50 | T4-36 |
| T3-50 | T4-37 |
| T3-50 | T4-38 |
| T3-50 | T4-39 |
| T3-50 | T4-4 |
| T3-50 | T4-40 |
| T3-50 | T4-41 |
| T3-50 | T4-42 |
| T3-50 | T4-43 |
| T3-50 | T4-44 |

| | |
|---|---|
| T3-50 | T4-45 |
| T3-50 | T4-46 |
| T3-50 | T4-47 |
| T3-50 | T4-48 |
| T3-50 | T4-49 |
| T3-50 | T4-5 |
| T3-50 | T4-50 |
| T3-50 | T4-51 |
| T3-50 | T4-52 |
| T3-50 | T4-53 |
| T3-50 | T4-54 |
| T3-50 | T4-55 |
| T3-50 | T4-56 |
| T3-50 | T4-57 |
| T3-50 | T4-58 |
| T3-50 | T4-59 |
| T3-50 | T4-6 |
| T3-50 | T4-60 |
| T3-50 | T4-61 |
| T3-50 | T4-62 |
| T3-50 | T4-63 |
| T3-50 | T4-64 |
| T3-50 | T4-7 |
| T3-50 | T4-8 |
| T3-50 | T4-9 |
| T3-6 | T4-1 |
| T3-6 | T4-10 |
| T3-6 | T4-11 |
| T3-6 | T4-12 |
| T3-6 | T4-13 |
| T3-6 | T4-14 |
| T3-6 | T4-15 |
| T3-6 | T4-16 |
| T3-6 | T4-17 |
| T3-6 | T4-18 |
| T3-6 | T4-19 |
| T3-6 | T4-2 |
| T3-6 | T4-20 |
| T3-6 | T4-21 |
| T3-6 | T4-22 |
| T3-6 | T4-23 |
| T3-6 | T4-24 |
| T3-6 | T4-25 |
| T3-6 | T4-26 |
| T3-6 | T4-27 |

| | |
|---|---|
| T3-6 | T4-28 |
| T3-6 | T4-29 |
| T3-6 | T4-3 |
| T3-6 | T4-30 |
| T3-6 | T4-31 |
| T3-6 | T4-32 |
| T3-6 | T4-33 |
| T3-6 | T4-34 |
| T3-6 | T4-35 |
| T3-6 | T4-36 |
| T3-6 | T4-37 |
| T3-6 | T4-38 |
| T3-6 | T4-39 |
| T3-6 | T4-4 |
| T3-6 | T4-40 |
| T3-6 | T4-41 |
| T3-6 | T4-42 |
| T3-6 | T4-43 |
| T3-6 | T4-44 |
| T3-6 | T4-45 |
| T3-6 | T4-46 |
| T3-6 | T4-47 |
| T3-6 | T4-48 |
| T3-6 | T4-49 |
| T3-6 | T4-5 |
| T3-6 | T4-50 |
| T3-6 | T4-51 |
| T3-6 | T4-52 |
| T3-6 | T4-53 |
| T3-6 | T4-54 |
| T3-6 | T4-55 |
| T3-6 | T4-56 |
| T3-6 | T4-57 |
| T3-6 | T4-58 |
| T3-6 | T4-59 |
| T3-6 | T4-6 |
| T3-6 | T4-60 |
| T3-6 | T4-61 |
| T3-6 | T4-62 |
| T3-6 | T4-63 |
| T3-6 | T4-64 |
| T3-6 | T4-7 |
| T3-6 | T4-8 |
| T3-6 | T4-9 |
| T3-7 | T4-1 |

| | |
|---|---|
| T3-7 | T4-10 |
| T3-7 | T4-11 |
| T3-7 | T4-12 |
| T3-7 | T4-13 |
| T3-7 | T4-14 |
| T3-7 | T4-15 |
| T3-7 | T4-16 |
| T3-7 | T4-17 |
| T3-7 | T4-18 |
| T3-7 | T4-19 |
| T3-7 | T4-2 |
| T3-7 | T4-20 |
| T3-7 | T4-21 |
| T3-7 | T4-22 |
| T3-7 | T4-23 |
| T3-7 | T4-24 |
| T3-7 | T4-25 |
| T3-7 | T4-26 |
| T3-7 | T4-27 |
| T3-7 | T4-28 |
| T3-7 | T4-29 |
| T3-7 | T4-3 |
| T3-7 | T4-30 |
| T3-7 | T4-31 |
| T3-7 | T4-32 |
| T3-7 | T4-33 |
| T3-7 | T4-34 |
| T3-7 | T4-35 |
| T3-7 | T4-36 |
| T3-7 | T4-37 |
| T3-7 | T4-38 |
| T3-7 | T4-39 |
| T3-7 | T4-4 |
| T3-7 | T4-40 |
| T3-7 | T4-41 |
| T3-7 | T4-42 |
| T3-7 | T4-43 |
| T3-7 | T4-44 |
| T3-7 | T4-45 |
| T3-7 | T4-46 |
| T3-7 | T4-47 |
| T3-7 | T4-48 |
| T3-7 | T4-49 |
| T3-7 | T4-5 |
| T3-7 | T4-50 |

| | |
|---|---|
| T3-7 | T4-51 |
| T3-7 | T4-52 |
| T3-7 | T4-53 |
| T3-7 | T4-54 |
| T3-7 | T4-55 |
| T3-7 | T4-56 |
| T3-7 | T4-57 |
| T3-7 | T4-58 |
| T3-7 | T4-59 |
| T3-7 | T4-6 |
| T3-7 | T4-60 |
| T3-7 | T4-61 |
| T3-7 | T4-62 |
| T3-7 | T4-63 |
| T3-7 | T4-64 |
| T3-7 | T4-7 |
| T3-7 | T4-8 |
| T3-7 | T4-9 |
| T3-8 | T4-1 |
| T3-8 | T4-10 |
| T3-8 | T4-11 |
| T3-8 | T4-12 |
| T3-8 | T4-13 |
| T3-8 | T4-14 |
| T3-8 | T4-15 |
| T3-8 | T4-16 |
| T3-8 | T4-17 |
| T3-8 | T4-18 |
| T3-8 | T4-19 |
| T3-8 | T4-2 |
| T3-8 | T4-20 |
| T3-8 | T4-21 |
| T3-8 | T4-22 |
| T3-8 | T4-23 |
| T3-8 | T4-24 |
| T3-8 | T4-25 |
| T3-8 | T4-26 |
| T3-8 | T4-27 |
| T3-8 | T4-28 |
| T3-8 | T4-29 |
| T3-8 | T4-3 |
| T3-8 | T4-30 |
| T3-8 | T4-31 |
| T3-8 | T4-32 |
| T3-8 | T4-33 |

| T3-8 | T4-34 |
|---|---|
| T3-8 | T4-35 |
| T3-8 | T4-36 |
| T3-8 | T4-37 |
| T3-8 | T4-38 |
| T3-8 | T4-39 |
| T3-8 | T4-4 |
| T3-8 | T4-40 |
| T3-8 | T4-41 |
| T3-8 | T4-42 |
| T3-8 | T4-43 |
| T3-8 | T4-44 |
| T3-8 | T4-45 |
| T3-8 | T4-46 |
| T3-8 | T4-47 |
| T3-8 | T4-48 |
| T3-8 | T4-49 |
| T3-8 | T4-5 |
| T3-8 | T4-50 |
| T3-8 | T4-51 |
| T3-8 | T4-52 |
| T3-8 | T4-53 |
| T3-8 | T4-54 |
| T3-8 | T4-55 |
| T3-8 | T4-56 |
| T3-8 | T4-57 |

| T3-8 | T4-58 |
|---|---|
| T3-8 | T4-59 |
| T3-8 | T4-6 |
| T3-8 | T4-60 |
| T3-8 | T4-61 |
| T3-8 | T4-62 |
| T3-8 | T4-63 |
| T3-8 | T4-64 |
| T3-8 | T4-7 |
| T3-8 | T4-8 |
| T3-8 | T4-9 |
| T3-9 | T4-1 |
| T3-9 | T4-10 |
| T3-9 | T4-11 |
| T3-9 | T4-12 |
| T3-9 | T4-13 |
| T3-9 | T4-14 |
| T3-9 | T4-15 |
| T3-9 | T4-16 |
| T3-9 | T4-17 |
| T3-9 | T4-18 |
| T3-9 | T4-19 |
| T3-9 | T4-2 |
| T3-9 | T4-20 |
| T3-9 | T4-21 |
| T3-9 | T4-22 |

| T3-9 | T4-23 |
|---|---|
| T3-9 | T4-24 |
| T3-9 | T4-25 |
| T3-9 | T4-26 |
| T3-9 | T4-27 |
| T3-9 | T4-28 |
| T3-9 | T4-29 |
| T3-9 | T4-3 |
| T3-9 | T4-30 |
| T3-9 | T4-31 |
| T3-9 | T4-32 |
| T3-9 | T4-33 |
| T3-9 | T4-34 |
| T3-9 | T4-35 |
| T3-9 | T4-36 |
| T3-9 | T4-37 |
| T3-9 | T4-38 |
| T3-9 | T4-39 |
| T3-9 | T4-4 |
| T3-9 | T4-40 |
| T3-9 | T4-41 |
| T3-9 | T4-42 |
| T3-9 | T4-43 |
| T3-9 | T4-44 |
| T3-9 | T4-45 |
| T3-9 | T4-46 |

| T3-9 | T4-47 |
|---|---|
| T3-9 | T4-48 |
| T3-9 | T4-49 |
| T3-9 | T4-5 |
| T3-9 | T4-50 |
| T3-9 | T4-51 |
| T3-9 | T4-52 |
| T3-9 | T4-53 |
| T3-9 | T4-54 |
| T3-9 | T4-55 |
| T3-9 | T4-56 |
| T3-9 | T4-57 |
| T3-9 | T4-58 |
| T3-9 | T4-59 |
| T3-9 | T4-6 |
| T3-9 | T4-60 |
| T3-9 | T4-61 |
| T3-9 | T4-62 |
| T3-9 | T4-63 |
| T3-9 | T4-64 |
| T3-9 | T4-7 |
| T3-9 | T4-8 |
| T3-9 | T4-9 |

One embodiment according the present invention refers to a transgenic plant, transgenic plant parts, transgenic plant cells and/or transgenic plant propagation materials obtainable by the method for controlling fungal infection and/or increasing plant health as decribed in this application.

Synergistic effect according to the present invention means that

a) the use of a fungicide as defined above in combination with treating transgenic plants, transgenic plant parts, transgenic plant cells, transgenic plant propagation materials and/or their locus of growth as defined above exceeds the additive effect of the range of action of the fungicide and exogenous gene expressed by the transgenic plants, transgenic plant parts, transgenic plant cells, transgenic plant propagation materials and/or

b) results in an increased plant health effect in such transgenic plants compared to the plant health effects that are possible with the fungicide, if applied to the non-transgenic plant.

Thus, the term "synergistic effect", however, is to be understood in this connection as synergistic fungicidal activity and/or synergistic plant health effects.

The additive effect may be calculated as follows:

% reduction of fungal infection is measured in the transgenic plant relative to the fungal infection in the wildtype plant (100%) =A

Furthermore

% reduction of fungal infection is measured in the wildtype plant treated with fungicide relative to the fungal infection in the untreated wildtype plant (100%) =B

Additive effect is a % reduction of fungal infection greater A but not greater than:

$$A + (100\% - A) \times B/ 100\%$$

Example

Transgenic plant: 60% reduction of fungal infection compared to wildtype plant = A Remaining fungal infection: 40% (100% - A) Wildtype plant treated with fungicide: 50% reduction of fungal infection = B

$$\text{Effect on remaining fungal infection } 20\% = (100\% - A) \times B/ 100\%$$

Additive effect:

$$60\% + (100\% - 60\%) \times 50\%/ 100\% = 80\%$$

Addtive effect: reduction of fungal infection greater than 50% but not more than 80%.

A synergistic effect exceeds the additive effect.

Alternatively a synergistic effect may be a combination of a reduction of fungal infection observed in the transgenic plant without fungicide treatment and an increased effect on plant health observed in the transgenic plant with fungicide treatment which is not present without fungicide treatment.

The invention relates to a method for improving plant health by treating transgenic plants, transgenic plant parts, transgenic plant cells, transgenic plant propagation materials and/or the locus where the plants is growing and/or at the locus where they are to grow with an effective amount of fungicides.

The application can be carried out both before and after the infection of the plants, plant propagation materials, such as seeds, soil, surfaces, materials or rooms by the fungi.

Plant propagation materials may be treated with fungicides prophylactically either at or before planting or transplanting.

The method of treatment according to the invention can also be used in the field of protecting stored products or harvest against attack of fungi. According to the present invention, the term "stored products" is understood to denote natural substances of plant and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as predried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Transgenic plants, transgenic plants parts, transgenic plants cells, transgenic plant propagation material treated with a fungicide can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

In one embodiment of the present invention the transgenic plant shows herbicide tolerance compared to the wildtype plant. In particular, the transgenic plant is a plant, which is tolerant to the action of herbicides selected form the group consisting of glyphosate, glufosinate, imidazolinone-herbidicides a, dicamba or combinations thereof.

The transgenic plants optionally comprises at least one trait for herbicide tolerance. The term "trait" refers to a property, which is present in the plant either by genetic enginieering or by conventional breeding techniques. In the context of herbicide tolerance the definition wildtype refers to a transgenic plants, transgenic plant parts, transgenic plant cells and/or transgenic plant propagation materials which has not been modified by genetic enginieering or by conventional breeding techniques to obtain herbicide tolerance.

Specific examples for herbicide tolerance are glyphosate tolerance, glufosinate tolerance, sulfonylurea tolerance, imidazolinone tolerance, resistance against 2,4-D Choline, dicamba tolerance, glyphosate & dicamba tolerance, HPPD inhibitor resistance, oxynil herbicide tolerance (e.g. bromoxynil), cyclohexanone herbicide tolerance (e.g. sethoxydim).

Tolerance to herbicides can be obtained by creating insensitivity at the site of action of the herbicide by expression of a target enzyme which is resistant to herbicide; rapid metabolism (conjugation or degradation) of the herbicide by expression of enzymes which inactivate herbicide; or poor uptake and translocation of the herbicide. Examples are the expression of enzymes which are tolerant to the herbicide in comparison to wild type enzymes, such as the expression of 5-enolpyruvylshikimate-3-phosphate synthase (EPSPS), which is tolerant to glyphosate (see e.g. Heck et.al, Crop Sci. 45, 2005, 329-339; Funke et.al, PNAS 103, 2006, 13010-13015; US5188642, US4940835, US5633435, US5804425, US5627061), the expression of glutamine synthase which is tolerant to glufosinate and bialaphos (see e.g. US5646024, US5561236) and DNA constructs coding for dicamba-degrading enzymes (see e.g. US7105724). Gene constructs can be obtained, for example, from micro-organism or plants, which are tolerant to said herbicides, such as the Agrobacterium strain CP4 EPSPS which is resistant to glyphosate; Streptomyces bacteria which are resistance to glufosinate; Arabidopsis, Daucus carotte, Pseudomonoas sp. or Zea mais with chimeric gene sequences coging for HDDP (see e.g. WO1996/38567, WO 2004/55191); Arabidopsis thaliana which is resistant to protox inhibitors (see e.g. US2002/0073443).

Examples of commercial available transgenic plants with tolerance to herbicides, are the corn varieties "Roundup Ready Corn", "Roundup Ready 2" (Monsanto), "Agrisure GT", "Agrisure GT/CB/LL", "Agrisure GT/RW", "Agrisure 3000GT" (Syngenta), "YieldGard VT Rootworm/RR2" and "YieldGard VT Triple" (Monsanto) with tolerance to glyphosate; the corn varieties "Liberty Link" (Bayer), "Herculex I", "Herculex RW", "Herculex Xtra"(Dow, Pioneer), "Agrisure GT/CB/LL" and "Agrisure CB/LL/RW" (Syngenta) with tolerance to glufosinate; the soybean varieties "Roundup Ready Soybean" (Monsanto) and "Optimum GAT" (DuPont, Pioneer) with tolerance to glyphosate; the cotton varieties "Roundup Ready Cotton" and "Roundup Ready Flex" (Monsanto) with tolerance to glyphosate; the cotton variety "FiberMax Liberty Link" (Bayer) with tolerance to glufosinate; the cotton variety "BXN" (Calgene) with tolerance to bromoxynil; the canola varieties "Navigator" und "Compass" (Rhone-Poulenc) with bromoxynil tolerance; the canola varierty"Roundup Ready Canola" (Monsanto) with glyphosate tolerance; the canola variety "InVigor" (Bayer) with glufosinate tolerance; the rice variety "Liberty Link Rice" (Bayer) with glulfosinate tolerance and the alfalfa variety "Roundup Ready Alfalfa" with glyphosate tolerance. Further transgenic plants with herbicide are commonly known, for instance alfalfa, apple, eucalyptus, flax, grape, lentils, oil seed rape, peas, potato, rice, sugar beet, sunflower, tobacco, tomatom turf grass and wheat with tolerance to glyphosate (see e.g. US5188642, US4940835, US5633435, US5804425, US5627061); beans, soybean, cotton, peas, potato, sunflower, tomato, tobacco, corn, sorghum and sugarcane with tolerance to dicamba (see e.g. US7105724 and US5670454); pepper, apple, tomato, hirse, sunflower, tobacco, potato, corn, cucumber, wheat and sorghum with tolerance to 2,4-D (see e.g. US6153401, US6100446, WO2005107437, US5608147 and US5670454); sugarbeet, potato, tomato and tobacco with tolerance to gluphosinate (see e.g. US5646024, US5561236); canola, barley, cotton, lettuce, melon, millet, oats, potato, rice, rye, sorghum, soybean, sugarbeet, sunflower, tobacco, tomato and wheat with tolerance to acetolactate synthase (ALS) inhibiting herbicides, such as triazolopyrimidine sulfonamides, sulfonylureas and imidazolinones (see e.g. US5013659, WO2006060634, US4761373, US5304732, US6211438, US6211439 and US6222100); cereal, sugarcane, rice, corn, tobacco, soybean, cotton, rapeseed, sugar beet and potato with tolerance to HPPD inhibitor herbicides (see e.g. WO2004/055191, WO199638567, WO1997049816 and US6791014); wheat, soybean, cotton, sugar beet, rape, rice, sorghum and sugar cane with tolerance to protoporphyrinogen oxidase (PPO) inhibitor herbicides (see e.g. US2002/0073443, US20080052798, Pest Management Science, 61, 2005, 277-285). The methods of producing such transgenic plants are generally known to the person skilled in the art and are described, for example, in the publications mentioned above.

Thus, in one embodiment, the present invention relates to a method of controlling fungal infection and/or increasing the health of transgenic plants, parts of such transgenic plants, transgenic plant cells, transgenic plant propagation materials by treating transgenic plants, parts of such transgenic plants, transgenic plant cells, transgenic plant propagation materials, or at their locus of growth and/or at the locus where they are to grow with a fungicide as defined above, wherein the plant is a plant, which is rendered tolerant to herbicides, more preferably to herbicides such as glutamine synthetase inhibitors, 5-enol-pyrovyl-shikimate-3-phosphate-synthase inhibitors, acetolactate synthase (ALS) inhibitors, protoporphyrinogen oxidase (PPO) inhibitors, auxine type herbicides, most preferably to herbicides such as glyphosate, glufosinate, imazapyr, imazapic, imazamox, imazethapyr, imazaquin, imazamethabenz methyl, dicamba, 2,4-D and combinations thereof.

The fungicide as defined above may also be converted into agrochemical compositions comprising a solvent or solid carrier and at least one of the fungicides according to the present invention.

An agrochemical composition comprises a fungicidally effective amount of a fungicide. The term "effective amount" denotes an amount of the composition or of the fungicide, which is sufficient for controlling fungi on transgenic plants, transgenic plants parts, transgenic plant cells, transgenic plant propagation materials and/or their locus of growth and/or at the locus where they are to grow and which does not result in a substantial damage to the treated plants. Such an amount can vary in a broad range and is dependent on various factors, such as the fungal species to be controlled, the treated cultivated plant or material, the climatic conditions and the fungicide used.

The fungicides, optionally their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the fungicide on the target. Examples are surfactants, mineral or vegetable oils, and other auxiliaries. Further examples are listed by Knowles, Adjuvants and additives, Agrow Reports DS256, T&F Informa UK, 2006, chapter 5. Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:

i) Water-soluble concentrates (SL, LS) 10-60 wt% of a fungicide and 5-15 wt% wetting agent (e. g. alcohol alkoxylates) are dissolved in water and/or in a water-soluble solvent (e. g. alcohols) ad 100 wt%. The active substance dissolves upon dilution with water.

ii) Dispersible concentrates (DC) 5-25 wt% of a fungicide and 1-10 wt% dispersant (e. g. polyvinyl pyrrolidone) are dissolved in organic solvent (e. g. cyclohexanone) ad 100 wt%. Dilution with water gives a dispersion.

iii) Emulsifiable concentrates (EC) 15-70 wt% of a fungicide and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.

iv) Emulsions (EW, EO, ES) 5-40 wt% of a fungicide and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.

v) Suspensions (SC, OD, FS) In an agitated ball mill, 20-60 wt% of a fungicide are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.

vi) Water-dispersible granules and water-soluble granules (WG, SG) 50-80 wt% of a fungicide are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

vii) Water-dispersible powders and water-soluble powders (WP, SP, WS) 50-80 wt% of a fungicide are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.

viii) Gel (GW, GF) In an agitated ball mill, 5-25 wt% of a fungicide are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.

ix) Microemulsion (ME) 5-20 wt% of a fungicide are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.

x) Microcapsules (CS) An oil phase comprising 5-50 wt% of a fungicide, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of fungicide, 0-40 wt% water insoluble organic solvent (e. g. aromatic hydrocarbon), and an isocyanate monomer (e. g. diphenyl-methene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.

xi) Dustable powders (DP, DS) 1-10 wt% of a fungicide are ground finely and mixed intimately with solid carrier (e. g. finely divided kaolin) ad 100 wt%.

xii) Granules (GR, FG) 0.5-30 wt% of a fungicide is ground finely and associated with solid carrier (e. g. silicate) ad 100 wt%. Granulation is achieved by extrusion, spray-drying or fluidized bed.

xiii) Ultra-low volume liquids (UL) 1-50 wt% of a fungicide are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying fungicide and compositions thereof, respectively, onto plant propagation material, especially seeds, include dressing, coating, pelleting, dusting, and soaking as well as in-furrow application methods. Preferably, fungicide or the compositions thereof, respectively, are applied on to the plant propagation material by a method such that germination is not induced, e. g. by seed dressing, pelleting, coating and dusting.

In a further embodiment, a suspension-type (FS) composition is used for seed treatment. Typcially, a FS composition may comprise 1-800 g/l of active substance, 1-200 g/l Surfactant, 0 to 200 g/l antifreezing agent, 0 to 400 g/l of binder, 0 to 200 g/l of a pigment and up to 1 liter of a solvent, preferably water.

The the fungicides according to the present invention can be used as such or in the form of their compositions, e. g. in the form of directly sprayable solutions, powders, suspensions, dispersions, emulsions, oil dispersions, pastes, dustable products, materials for spreading, or granules, by means of spraying, atomizing, dusting, spreading, brushing, immersing or pouring. The application forms depend entirely on the intended purposes; it is intended to ensure in each case the finest possible distribution of the fungicide(s) as defined pursuant to the present invention.

Aqueous application forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of fungicide(s), wetter, tackifier, dispersant or emulsifier and, if appropriate, solvent or oil, and such concentrates are suitable for dilution with water.

The fungicide(s) concentrations in the ready-to-use preparations can be varied within relatively wide ranges. In general, they are from 0.0001 to 10%, preferably from 0.001 to 1 % by weight of fungicide(s).

The fungicide(s)may also be used successfully in the ultra-low-volume process (ULV), it being possible to apply compositions comprising over 95% by weight of fungicide(s) or even to apply the fungicide(s) without additives.

The amounts of fungicide(s) applied are, depending on the kind of effect desired, from 0.001 to 2 kg per ha, preferably from 0.005 to 2 kg per ha, more preferably from 0.05 to 0.9 kg per ha, in particular from 0.1 to 0.75 kg per ha.

In treatment of plant propagation materials such as seeds, e. g. by dusting, coating or drenching seed, amounts of fungicide(s) of from 0.1 to 1000 g, preferably from 1 to 1000 g, more preferably from 1 to 100 g and most preferably from 5 to 100 g, per 100 kilogram of plant propagation material (preferably seed) are generally required.

Various types of oils, wetters, adjuvants, herbicides, bactericides, and/or pesticides may be added to the fungicide(s)or the compositions comprising them, if appropriate not until immediately prior to use (tank mix). These agents can be admixed with the compositions according to the invention in a weight ratio of 1:100 to 100:1, preferably 1:10 to 10:1.

Adjuvants which can be used are in particular organic modified polysiloxanes such as Break Thru S 240®; alcohol alkoxylates such as Atplus 245®, Atplus MBA 1303®, Plurafac LF 300® and Lutensol ON 30®; EO/PO block polymers, e. g. Pluronic RPE 2035® and Genapol B®; alcohol ethoxylates such as Lutensol XP 80®; and dioctyl sulfosuccinate sodium such as Leophen RA®.

The fungicides used according to the invention can also be present together with other active substances, e. g. with herbicides, insecticides, growth regulators, biopesticides or else with fertilizers, as pre-mix or, if appropriate, not until immediately prior to use (tank mix). Especially suitable compositions and mixtures that can be used according to the present invention and mixing partners or further active ingredients that may be preferably present together with any one of fungicides T4-1to T4-64 as defined above, are detailled in patent application WO 2014/095994. In WO2014/095932 further mixtures are disclosed.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

In particular, soybean rust resistant transgenic soybean events are grown in the field under conditions as already known for currently available conventional and transgenic (e.g. fungal resistant and/or herbicide resistant) soybean varieties. Exact conditions depend on region, climate and genetic background of the soybean rust resistant events. Farmers and other soybean experts know the exact conditions that are required to optimally cultivate soybean plants in their region. So all numbers and details given here are only exemplary for a specific region, specific soil type and specific genetic background (i.e. maturity group).

In one embodiment soybean seeds are planted 4 cm deep into the prepared soil with a density of 260.000 plants/ha (12-14 seeds/m, row distance 50cm). The preparation of the soil (e.g. tilling, strip-till or no-till) depends on region and operational procedures and strategies of each farmer. Planting is preferably performed either in late spring (first season) or in summer for a second season as soon as harvesting of the previous crop allows. The exact planting timepoint is dependent on region and weather conditions, such as temperature and moisture. Fertilization (e.g. 200-300 kg fertilizer/ha of a 0-20-20 (N-P-K) fertilizer) and other plant protection treatments, such as herbicide treatment and/or insecticide treatment are performed as required in the particular region.
Fungicide are applied depending on disease occurrence and progression. In practice also webbased disease forecasting/prediction software is used.

In one embodiment in average 3 fungicide treatments are applied. All plant protective treatments are performed according to the description provided by the supplier, i.e. in terms of used concentrations and mixtures with other fungicides, herbicides, insecticide or nematocides. The first fungicide treatment is generally applied 40-45 days after planting or, if required, when the disease is scouted earlier. In general the second and third treatment are applied in a -15 days interval after the first treatment. The first and second treatment are generally targeting soybean rust disease only, whereas the third treatment partially also targets other late-season diseases. Preferably, it should be avoided to use the same fungicide multiple times in one growing season or to use fungicides which consist of a single active compound to inhibit (or delay) the development of fungicide resistance. An exemplary scheme of fungicide treatment can be found in the Table 6.

Table 6

| Treatment | Product | Active compounds | Concentration |
|---|---|---|---|
| 1. | Elatus | Azoxystrobin + Benzovindiflupyr | 300 g/ha |
| 2. | Fox | Prothiconazole + Trifloxystrobin | 400 ml/ha |
| 3. | Aproach Prima | Cyproconazole + Picoxystrobin | 300 ml/ha |
| Seed treatment can be made into the seedbox before planting into the field. | | | |

For seed treatment purposes, the weight ratio in the binary, ternary and quaternary mixtures of the present invention generally depends from the properties of the fungicides according to the present invention.

Compositions, which are especially useful for seed treatment are e.g.:

A Soluble concentrates (SL, LS)
D Emulsions (EW, EO, ES)
E Suspensions (SC, OD, FS)
F Water-dispersible granules and water-soluble granules (WG, SG)
G Water-dispersible powders and water-soluble powders (WP, SP, WS)
H Gel-Formulations (GF)
I Dustable powders (DP, DS)

These compositions can be applied to transgenic plant propagation materials, particularly seeds, diluted or undiluted. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying or treating agrochemical compounds and compositions thereof, respectively, on to transgenic plant propagation material, especially seeds, are known in the art, and include dressing, coating, pelleting, dusting and soaking application methods of the transgenic propagation material (and also in furrow treatment). In a preferred embodiment, the compounds or the compositions thereof, respectively, are applied on to the transgenic plant propagation material by a method such that germination is not induced, e. g. by seed dressing, pelleting, coating and dusting.

In the treatment of transgenic plant propagation material (preferably seed), the application rates of the mixture are generally for the formulated product (which usually comprises from10 to 750 g/l of the fungicide(s)

The method for controlling fungal infection and/or increasing plant health according to the present invention also relates to the propagation products of transgenic plants, and especially the transgenic seed comprising, that is, coated with and/or containing, a mixture as defined above or a composition containing the mixture of two or more fungicide(s). The transgenic plant propagation material (preferably seed) comprises the fungicide(s)in an amount of from 0.1 g to 10 kg per 100 kg of transgenic plant propagation material (preferably seed).

**Examples**

The following examples are not intended to limit the scope of the claims to the invention, but are rather intended to be exemplary of certain embodiments. Any variations in the exemplified methods that occur to the skilled artisan are intended to fall within the scope of the present invention.

**Example 1: General methods**

The chemical synthesis of oligonucleotides can be affected, for example, in the known fashion using the phosphoamidite method (Voet, Voet, 2nd Edition, Wiley Press New York, pages 896-897). The cloning steps carried out for the purposes of the present invention such as, for example, restriction cleavages, agarose gel electrophoresis, purification of DNA fragments, transfer of nucleic acids to nitrocellulose and nylon membranes, linking DNA fragments, transformation of E. coli cells, bacterial cultures, phage multiplication and sequence analysis of recombinant DNA, are carried out as described by Sambrook et al. Cold Spring Harbor Laboratory Press (1989), ISBN 0-87969-309-6. The sequencing of recombinant DNA molecules is carried out with an MWG-Licor laser fluorescence DNA sequencer following the method of Sanger (Sanger et al., Proc. Natl. Acad. Sci. USA 74, 5463 (1977).

**Example 2: Cloning of overexpression vector constructs for stable soybean transformation**

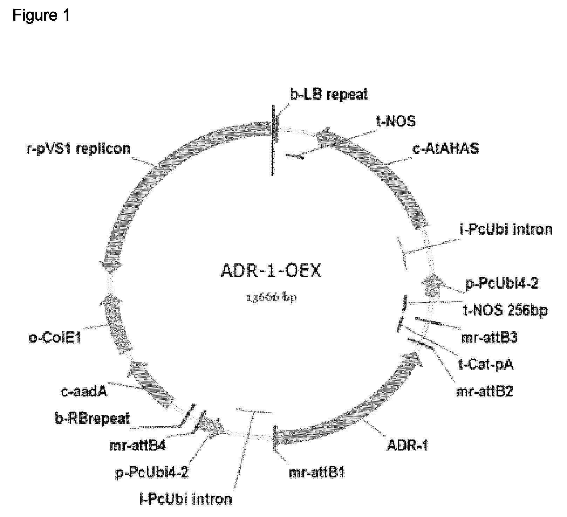

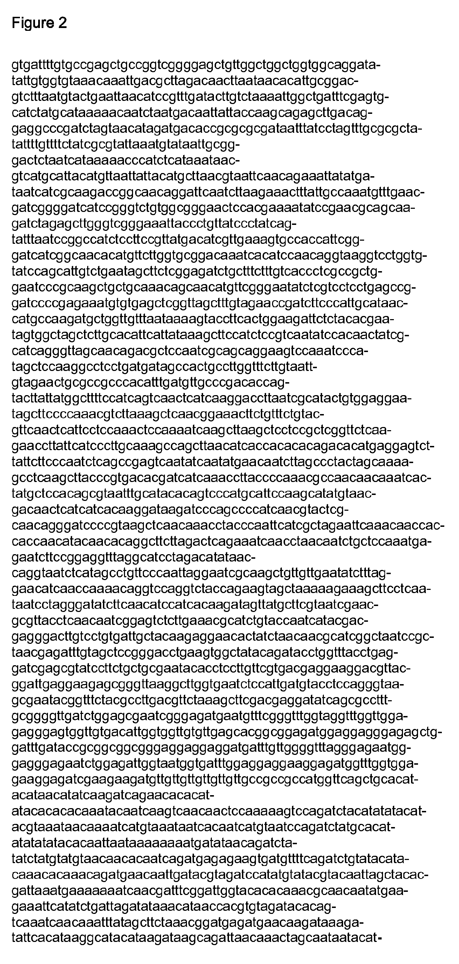

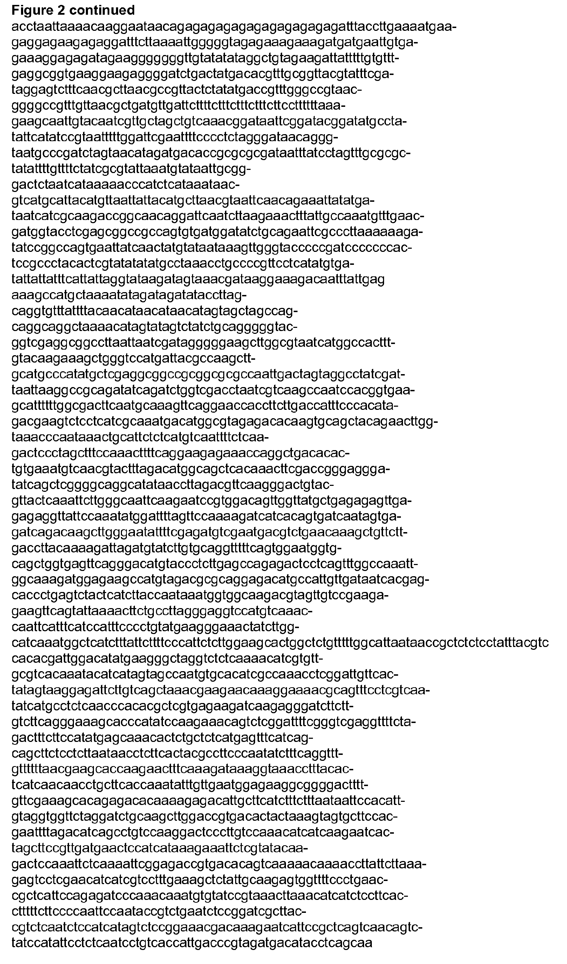

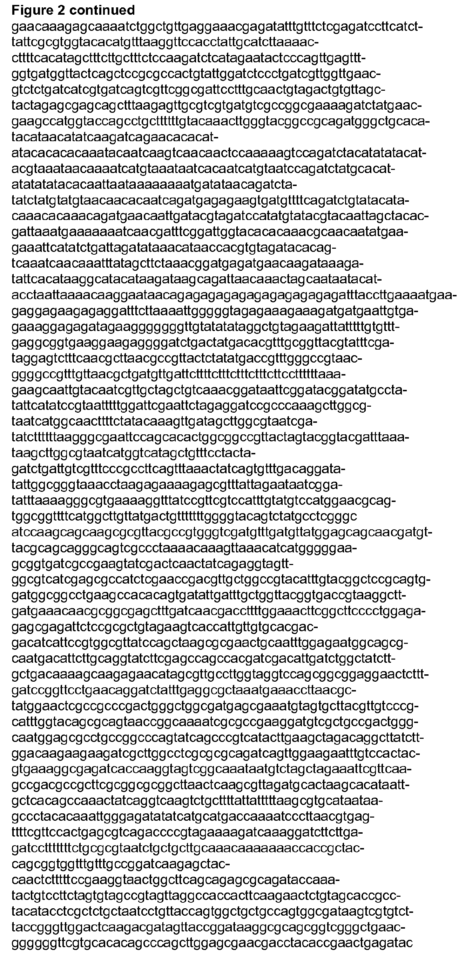

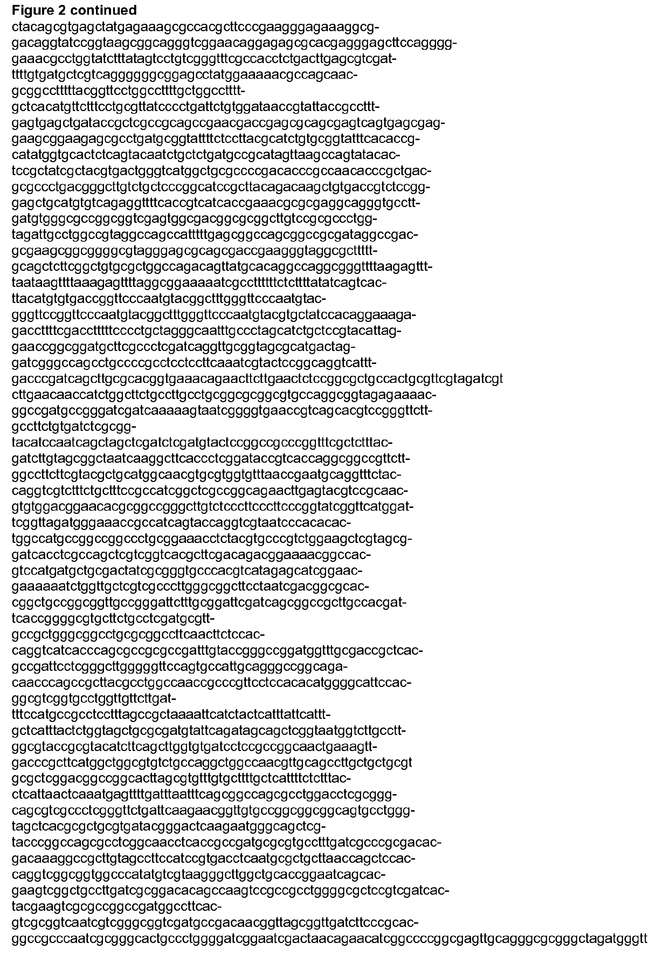

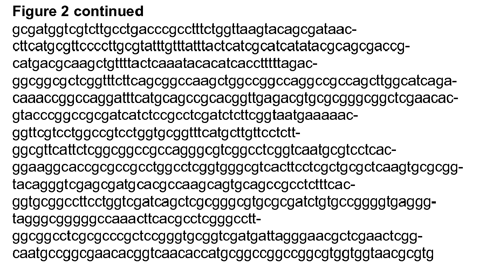

The overexpression ADR-1 vector construct (Figures 1 and 2, SEQ-ID-No. 235) is prepaired as follows:

Unless otherwise specified, standard methods as described in Sambrook et al., Molecular Cloning: A laboratory manual, Cold Spring Harbor 1989, Cold Spring Harbor Laboratory Press are used.

cDNA is produced from Arabidopsis thaliana (ecotype Col-0) RNA by using the Superscript II cDNA synthesis kit (Invitrogen). All steps of cDNA preparation and purification are performed according as described in the manual. Alternatively, the cDNA can be obtained by chemical synthesis.

The SEQ-ID-No.7-sequence is amplified from cDNA by PCR as described in the protocol of the Phusion hot-start, Pfu Ultra, Pfu Turbo or Herculase DNA polymerase (Stratagene).

The composition for the protocol of the Pfu Ultra, Pfu Turbo or Herculase DNA polymerase was as follows: 1x PCR buffer, 0.2 mM of each dNTP, 100 ng cDNA of Arabidopsis thaliana (var Columbia-0) , 50 pmol forward primer, 50 pmol reverse primer, 1 u Phusion hot-start, Pfu Ultra, Pfu Turbo or Herculase DNA polymerase.

The amplification cycles are as follows:

1 cycle of 60 seconds at 98°C, followed by 35 cycles of in each case 10 seconds at 98°C, 30 seconds at 60°C and 60 seconds at 72°C, followed by 1 cycle of 10 minutes at 72°C, then 4°C. The following primer sequences are used to specifically amplify the ADR-1 full-length ORF for cloning purposes:

i) foward primer: 5'CCGGTACCATGGCTTCGTTCATAGAT-3' (SEQ ID NO: 236)
ii) reverse primer: 5'-TTGTCGACCTAATCGTCAAGCCAATC-3' (SEQ ID NO: 237)

The amplified fragments are digested using the resitriction enzymes Acc65I and SalI (NEB Biolabs) and ligated in a Acc65I / SalI digested Gateway pENTRY vector (Invitrogen, Life Technologies, Carlsbad, California, USA) in a way that the full-length ADR-1 fragment is located in sense direction between the attL1 and attL2 recombination sites.

To obtain the binary plant transformation vector, a triple LR reaction (Gateway system, (Invitrogen, Life Technologies, Carlsbad, California, USA) is performed according to manufacturers protocol by using a pENTRY-A vector containing a parsley ubiquitine promoter, the ADR-1 in a pENTRY-B vector and a pENTRY-C vector containing a t-Nos terminator. As target a binary pDEST vector is used which is composed of: (1) a Kanamycin resistance cassette for bacterial selection (2) a pVS1 origin for replication in Agorbacteria (3) a pBR322 origin of replication for stable maintenance in E. coli and (4) between the right and left border an AHAS selection under control of a pcUbi-promoter (Figure 1). The recombination reaction is transformed into E. coli (DH5alpha), mini-prepped and screened by specific restriction digestions. A positive clone from each vector construct is sequenced and submitted soy transformation.

**Example 3: Soy transformation**

The expression vector constructs (see example 2) is transformed into soy.

8.1 Sterilization and Germination of Soy Seeds

Virtually any seed of any soy variety can be employed in the method of the invention. A variety of soybean cultivar (including Jack, Williams 82, Jake, Stoddard, CD215 and Resnik) is appropriate for soy transformation. Soy seeds are sterilized in a chamber with a chlorine gas produced by adding 3.5 ml 12N HCl drop wise into 100 ml bleach (5.25% sodium hypochlorite) in a desiccator with a tightly fitting lid. After 24 to 48 hours in the chamber, seeds are removed and approximately 18 to 20 seeds are plated on solid GM medium with or without 5 μM 6-benzyl-aminopurine (BAP) in 100 mm Petri dishes. Seedlings without BAP are more elongated and roots develop especially secondary and lateral root formation. BAP strengthens the seedling by forming a shorter and stockier seedling.

Seven-day-old seedlings grown in the light (>100 μEinstein/$m^2$s) at 25 degreeC are used for explant material for the three-explant types. At this time, the seed coat is split, and the epicotyl with the unifoliate leaves are grown to, at minimum, the length of the cotyledons. The epicotyl should be at least 0.5 cm to avoid the cotyledonary-node tissue (since soycultivars and seed lots may vary in the developmental time a description of the germination stage is more accurate than a specific germination time).

For inoculation of entire seedlings, see Method A (example 3.3. and 3.3.2) or leaf explants see Method B (example 3.3.3).

For method C (see example 3.3.4), the hypocotyl and one and a half or part of both cotyledons are removed from each seedling. The seedlings are then placed on propagation media for 2 to 4 weeks. The seedlings produce several branched shoots to obtain explants from. The majority of the explants originated from the plantlet growing from the apical bud. These explants are preferably used as target tissue.

3.2 - Growth and Preparation of Agrobacterium Culture

Agrobacterium cultures are prepared by streaking Agrobacterium (e.g., A. tumefaciens or A. rhizogenes) carrying the desired binary vector (e.g. H. Klee. R. Horsch and S. Rogers 1987 Agrobacterium-Mediated Plant Transformation and its further Applications to Plant Biology; Annual Review of Plant Physiology Vol. 38: 467-486) onto solid YEP growth medium YEP media: 10 g yeast extract. 10 g Bacto Peptone. 5 g NaCl. Adjust pH to 7.0, and bring final volume to 1 liter with H2O, for YEP agar plates add 20g Agar, autoclave) and incubating at 25.degree C. until colonies appeared (about 2 days). Depending on the selectable marker genes present on the Ti or Ri plasmid, the binary vector, and the bacterial chromosomes, different selection compounds are be used for A. tumefaciens and A. rhizogenes selection in the YEP solid and liquid media. Various Agrobacterium strains can be used for the transformation method.

After approximately two days, a single colony (with a sterile toothpick) is picked and 50 ml of liquid YEP is inoculated with antibiotics and shaken at 175 rpm (25 °C.) until an $OD_{600}$ between 0.8-1.0 is reached (approximately 2 d). Working glycerol stocks (15%) for transformation are prepared and one-ml of Agrobacterium stock aliquoted into 1.5 ml Eppendorf tubes then stored at -80 °C.

The day before explant inoculation, 200 ml of YEP are inoculated with 5 μl to 3 ml of working Agrobacterium stock in a 500 ml Erlenmeyer flask. The flask is shaken overnight at 25 °C. until the $OD_{600}$ is between 0.8 and 1.0. Before preparing the soy explants, the Agrobacteria ARE pelleted by centrifugation for 10 min at 5,500xg at 20 °C. The pellet Is resuspended in liquid CCM to the desired density ($OD_{600}$ 0.5-0.8) and placed at room temperature at least 30 min before use.

3.3 - Explant Preparation and Co-Cultivation (Inoculation)

3.3.1 Method A: Explant Preparation on the Day of Transformation.

Seedlings at this time have elongated epicotyls from at least 0.5 cm but generally between 0.5 and 2 cm. Elongated epicotyls up to 4 cm in length are successfully employed. Explants are then prepared with: i) with or without some roots, ii) with a partial, one or both cotyledons, all preformed leaves are removed including apical meristem, and the node located at the first set of leaves is injured with several cuts using a sharp scalpel.

This cutting at the node not only induces Agrobacterium infection but also distributes the axillary meristem cells and damaged pre-formed shoots. After wounding and preparation, the explants are set aside in a Petri dish and subsequently co-cultivated with the liquid CCM/Agrobacterium mixture for 30 minutes. The explants are then removed from the liquid medium and plated on top of a sterile filter paper on 15x100 mm Petri plates with solid co-cultivation medium. The wounded target tissues are placed such that they are in direct contact with the medium.

3.3.2 Modified Method A: Epicotyl Explant Preparation

Soyepicotyl segments prepared from 4 to 8 d old seedlings are used as explants for regeneration and transformation. Seeds of soya cv. L00106CN, 93-41131 and Jack are germinated in 1/10 MS salts or a similar composition medium with or without cytokinins for 4 to 8 d. Epicotyl explants are prepared by removing the cotyledonary node and stem node from the stem section. The epicotyl is cut into 2 to 5 segments. Especially preferred are segments attached to the primary or higher node comprising axillary meristematic tissue.

The explants are used for Agrobacterium infection. Agrobacterium AGL1 harboring a plasmid with the gene of interest (GOI) and the AHAS, bar or dsdA selectable marker gene is cultured in LB medium with appropriate antibiotics overnight, harvested and resuspended in a inoculation medium with acetosyringone. Freshly prepared epicotyl segments are soaked in the Agrobacterium suspension for 30 to 60 min and then the explants were blotted dry on sterile filter papers. The inoculated explants are then cultured on a co-culture medium with L-cysteine and TTD and other chemicals such as acetosyringone for increasing T-DNA delivery for 2 to 4 d. The infected epicotyl explants are then placed on a shoot induction medium with selection agents such as imazapyr (for AHAS gene), glufosinate (for bar gene), or D-serine (for dsdA gene). The regenerated shoots are subcultured on elongation medium with the selective agent.

For regeneration of transgenic plants the segments are then cultured on a medium with cytokinins such as BAP, TDZ and/or Kinetin for shoot induction. After 4 to 8 weeks, the cultured tissues are transferred to a medium with lower concentration of cytokinin for shoot elongation. Elongated shoots are transferred to a medium with auxin for rooting and plant development. Multiple shoots are regenerated.

Many stable transformed sectors showing strong cDNA expression are recovered. Soybean plants are regenerated from epicotyl explants. Efficient T-DNA delivery and stable transformed sectors are demonstrated.

3.3.3 Method B: Leaf Explants

For the preparation of the leaf explant the cotyledon is removed from the hypocotyl. The cotyledons are separated from one another and the epicotyl is removed. The primary leaves, which consist of the lamina, the petiole, and the stipules, are removed from the epicotyl by carefully cutting at the base of the stipules such that the axillary meristems are included on the explant. To wound the explant as well as to stimulate de novo shoot formation, any pre-formed shoots are removed and the area between the stipules was cut with a sharp scalpel 3 to 5 times.

The explants are either completely immersed or the wounded petiole end dipped into the Agrobacterium suspension immediately after explant preparation. After inoculation, the explants are blotted onto sterile filter paper to remove excess Agrobacterium culture and place explants with the wounded side in contact with a round 7 cm Whatman paper overlaying the solid CCM medium (see above). This filter paper prevents A. tumefaciens overgrowth on the soy-explants. Wrap five plates with Parafilm.TM. "M" (American National Can, Chicago, Ill., USA) and incubate for three to five days in the dark or light at 25 °C.

3.3.4 Method C: Propagated Axillary Meristem

For the preparation of the propagated axillary meristem explant propagated 3-4 week-old plantlets are used. Axillary meristem explants can be pre-pared from the first to the fourth node. An average of three to four explants could be obtained from each seedling. The explants are prepared from plantlets by cutting 0.5 to 1.0 cm below the axillary node on the internode and removing the petiole and leaf from the explant. The tip where the axillary meristems lie is cut with a scalpel to induce de novo shoot growth and allow access of target cells to the Agrobacterium. Therefore, a 0.5 cm explant includes the stem and a bud.

Once cut, the explants are immediately placed in the Agrobacterium suspension for 20 to 30 minutes. After inoculation, the explants are blotted onto sterile filter paper to remove excess Agrobacterium culture then placed almost completely immersed in solid CCM or on top of a round 7 cm filter paper overlaying the solid CCM, depending on the Agrobacterium strain. This filter paper prevents Agrobacterium overgrowth on the soy-explants. Plates are wrapped with Parafilm.TM. "M" (American National Can, Chicago, Ill., USA) and incubated for two to three days in the dark at 25 °C.

3.4 - Shoot Induction

After 3 to 5 days co-cultivation in the dark at 25 °C., the explants are rinsed in liquid SIM medium (to remove excess Agrobacterium) (SIM, see Olhoft et al 2007 A novel Agrobacterium rhizogenes-mediated transformation method of soy using primary-node explants from seedlings In Vitro Cell. Dev. Biol.-Plant (2007) 43:536-549; to remove excess Agrobacterium) or Modwash medium (1X B5 major salts, 1X B5 minor salts, 1X MSIII iron, 3% Sucrose, 1X B5 vitamins, 30 mM MES, 350 mg/L Timentin pH 5.6, WO 2005/121345) and blotted dry on sterile filter paper (to prevent damage especially on the lamina) before placing on the solid SIM medium. The approximately 5 explants (Method A) or 10 to 20 (Methods B and C) explants are placed such that the target tissue is in direct contact with the medium. During the first 2 weeks, the explants can be cultured with or without selective medium. Preferably, explants are transferred onto SIM without selection for one week.

For leaf explants (Method B), the explant should be placed into the medium such that it is perpendicular to the surface of the medium with the petiole imbedded into the medium and the lamina out of the medium.

For propagated axillary meristem (Method C), the explant is placed into the medium such that it is parallel to the surface of the medium (basipetal) with the explant partially embedded into the medium.

Wrap plates with Scotch 394 venting tape (3M, St. Paul, Minn., USA) are placed in a growth chamber for two weeks with a temperature averaging 25.degree. C. under 18 h light/6 h dark cycle at 70-100 $\mu E/m^2 s$. The explants remaines on the SIM medium with or without selection until de novo shoot growth occurred at the target area (e.g., axillary meristems at the first node above the epicotyl). Transfers to fresh medium can occur during this time. Explants are transferred from the SIM with or without selection to SIM with selection after about one week. At this time, there is considerable de novo shoot development at the base of the petiole of the leaf explants in a variety of SIM (Method B), at the primary node for seedling explants (Method A), and at the axillary nodes of propagated explants (Method C).

Preferably, all shoots formed before transformation are removed up to 2 weeks after co-cultivation to stimulate new growth from the meristems. This helped to reduce chimerism in the primary transformant and increase amplification of transgenic meristematic cells. During this time the explant may or may not be cut into smaller pieces (i.e. detaching the node from the explant by cutting the epicotyl).

3.5 - Shoot Elongation

After 2 to 4 weeks (or until a mass of shoots is formed) on SIM medium (preferably with selection), the explants are transferred to SEM medium (shoot elongation medium, see Olhoft et al 2007 A novel Agrobacterium rhizogenes-mediated transformation method of soy using primary-node explants from seedlings. In Vitro Cell. Dev. Biol.-Plant (2007) 43:536-549) that stimulates shoot elongation of the shoot primordia. This medium may or may not contain a selection compound.

After every 2 to 3 weeks, the explants are transferred to fresh SEM medium (preferably containing selection) after carefully removing dead tissue. The explants should hold together and not fragment into pieces and retain somewhat healthy. The explants are continued to be transferred until the explant dies or shoots elongate. Elongated shoots >3 cm are removed and placed into RM medium for about 1 week (Methods A and B), or about 2 to 4 weeks depending on the cultivar (Method C) at which time roots began to form. In the case of explants with roots, they are transferred directly into soil. Rooted shoots are transferred to soil and hardened in a growth chamber for 2 to 3 weeks before transferring to the greenhouse. Regenerated plants obtained using this method are fertile and produced on average 500 seeds per plant.

After 5 days of co-cultivation with Agrobacterium tumefaciens transient expression of the gene of interest (GOI) is widespread on the seedling axillary meristem explants especially in the regions wounding during explant preparation (Method A). Explants are placed into shoot induction medium without selection to see how the primary-node responds to shoot induction and regeneration. Thus far, greater than 70% of the explants were formed new shoots at this region. Expression of the GOI is stable after 14 days on SIM, implying integration of the T-DNA into the soybean genome. In addition, preliminary experiments results in the formation of cDNA expressing shoots forming after 3 weeks on SIM.

For Method C, the average regeneration time of a soybean plantlet using the propagated axillary meristem protocol is 14 weeks from explant inoculation. Therefore, this method has a quick regeneration time that leads to fertile, healthy soybean plants.

**Example 4: Pathogen assay for soybean**

4.1. Growth of plants 10 T1 soy plants per event are potted and grown for 3-4 weeks in the Phytochamber (16 h-day- und 8 h-night-Rhythm at a temperature of 16° and 22° C und a humidity of 75 %) till the first 2 trifoliate leaves were fully expanded.

4.2 Inoculation

The plants are inoculated with spores of *P.pachyrhizi.*

In order to obtain appropriate spore material for the inoculation, soybean leaves which are infected with rust 15-20 days ago, are taken 2-3 days before the inoculation and transferred to agar plates (1 % agar in H2O). The leaves are placed with their upper side onto the agar, which allowed the fungus to grow through the tissue and to produce very young spores. For the inoculation solution, the spores are knocked off the leaves and are added to a Tween-H2O solution. The counting of spores is performed under a light microscope by means of a Thoma counting chamber. For the inoculation of the plants, the spore suspension is added into a compressed-air operated spray flask and applied uniformly onto the plants or the leaves until the leaf surface is well moisturized. For macroscopic assays a spore density of 1-5 x $10^5$ spores/ml is used. For the microscopy, a density of >5 x $10^5$ spores / ml is used. The inoculated plants are placed for 24 hours in a greenhouse chamber with an average of 22°C and >90% of air humidity. The following cultivation is performed in a chamber with an average of 25°C and 70% of air humidity.

**Example 5: Microscopical screening:**

For the evaluation of the pathogen development, the inoculated leaves of plants are stained with aniline blue 48 hours after infection.

The aniline blue staining serves for the detection of fluorescent substances. During the defense reactions in host interactions and non-host interactions, substances such as phenols, callose or lignin accumulate or are produced and are incorporated at the cell wall either locally in papillae or in the whole cell (hypersensitive reaction, HR). Complexes are formed in association with aniline blue, which lead e.g. in the case of callose to yellow fluorescence. The leaf material is transferred to falcon tubes or dishes containing destaining solution II (ethanol / acetic acid 6/1) and is incubated in a water bath at 90°C for 10-15 minutes. The destaining solution II is removed immediately thereafter, and the leaves are washed 2x with water. For the staining, the leaves are incubated for 1.5-2 hours in staining solution II (0.05 % aniline blue = methyl blue, 0.067 M di-potassium hydrogen phosphate) and analyzed by microscopy immediately thereafter.

The different interaction types are evaluated (counted) by microscopy. An Olympus UV microscope BX61 (incident light) and a UV Longpath filter (excitation: 375/15, Beam splitter: 405 LP) are used. After aniline blue staining, the spores appeared blue under UV light. The papillae can be recognized beneath the fungal appressorium by a green/yellow staining. The hypersensitive reaction (HR) is characterized by a whole cell fluorescence

**Example 6: Evaluating the susceptibility to soybean rust**

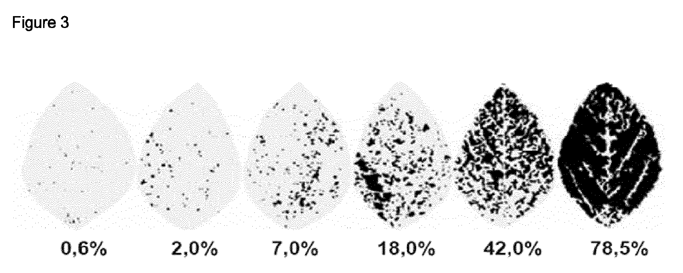

The progression of the soybean rust disease is scored by the estimation of the diseased area (area which was covered by sporulating uredinia) on the backside (abaxial side) of the leaf. Additionally the yellowing of the leaf is taken into account. (for scheme see Figure 3)

At all 50 T1 soybean plants per construct are inoculated with spores of *Phakopsora pachyrhizi.* The macroscopic disease symptoms of soy against P. pachyrhizi of the inoculated soybean plants are scored 14 days after inoculation.

The average of the percentage of the leaf area showing fungal colonies or strong yellowing/browning on all leaves is considered as diseased leaf area. At all 50 soybean T1 plants per construct (expression checked by RT-PCR) are evaluated in parallel to non-transgenic control plants. Non-transgenic soy plants grown in parallel to the transgenic plants are used as controls.

**Example 7: Maize transformation**

*Agrobacterium* cells harboring a plasmid containing the gene of interest (see above) and the mutated maize AHAS gene are grown in YP medium supplemented with appropriate antibiotics for 1-2 days. One loop of *Agrobacterium* cells is collected and suspended in 1.8 ml M-LS-002 medium (LS-inf). The cultures are incubated while shaking at 1,200 rpm for 5 min-3 hrs. Corn cobs are harvested at 8-11 days after pollination. The cobs are sterilized in 20% Clorox solution for 5 min, followed by spraying with 70% Ethanol and then thoroughly rinsed with sterile water. Immature embryos 0.8-2.0 mm in size are dissected into the tube containing *Agrobacterium* cells in LS-inf solution.

The constructs are transformed into immature embryos by a protocol modified from Japan Tobacco *Agrobacterium* mediated plant transformation method (US Patent Nos. 5,591,616; 5,731,179; 6,653,529; and U.S. Patent Application Publication No. 2009/0249514). Two types of plasmid vectors are used for transformation. One type has only one T-DNA border on each of left and right side of the border, and selectable marker gene and gene of interest are between the left and right T-DNA borders. The other type is so called "two T-DNA constructs" as described in Japan Tobacco U.S. Patent No. 5,731,179. In the two DNA constructs, the selectable marker gene is located between one set of T-DNA borders and the gene of interest is included in between the second set of T-DNA borders. Either plasmid vector can be used. The plasmid vector is electroporated into *Agrobacterium.*

*Agrobacterium* infection of the embryos is carried out by inverting the tube several times. The mixture is poured onto a filter paper disk on the surface of a plate containing co-cultivation medium (M-LS-011). The liquid agro-solution is removed and the embryos are checked under a microscope and placed scutellum side up. Embryos are cultured in the dark at 22°C for 2-4 days, and transferred to M-MS-101 medium without selection and incubated for four to seven days. Embryos are then transferred to M-LS-202 medium containing 0.75μM imazethapyr and grown for three weeks at 27°C to select for transformed callus cells.

Plant regeneration is initiated by transferring resistant calli to M-LS-504 medium supplemented with 0.75μM imazethapyr and growing under light at 26°C for two to three weeks. Regenerated shoots are then transferred to a rooting box with M-MS- 618 medium (0.5μM imazethapyr). Plantlets with roots are transferred to soil-less potting mixture and grown in a growth chamber for a week, then transplanted to larger pots and maintained in a greenhouse until maturity. Transgenic maize plant production is also described, for example, in U.S. Patent No. 5,591,616 and 6,653,529; U.S. Patent Application Publication No. 2009/0249514; and WO/2006136596, each of which are hereby incorporated by reference in their entirety. Transformation of maize may be made using *Agrobacterium* transformation, as described in U.S. Patent Nos. 5,591,616; 5,731,179; U.S. Patent Application Publication No. 2002/0104132, and the like. Transformation of maize (*Zea mays* L.) can also be performed with a modification of the method described by Ishida et al. (Nature Biotech., 1996, 14:745-750). The inbred line A188 (University of Minnesota) or hybrids with A188 as a parent are good sources of donor material for transformation (Fromm et al., Biotech, 1990, 8:833), but other genotypes can be used successfully as well. Ears are harvested from corn plants at approximately 11 days after pollination (DAP) when the length of immature embryos is about 1 to 1.2 mm. Immature embryos are co-cultivated with Ag*robacterium tumefaciens* that carry "super binary" vectors and transgenic plants are recovered through organogenesis. The super binary vector system is described in WO 94/00977 and WO 95/06722. Vectors are constructed as described. Various selection marker genes are used including the maize gene encoding a mutated acetohydroxy acid synthase (AHAS) enzyme (U.S. Patent No. 6,025,541). Similarly, various promoters are used to regulate the trait gene to provide constitutive, developmental, inducible, tissue or environmental regulation of gene transcription. Excised embryos can be used and can be grown on callus induction medium, then maize regeneration medium, containing imidazolinone as a selection agent. The Petri dishes are incubated in the light at 25°C for 2-3 weeks, or until shoots develop. The green shoots are transferred from each embryo to maize rooting medium and incubated at 25°C for 2-3 weeks, until roots develop. The rooted shoots are transplanted to soil in the greenhouse. T1 seeds are produced from plants that exhibit tolerance to the imidazolinone herbicides and which are PCR positive for the transgenes.

**Example 8: Fusarium and Colletotrichum resistance screening**

Transgenic maize plants expressing the genes according to the present invention are grown in greenhouse or phyto-chamber under standard growing conditions in a controlled environment (20-25°C, 60-90% humidity).

Shortly after the transgenic maize plants enter the reproductive phase they are inoculated near the base of the stalk using a fungal suspension of spores ($10^5$ spores in PBS solution) of Fusarium ssp. or Colletotrichum graminicola. Plants are incubated for 2-4 weeks at 20-25°C and 60-90% humidity.

For scoring the stalk rot disease, stalks are split and the progression of the disease is scored by observation of the characteristic brown to black color of the fungus as it grows up the stalk. Disease ratings are conducted by assigning a visual score. Per experiment the diseased leaf area of more than 10 transgenic plants (and wild-type plants as control) is scored. For analysis the average of the diseased leaf area of the non-transgenic mother plant is set to 100% to calculate the relative diseased leaf area of the transgenic lines

**Example 9: Cultivation and Fungicide application**

Soybean rust resistant transgenic soybean events were generated. The soybean rust resistant transgenic soybean events are grown in the field. Before planting the field was fertilized with 200 kg/ha of a 0-20-20 (N-P-K) fertilizer. Soybean seeds are planted 4 cm deep in the soil with a density of 260.000 plants/ha (12-14 seeds/m, row distance 50cm).

At all 3 fungicide treatments are applied according to the description provided by the supplier. The first fungicide treatment was performed 45 days after planting using 300g/ha Azoxystrobin + Benzovindiflupyr (Elatus ® ;Syngenta) solved in 200l water. The second fungicide treatment was performed 60 days after planting using Prothiconazole + Trifloxystrobin (Fox ®, Bayer) with a concentration of 400ml/ha solved in 200lm water. The third fungicide treatment was performed 75 days after planting using Cyproconazole + Picoxystrobine (Aproach Prima®; DuPont) with a concentration of 300ml/ha solved in 200l water.

| Treatment | Product | Active compounds | Concentration |
|---|---|---|---|
| 1. | Elatus | Azoxystrobin + Benzovindiflupyr | 300 g/ha |
| 2. | Fox | Prothiconazole + Trifloxystrobin | 400 ml/ha |
| 3. | Aproach Prima | Cyproconazole + Picoxystrobin | 300 ml/ha |

Soybeans are harvested using a combine Wintersteiger Plot Combine 130 days after planting when mature plants became dry.

The treatment of transgenic soy plants obtained by the methods (transformation method A) described in the aforementioned examples with the fungicides in this example provide a synergistic effect on the reduction of fungal infection.

## Claims

**1.** A method for controlling fungal infection and/or increasing plant health in transgenic plants, transgenic plant parts, transgenic plant cells and/or transgenic plant propagation materials comprising the application of a fungicide to the transgenic plant, transgenic plants parts, transgenic plant cells, transgenic plant propagation materials, and/or at their locus of growth and/or at the locus where are they to grow, wherein the fungicide is selected from the group consisting

a) Respiration inhibitors,
b) Sterol biosynthesis inhibitors,
c) Nucleic acid synthesis inhibitors,
d) Inhibitors of cell division and cytoskeleton,
e) Inhibitors of amino acid and protein synthesis,
f) Signal transduction inhibitors,
g) Lipid and membrane synthesis inhibitors,
h) Inhibitors with Multi Site Action,
i) Cell wall synthesis inhibitors,
j) Plant defence inducers, and
k) Unknown mode of action
and combinations thereof
wherein the transgenic plant is overexpressing exogenous protein(s),

(a) wherein said exogenous protein(s) is(are) encoded by

(i) nucleic acid(s) having at least 70% identity, at least 80% identity, at least 90% identity, at least 95% identity with sequence(s) selected from the group consisting of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97 and 99 or functional fragment(s) thereof, or splice variant(s) thereof;
(ii) nucleic acid(s) coding for protein(s) having at least 70% identity, at least 80% identity, at least 90% identity, at least 95% identity with sequence(s) selected from the group consisting of SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98 and 100 or functional fragment(s) thereof;
(iii) exogenous nucleic acid(s) capable of hybridizing under stringent conditions with complementary sequence(s) of any of the nucleic acid(s) according to (i) or (ii); or
(iv) nucleic acid(s) encoding the same protein(s) as the nucleic acid(s) of (i) to (iii) above, but differing from the nucleic acid(s) of (i) to (iii) above due to the degeneracy of the genetic code;

operably linked with a promoter and a transcription termination sequence.

**2.** The method of claim 1, wherein the fungicide is selected from the group consisting of

A) Respiration inhibitors

- Inhibitors of complex III at Qo site (e. g. strobilurins): azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-N-[2-[(1,4-dimethyl-5-phenyl-pyrazol-3-yl)oxylmethyl]phenyl]-N-methoxy-carbamate (A.1.22), 1-[3-chloro-2-[[1-(4-chlorophenyl)-1 H-pyrazol-3-yl]oxymethyl]phenyl]-4-methyl-tetrazol-5-one (A.1.23), 1-[3-bromo-2-[[1-(4-chlorophenyl)pyrazol-3-yl]oxymethyl]phenyl]-4-methyltetrazol-5-one (A.1.24), 1-[2-[[1-(4-chlorophenyl)pyrazol-3-yl]oxymethyl]-3-methylphenyl]-4-methyl-tetrazol-5-one (A.1.25), 1-[2-[[1-(4-chlorophenyl)pyrazol-3-yl]oxymethyl]-3-fluoro-phenyl]-4-methyl-tetrazol-5-one (A.1.26), 1-[2-[[1-(2,4-dichlorophenyl)pyrazol-3-yl]oxymethyl]-3-fluoro-phenyl]-4-methyl-tetrazol-5-one (A. 1.27), 1-[2-[[4-(4-chlorophenyl)thiazol-2-yl]oxymethyl]-3-methylphenyl]-4-methyl-tetrazol-5-one (A.1.28), 1-[3-chloro-2-[[4-(p-tolyl)thiazol-2-yl]oxymethyl]phenyl]-4-methyl-tetrazol-5-one (A.1.29), 1-[3-cyclopropyl-2-[[2-methyl-4-(1-methylpyrazol-3-yl)phenoxy]methyl]phenyl]-4-methyl-tetrazol-5-one (A.1.30), 1-[3-(difluoromethoxy)-2-[[2-methyl-4-(1-methylpyrazol-3-yl)phenoxy]methyl]phenyl]-4-methyl-tetrazol-5-one (A.1.31), 1-methyl-4-[3-methyl-2-[[2-methyl-4-(1-methylpyrazol-3-yl)phenoxy]methyl]phenyl]tetrazol-5-one (A.1.32), 1-methyl-4-[3-methyl-2-[[1-[3-(trifluoromethyl)phenyl]-ethylideneamino]oxymethyl]phenyl]tetrazol-5-one (A.1.33), (Z,2E)-5-[1-(2,4-dichlorophenyl)pyrazol-3-yl]-oxy-2-methoxyimino-N,3-dimethyl-pent-3-enamide (A.1.34), (Z,2E)-5-[1-(4-chlorophenyl)pyrazol-3-yl]oxy-2-methoxyimino-N,3-dimethyl-pent-3-enamide (A.1.35), (Z,2E)-5-[1-(4-chloro-2-fluoro-phenyl)pyrazol-3-yl]oxy-2-methoxyimino-N,3-dimethyl-pent-3-enamide (A.1.36),
- inhibitors of complex III at Qi site: cyazofamid (A.2.1), amisulbrom (A.2.2), [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate (A.2.3), [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate (A.2.4), [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate (A.2.5), [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate (A.2.6); (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate (A.2.7), (3S,6S,7R,8R)-8-benzyl-3-[3-[(isobutyryloxy)methoxy]-4-methoxypicolinamido]-6-methyl-4,9-dioxo-1,5-dioxonan-7-yl isobutyrate (A.2.8);
- inhibitors of complex II (e. g. carboxamides): benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.14), penthiopyrad (A.3.15), sedaxane (A.3.16), tecloftalam (A.3.17), thifluzamide (A.3.18), N-(4'-trifluoromethylthiobiphenyl-2-yl)-3-difluoromethyl-1-methyl-1 H-pyrazole-4-carboxamide (A.3.19), N-(2-(1,3,3-trimethyl-butyl)-phenyl)-1,3-dimethyl-5-fluoro-1 H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethylindan-4-yl)pyrazole-4-carboxamide (A.3.22), 1,3-dimethyl-N-(1,1,3-trimethylindan-4-yl)pyrazole-4-carboxamide (A.3.23), 3-(trifluoromethyl)-1,5-dimethyl-N-(1,1,3-trimethylindan-4-yl)pyrazole-4-carboxamide (A.3.24), 1,3,5-trimethyl-N-(1,1,3-trimethylindan-4-yl)pyrazole-4-carboxamide (A.3.25), N-(7-fluoro-1,1,3-trimethyl-indan-4-yl)-1,3-dimethyl-pyrazole-4-carboxamide (A.3.26), N-[2-(2,4-dichlorophenyl)-2-methoxy-1-methyl-ethyl]-3-(difluoromethyl)-1-methyl-pyrazole-4-carboxamide (A.3.27);
- other respiration inhibitors (e. g. complex I, uncouplers): diflumetorim (A.4.1), (5,8-difluoroquinazolin-4-yl)-{2-[2-fluoro-4-(4-trifluoromethylpyridin-2-yloxy)-phenyl]-ethyl}-amine (A.4.2); nitrophenyl derivates: binapacryl (A.4.3), dinobuton (A.4.4), dinocap (A.4.5), fluazinam (A.4.6); ferimzone (A.4.7); organometal compounds: fentin salts, such as fentin-acetate (A.4.8), fentin chloride (A.4.9) or fentin hydroxide (A.4.10); ametoctradin (A.4.11); and silthiofam (A.4.12);

B) Sterol biosynthesis inhibitors (SBI fungicides)

- C14 demethylase inhibitors (DMI fungicides): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[rel-(2S;3R)-3-(2-chlorophenyl)-2-(2,4-difluorophenyl)-oxiranylmethyl]-5-thiocyanato-1 H-[1,2,4]triazolo (B.1.31), 2-[rel-(2S;3R)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chlorophenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1 -(1,2,4-triazol-1 -yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.51); imidazoles: imazalil (B.1.42), pefurazoate (B.1.43), prochloraz (B.1.44), triflumizol (B.1.45); pyrimidines, pyridines and piperazines: fenarimol (B.1.46), nuarimol (B.1.47), pyrifenox (B.1.48), triforine (B.1.49), [3-(4-chloro-2-fluoro-phenyl)-5-(2,4-difluorophenyl)isoxazol-4-yl]-(3-pyridyl)methanol (B.1.50);
- Delta14-reductase inhibitors: aldimorph (B.2.1), dodemorph (B.2.2), dodemorphacetate (B.2.3), fenpropimorph (B.2.4), tridemorph (B.2.5), fenpropidin (B.2.6), piperalin (B.2.7), spiroxamine (B.2.8);
- Inhibitors of 3-keto reductase: fenhexamid (B.3.1);

C) Nucleic acid synthesis inhibitors

- phenylamides or acyl amino acid fungicides: benalaxyl (C.1.1), benalaxyl-M (C.1.2), kiralaxyl (C.1.3), metalaxyl (C.1.4), metalaxyl-M (mefenoxam, C.1.5), ofurace (C.1.6), oxadixyl (C.1.7);
- others: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7);

D) Inhibitors of cell division and cytoskeleton

- tubulin inhibitors, such as benzimidazoles, thiophanates: benomyl (D1.1), carbendazim (D1.2), fuberidazole (D1.3), thiabendazole (D1.4), thiophanate-methyl (D1.5); triazolopyrimidines: 5-chloro-7-(4-methylpiperidin-1-yl)-6-(2,4,6-trifluorophenyl)-[1,2,4]triazolo[1,5-a]pyrimidine (D1.6)
- other cell division inhibitors: diethofencarb (D2.1), ethaboxam (D2.2), pencycuron (D2.3), fluopicolide (D2.4), zoxamide (D2.5), metrafenone (D2.6), pyriofenone (D2.7);

E) Inhibitors of amino acid and protein synthesis

- methionine synthesis inhibitors (anilino-pyrimidines): cyprodinil (E.1.1), mepanipyrim (E.1.2), pyrimethanil (E.1.3);
- protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6), polyoxine (E.2.7), validamycin A (E.2.8);

F) Signal transduction inhibitors

- MAP / histidine kinase inhibitors: fluoroimid (F.1.1), iprodione (F.1.2), procymidone (F.1.3), vinclozolin (F.1.4), fenpiclonil (F.1.5), fludioxonil (F.1.6);
- G protein inhibitors: quinoxyfen (F.2.1);

G) Lipid and membrane synthesis inhibitors

- Phospholipid biosynthesis inhibitors: edifenphos (G.1.1), iprobenfos (G.1.2), pyrazophos (G.1.3), isoprothiolane (G.1.4);
- lipid peroxidation: dicloran (G.2.1), quintozene (G.2.2), tecnazene (G.2.3), tolclofosmethyl (G.2.4), biphenyl (G.2.5), chloroneb (G.2.6), etridiazole (G.2.7);
- phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7) and N-(1-(1-(4-cyano-phenyl)ethanesulfonyl)-but-2-yl) carbamic acid-(4-fluorophenyl) ester (G.3.8);
- compounds affecting cell membrane permeability and fatty acides: propamocarb (G.4.1);
- fatty acid amide hydrolase inhibitors: oxathiapiprolin (G.5.1), 2-{3-[2-(1-{[3,5-bis(difluoromethyl-1 H-pyrazol-1 -yl]acetyl}piperidin-4-yl)-1,3-thiazol-4-yl]-4,5-dihydro-1,2-oxazol-5-yl}phenyl methanesulfonate (G.5.2), 2-{3-[2-(1-{[3,5-bis(difluoromethyl)-1 H-pyrazol-1-yl]acetyl}piperidin-4-yl) 1,3-thiazol-4-yl]-4,5-dihydro-1,2-oxazol-5-yl}-3-chlorophenyl methanesulfonate (G.5.3);

H) Inhibitors with Multi Site Action

- inorganic active substances: Bordeaux mixture (H.1.1), copper acetate (H.1.2), copper hydroxide (H.1.3), copper oxychloride (H.1.4), basic copper sulfate (H.1.5), sulfur (H.1.6);
- thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
- organochlorine compounds (e. g. phthalimides, sulfamides, chloronitriles): anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11), N-(4-chloro-2-nitro-phenyl)-N-ethyl-4-methyl-benzenesulfonamide (H.3.12);
- guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1 H,5H-[1,4]dithiino[2,3-c:5,6-c']dipyrrole-1,3,5,7(2H,6H)-tetraone (H.4.10);

I) Cell wall synthesis inhibitors

- inhibitors of glucan synthesis: validamycin (I.1.1), polyoxin B (I.1.2);
- melanin synthesis inhibitors: pyroquilon (I.2.1), tricyclazole (I.2.2), carpropamid (I.2.3), dicyclomet (I.2.4), fenoxanil (I.2.5);

J) Plant defence inducers;

- acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);

K) Unknown mode of action

- bronopol (K.1.1), chinomethionat (K.1.2), cyflufenamid (K.1.3), cymoxanil (K.1.4), dazomet (K.1.5), debacarb (K.1.6), diclomezine (K.1.7), difenzoquat (K.1.8), difenzoquat-methylsulfate (K.1.9), diphenylamin (K.1.10), fenpyrazamine (K.1.11), flumetover (K.1.12), flusulfamide (K.1.13), flutianil (K.1.14), methasulfocarb (K.1.15), nitrapyrin (K.1.16), nitrothal-isopropyl (K.1.18), oxathiapiprolin (K.1.19), tolprocarb (K.1.20), oxin-copper (K.1.21), proquinazid (K.1.22), tebufloquin (K.1.23), tecloftalam (K.1.24), triazoxide (K.1.25), 2-butoxy-6-iodo-3-propylchromen-4-one (K.1.26), 2-[3,5-bis(difluoromethyl)-1 H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone (K.1.27), 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone (K.1.28), 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone (K.1.29), N-(cyclopropylmethoxyimino-(6-difluoro-methoxy-2,3-difluorophenyl)-methyl)-2-phenyl acetamide (K.1.30), N'-(4-(4-chloro-3-trifluoromethyl-phenoxy)-2,5-dimethylphenyl)-N-ethyl-N-methyl formamidine (K.1.31), N'-(4-(4-fluoro-3-trifluoromethyl-phenoxy)-2,5-dimethylphenyl)-N-ethyl-N-methyl formamidine (K.1.32), N'-(2-methyl-5-trifluoromethyl-4-(3-trimethylsilanyl-propoxy)-phenyl)-N-ethyl-N-methyl formamidine (K.1.33), N'-(5-difluoromethyl-2-methyl-4-(3-trimethylsilanyl-propoxy)-phenyl)-N-ethyl-N-methyl formamidine (K.1.34), methoxyacetic acid 6-tert-butyl-8-fluoro-2,3-dimethyl-quinolin-4-yl ester (K.1.35), 3-[5-(4-methylphenyl)-2,3-dimethyl-isoxazolidin-3-yl]-pyridine (K.1.36), 3-[5-(4-chlorophenyl)-2,3-dimethyl-isoxazolidin-3-yl]-pyridine (pyrisoxazole) (K.1.37), N-(6-methoxy-pyridin-3-yl) cyclopropanecarboxylic acid amide (K.1.38), 5-chloro-1-(4,6-dimethoxy-pyrimidin-2-yl)-2-methyl-1 H-benzoimidazole (K.1.39), 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluorophenyl]propan-2-ol (K.1.43), 2-[2-fluoro-6-[(8-fluoro-2-methyl-3-quinolyl)oxy]phenyl]propan-2-ol (K.1.44), 3-(5-fluoro-3,3,4,4-tetramethyl-3,4-dihydroisoquinolin-1-yl)-quinoline (K.1.45), 3-(4,4-difluoro-3,3-dimethyl-3,4-dihydroisoquinolin-1-yl)quinoline (K.1.46), 3-(4,4,5-trifluoro-3,3-dimethyl-3,4-dihydroisoquinolin-1-yl)quinoline (K.1.47), 9-fluoro-2,2-dimethyl-5-(3-quinolyl)-3H-1,4-benzoxazepine (K.1.48)

**3.** The method of any one of the preceeding claims, wherein the transgenic plant shows herbicide tolerance present in the transgenic plant compared to the corresponding wild-type plant.

**4.** The method of any one of the preceeding claims, wherein the transgenic plant is a plant, which is tolerant to the action of herbicides selected form the group consisting of glyphosate, glufosinate, imidazolinone-herbidicides a, dicamba and combinations thereof.

**5.** The method according to anyone of the preceeding claims, wherein the fungal infection is rust fungus, downy mildew, powdery mildew, leaf spot, late blight, fusarium and / or septoria.

**6.** The method or use according to anyone of the preceeding claims, wherein the fungal infection is Phakopsora meibomiae, Phakopsora pachyrhizi, Fusarium graminearum and/or Fusarium verticillioides.

**7.** The method according to anyone of the preceeding claims, wherein the plant is selected from the group consisting of soy, rice, wheat, barley, rye, and corn, preferably wherein the plant is soy or corn.

**8.** The method according to claims 1 to 6, wherein the plant is soy and the fungicide is selected from the group consisting of:

Pyraclostrobin, Azoxystrobin, Trifloxystrobin, Picoxystrobin, (Z,2E)-5-[1-(2,4-dichlorophenyl)pyrazol-3-yl]-oxy-2-methoxyimino-N,3-dimethyl-pent-3-enamide, (Z,2E)-5-[1-(4-chlorophenyl)pyrazol-3-yl]oxy-2-methoxyimino-N,3-dimethyl-pent-3-enamide, 1-[3-chloro-2-[[1-(4-chlorophenyl)-1 H-pyrazol-3-yl]oxymethyl]phenyl]-4-methyl-tetrazol-5-one, 1-[3-bromo-2-[[1-(4-chlorophenyl)pyrazol-3-yl]oxymethyl]phenyl]-4-methyl-tetrazol-5-one, 1-[2-[[1-(4-chlorophenyl)pyrazol-3-yl]oxymethyl]-3-methyl-phenyl]-4-methyl-tetrazol-5-one, 1-[2-[[1-(4-chlorophenyl)pyrazol-3-yl]oxymethyl]-3-fluoro-phenyl]-4-methyl-tetrazol-5-one, 1-[2-[[1-(2,4-d ichlorophenyl)pyrazol-3-yl]oxymethyl]-3-fluoro-phenyl]-4-methyl-tetrazol-5-one, 1-[2-[[4-(4-chlorophenyl)thiazol-2-yl]oxymethyl]-3-methyl-phenyl]-4-methyltetrazol-5-one, 1-[3-chloro-2-[[4-(p-tolyl)thiazol-2-yl]oxymethyl]phenyl]-4-methyl-tetrazol-5-one, 1-[3-cyclopropyl-2-[[2-methyl-4-(1-methylpyrazol-3-yl)phenoxy]methyl]phenyl]-4 methyl-tetrazol-5-one, 1-[3-(difluoromethoxy)-2-[[2-methyl-4-(1methylpyrazol-3 yl)phenoxy]methyl]phenyl]-4-methyl-tetrazol-5-one, 1-methyl-4-[3-methyl-2 [[2 methyl-4-(1-methylpyrazol-3-yl)phenoxy]methyl]phenyl]tetrazol-5-one, 1-methyl-4-[3-methyl-2-[[1-[3-(trifluoromethyl)phenyl]-ethylideneamino]oxymethyl]phenyl]tetrazol-5 one, Fluxapyroxad, N-[9-(dichloromethylene)-1,2,3,4-tetrahydro-1,4-me-thanonaphthalen-5-yl]-3-(difluoromethyl)-1-methyl-1 H-pyrazole-4-carboxamide, Sedaxane, Penflufen, 3-(difluoromethyl)-1-methyl-N-(1,1,3-trimethylindan-4-yl)pyrazole-4-carboxamide; 3-(trifluoromethyl)-1-methyl-N-(1,1,3-trimethylindan-4-yl)pyrazole-4-carboxamide; 1,3-dimethyl-N-(1,1,3-trimethylindan-4-yl)pyrazole-4-carboxamide; 3-(trifluoromethyl)-1,5-dimethyl-N-(1,1,3-trimethylindan-4-yl)pyrazole-4-carboxamide; 3-(difluoromethyl)-1,5-dimethyl-N-(1,1,3-trimethylindan-4-yl)pyrazole-4-carboxamide; 1,3,5-trimethyl-N-(1,1,3-trimethylindan-4-yl)pyrazole-4-carboxamide, Carboxin, Fluazinam, Fentin salts (fentin acetate, fentin chloride, fentin hydroxide), epoxiconazole, prothioconazole, difenoconazole, tebuconazole, 1-[rel-(2S;3R)-3-(2-chlorophenyl)-2-(2,4-difluorophenyl)-oxiranylmethyl]-5 thiocyanato-1H-[1,2,4]triazole, 2-[rel-(2S;3R)-3-(2-chlorophenyl)-2-(2,4-difluorophenyl)-oxiranylmethyl]-2H [1,2,4]triazole-3-thiol, 2-[2-chloro-4-(4-chlorophenoxy)phenyl]-1 (1,2,4-triazol-1-yl)pentan-2-ol, 1-[4-(4-chlorophenoxy)-2-(trifluoromethyl)phenyl]-1 cyclopropyl-2-(1,2,4-triazol-1-yl)ethanol, 2-[4-(4-chlorophenoxy)-2-(trifluoromethyl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chlorophenoxy)-2-(trifluoromethyl)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)pentan-2-ol, 2-[4-(4-fluorophenoxy)-2-(trifluoromethyl)phenyl]-1-(1,2,4-triazol-1-yl)propan-2-ol, 2-[2-chloro-4-(4-chlorophenoxy)phenyl]-1-(1,2,4-triazol-1-yl)pent-3-yn-2-ol, cyproconazole, flutriafol, myclobutanil, Metalaxyl, Thiophanate-methyl, Carbendazim, Fludioxonil, thiram, chlorothalonil and combinations thereof.

**9.** The method according to claims 1 to 6, wherein the plant is corn and the fungicide is selected from the group consisting of:

Pyraclostrobin, Azoxystrobin, Trifloxystrobin. Picoxystrobin, Fluoxastrobin, Fluxapyroxad, Carboxin, epoxiconazole, metconazole, prothioconazole, difenoconazole, propiconazole, tebuconazole, cyproconazole, flutriafol, myclobutanil, diniconazole, ipconazole, triadimefon, captan, Metalaxyl, Carbendazim, Thiabendazole, Fludioxonil, mancozeb, thiram and combinations thereof.

**10.** Transgenic plants, transgenic plant parts, transgenic plant cells and/or transgenic plant propagation materials obtainable according to the method according to anyone of the preceeding claims.

Figure 1

b-LB repeat
t-NOS
c-AtAHAS
r-pVS1 replicon
i-PcUbi intron
ADR-1-OEX
13666 bp
p-PcUbi4-2
t-NOS 256bp
mr-attB3
o-ColE1
t-Cat-pA
mr-attB2
c-aadA
b-RBrepeat
ADR-1
mr-attB4
p-PcUbi4-2
mr-attB1
i-PcUbi intron

Figure 2

gtgattttgtgccgagctgccggtcggggagctgttggctggctggtggcaggata-
tattgtggtgtaaacaaattgacgcttagacaacttaataacacattgcggac-
gtctttaatgtactgaattaacatccgtttgatacttgtctaaaattggctgatttcgagtg-
catctatgcataaaaacaatctaatgacaattattaccaagcagagcttgacag-
gaggcccgatctagtaacatagatgacaccgcgcgcgataatttatcctagtttgcgcgcta-
tattttgttttctatcgcgtattaaatgtataattgcgg-
gactctaatcataaaaacccatctcataaataac-
gtcatgcattacatgttaattattacatgcttaacgtaattcaacagaaattatatga-
taatcatcgcaagaccggcaacaggattcaatcttaagaaactttattgccaaatgtttgaac-
gatcggggatcatccgggtctgtggcgggaactccacgaaaatatccgaacgcagcaa-
gatctagagcttgggtcgggaaattaccctgttatccctatcag-
tatttaatccggccatctccttccgttatgacatcgttgaaagtgccaccattcgg-
gatcatcggcaacacatgttcttggtgcggacaaatcacatccaacaggtaaggtcctggtg-
tatccagcattgtctgaatagcttctcggagatctgctttctttgtcaccctcgccgctg-
gaatcccgcaagctgctgcaaacagcaacatgttcgggaatatctcgtcctcctgagccg-
gatccccgagaaatgtgtgagctcggttagctttgtagaaccgatcttcccattgcataac-
catgccaagatgctggttgtttaataaaagtaccttcactggaagattctctacacgaa-
tagtggctagctcttgcacattcattataaagcttccatctccgtcaatatccacaactatcg-
catcagggttagcaacagacgctccaatcgcagcaggaagtccaaatccca-
tagctccaaggcctcctgatgatagccactgccttggtttcttgtaatt-
gtagaactgcgccgcccacatttgatgttgcccgacaccag-
tacttattatggcttttccatcagtcaactcatcaaggaccttaatcgcatactgtggaggaa-
tagcttccccaaacgtcttaaagctcaacggaaacttctgtttctgtac-
gttcaactcattcctccaaactccaaaatcaagcttaagctcctccgctcggttctcaa-
gaaccttattcatcccttgcaaagccagcttaacatcaccacacacagacacatgaggagtct-
tattcttcccaatctcagccgagtcaatatcaatatgaacaatcttagccctactagcaaaa-
gcctcaagcttacccgtgacacgatcatcaaaccttaccccaaacgccaacaacaaatcac-
tatgctccacagcgtaatttgcatacacagtcccatgcattccaagcatatgtaac-
gacaactcatcatcacaaggataagatcccagccccatcaacgtactcg-
caacagggatccccgtaagctcaacaaacctacccaattcatcgctagaattcaaacaaccac-
caccaacatacaacacaggcttcttagactcagaaatcaacctaacaatctgctccaaatga-
gaatcttccggaggtttaggcatcctagacatataac-
caggtaatctcatagcctgttcccaattaggaatcgcaagctgttgttgaatatctttag-
gaacatcaaccaaaacaggtccaggtctaccagaagtagctaaaaagaaagcttcctcaa-
taatcctagggatatcttcaacatccatcacaagatagttatgcttcgtaatcgaac-
gcgttacctcaacaatcggagtctcttgaaacgcatctgtaccaatcatacgac-
gagggacttgtcctgtgattgctacaagaggaacactatctaacaacgcatcggctaatccgc-
taacgagatttgtagctccgggacctgaagtggctatacagatacctggtttacctgag-
gatcgagcgtatccttctgctgcgaatacacctccttgttcgtgacgaggaaggacgttac-
ggattgaggaagagcgggttaaggcttggtgaatctccattgatgtacctccagggtaa-
gcgaatacggtttctacgccttgacgttctaaagcttcgacgaggatatcagcgccttt-
gcggggttgatctggagcgaatcgggagatgaatgtttcgggtttggtaggtttggttgga-
gagggagtggttgtgacattggtggttgtgttgagcacggcggagatggaggagggagagctg-
gatttgataccgcggcggcgggaggaggaggatgatttgttggggtttagggagaatgg-
gagggagaatctggagattggtaatggtgatttggaggaggaaggagatggtttggtgga-
gaaggagatcgaagaagatgttgttgttgttgttgccgccgccatggttcagctgcacat-
acataacatatcaagatcagaacacacat-
atacacacacaaatacaatcaagtcaacaactccaaaaagtccagatctacatatatacat-
acgtaaataacaaaatcatgtaaataatcacaatcatgtaatccagatctatgcacat-
atatatatacacaattaataaaaaaaatgatataacagatcta-
tatctatgtatgtaacaacacaatcagatgagagaagtgatgttttcagatctgtatacata-
caaacacaaacagatgaacaattgatacgtagatccatatgtatacgtacaattagctacac-
gattaaatgaaaaaaatcaacgatttcggattggtacacacaaacgcaacaatatgaa-
gaaattcatatctgattagatataaacataaccacgtgtagatacacag-
tcaaatcaacaaatttatagcttctaaacggatgagatgaacaagataaaga-
tattcacataaggcatacataagataagcagattaacaaactagcaataatacat-

**Figure 2 continued**

acctaattaaaacaaggaataacagagagagagagagagagagagatttaccttgaaaatgaa-
gaggagaagagaggatttcttaaaattgggggtagagaaagaaagatgatgaattgtga-
gaaaggagagatagaagggggggttgtatatataggctgtagaagattatttttgtgttt-
gaggcggtgaaggaagaggggatctgactatgacacgtttgcggttacgtatttcga-
taggagtctttcaacgcttaacgccgttactctatatgaccgtttgggccgtaac-
ggggccgtttgttaacgctgatgttgattcttttctttctttctttcttcctttttaaa-
gaagcaattgtacaatcgttgctagctgtcaaacggataattcggatacggatatgccta-
tattcatatccgtaatttttggattcgaattttcccctctagggataacaggg-
taatgcccgatctagtaacatagatgacaccgcgcgcgataatttatcctagtttgcgcgc-
tatattttgttttctatcgcgtattaaatgtataattgcgg-
gactctaatcataaaaacccatctcataaataac-
gtcatgcattacatgttaattattacatgcttaacgtaattcaacagaaattatatga-
taatcatcgcaagaccggcaacaggattcaatcttaagaaactttattgccaaatgtttgaac-
gatggtacctcgagcggccgccagtgtgatggatatctgcagaattcgcccttaaaaaaga-
tatccggccagtgaattatcaactatgtataataaagttgggtacccccgatcccccccac-
tccgccctacactcgtatatatatgcctaaacctgccccgttcctcatatgtga-
tattattatttcattattaggtataagatagtaaacgataaggaaagacaatttattgag
aaagccatgctaaaatatagatagatataccttag-
caggtgtttattttacaacataacataacatagtagctagccag-
caggcaggctaaaacatagtatagtctatctgcaggggggtac-
ggtcgaggcggccttaattaatcgatagggggaagcttggcgtaatcatggccacttt-
gtacaagaaagctgggtccatgattacgccaagctt-
gcatgcccatatgctcgaggcggccgcggcgcgccaattgactagtaggcctatcgat-
taattaaggccgcagatatcagatctggtcgacctaatcgtcaagccaatccacggtgaa-
gcatttttggcgacttcaatgcaaagttcaggaaccaccttcttgaccatttcccacata-
gacgaagtctcctcatcgcaaatgacatggcgtagagacacaagtgcagctacagaacttgg-
taaacccaataaactgcattctctcatgtcaattttctcaa-
gactccctagctttccaaacttttcaggaagagaaaccaggctgacacac-
tgtgaaatgtcaacgtactttagacatggcagctcacaaacttcgaccgggaggga-
tatcagctcggggcaggcatataaccttagacgttcaagggactgtac-
gttactcaaattcttgggcaattcaagaatccgtggacagttggttatgctgagagagttga-
gagaggttattccaaatatggattttagttccaaaagatcatcacagtgatcaatagtga-
gatcagacaagcttgggaatattttcgagatgtcgaatgacgtctgaacaaagctgttctt-
gaccttacaaaagattagatgtatcttgtgcaggtttttcagtggaatggtg-
cagctggtgagttcagggacatgtaccctcttgagccagagactcctcagtttggccaaatt-
ggcaaagatggagaagccatgtagacgcgcaggagacatgccattgttgataatcacgag-
caccctgagtctactcatcttaccaataaatggtggcaagacgtagttgtccgaaga-
gaagttcagtattaaaacttctgccttagggaggtccatgtcaaac-
caattcatttcatccatttcccctgtatgaagggaaactatcttgg-
catcaaatggctcatctttattcttttcccattctcttggaagcactggctctgtttttggcattaataaccgctctctcctatttacgtc
cacacgattggacatatgaagggctaggtctctcaaaacatcgtgtt-
gcgtcacaaatacatcatagtagccaatgtgcacatcgccaaacctcggattgttcac-
tatagtaaggagattcttgtcagctaaacgaagaacaaaggaaaacgcagtttcctcgtcaa-
tatcatgcctctcaacccacacgctcgtgagaagatcaagagggatcttctt-
gtcttcagggaaagcacccatatccaagaaacagtctcggattttcgggtcgaggttttcta-
gactttcttccatatgagcaaacactctgctctcatgagtttcatcag-
cagcttctcctcttaataacctcttcactacgccttcccaatatctttcaggttt-
gtttttaacgaagcaccaagaactttcaaagataaaggtaaacctttacac-
tcatcaacaacctgcttcaccaaatatttgttgaatggagaaggcggggactttt-
gttcgaaagcacagagacacaaaagagacattgcttcatctttctttaataattccacatt-
gtaggtggttctaggatctgcaagcttggaccgtgacactactaaagtagtgcttccac-
gaattttagacatcagcctgtccaaggactcccttgtccaaacatcatcaagaatcac-
tagcttccgttgatgaactccatcataaagaaattctcgtatacaa-
gactccaaattctcaaaattcggagaccgtgacacagtcaaaaacaaaaccttattcttaaa-
gagtcctcgaacatcatcgtcctttgaaagctctattgcaagagtggttttccctgaac-
cgctcattccagagatcccaaacaaatgtgtatccgtaaacttaaacatcatctccttcac-
ctttttcttccccaattccaataccgtctgaatctccggatcgcttac-
cgtctcaatctccatcatagtctccggaaacgacaaagaatcattccgctcagtcaacagtc-
tatccatattcctctcaatcctgtcaccattgacccgtagatgacatacctcagcaa

**Figure 2 continued**

gaacaaagagcaaaatctggctgttgaggaaacgagatatttgtttctcgagatccttcatct-
tattcgcgtggtacacatgtttaaggttccacctattgcatcttaaaac-
cttttcacatagctttcttgctttctccaagatctcatagaatactcccagttgagttt-
ggtgatggttactcagctccgcgccactgtattggatctccctgatcgttggttgaac-
gtctctgatcatcgtgatcagtcgttcggcgattcctttgcaactgtagactgtgttagc-
tactagagcgagcagctttaagagttgcgtcgtgatgtcgccggcgaaaagatctatgaac-
gaagccatggtaccagcctgctttttgtacaaacttgggtacggccgcagatgggctgcaca-
tacataacatatcaagatcagaacacacat-
atacacacacaaatacaatcaagtcaacaactccaaaaagtccagatctacatatatacat-
acgtaaataacaaaatcatgtaaataatcacaatcatgtaatccagatctatgcacat-
atatatatacacaattaataaaaaaaatgatataacagatcta-
tatctatgtatgtaacaacacaatcagatgagagaagtgatgttttcagatctgtatacata-
caaacacaaacagatgaacaattgatacgtagatccatatgtatacgtacaattagctacac-
gattaaatgaaaaaaatcaacgatttcggattggtacacacaaacgcaacaatatgaa-
gaaattcatatctgattagatataaacataaccacgtgtagatacacag-
tcaaatcaacaaatttatagcttctaaacggatgagatgaacaagataaaga-
tattcacataaggcatacataagataagcagattaacaaactagcaataatacat-
acctaattaaaacaaggaataacagagagagagagagagagagagatttaccttgaaaatgaa-
gaggagaagagaggatttcttaaaattgggggtagagaaagaaagatgatgaattgtga-
gaaaggagagatagaagggggggttgtatatataggctgtagaagattatttttgtgttt-
gaggcggtgaaggaagaggggatctgactatgacacgtttgcggttacgtatttcga-
taggagtctttcaacgcttaacgccgttactctatatgaccgtttgggccgtaac-
ggggccgtttgttaacgctgatgttgattcttttctttctttctttcttcctttttaaa-
gaagcaattgtacaatcgttgctagctgtcaaacggataattcggatacggatatgccta-
tattcatatccgtaatttttggattcgaattctagaggatccgcccaaagcttggcg-
taatcatggcaacttttctatacaaagttgatagcttggcgtaatcga-
tatctttttaagggcgaattccagcacactggcggccgttactagtacggtacgatttaaa-
taagcttggcgtaatcatggtcatagctgtttcctacta-
gatctgattgtcgtttcccgccttcagtttaaactatcagtgtttgacaggata-
tattggcgggtaaacctaagagaaaagagcgtttattagaataatcgga-
tatttaaaagggcgtgaaaaggtttatccgttcgtccatttgtatgtccatggaacgcag-
tggcggttttcatggcttgttatgactgtttttttggggtacagtctatgcctcgggc
atccaagcagcaagcgcgttacgccgtgggtcgatgtttgatgttatggagcagcaacgatgt-
tacgcagcagggcagtcgccctaaaacaaagttaaacatcatgggggaa-
gcggtgatcgccgaagtatcgactcaactatcagaggtagtt-
ggcgtcatcgagcgccatctcgaaccgacgttgctggccgtacatttgtacggctccgcagtg-
gatggcggcctgaagccacacagtgatattgatttgctggttacggtgaccgtaaggctt-
gatgaaacaacgcggcgagctttgatcaacgaccttttggaaacttcggcttcccctggaga-
gagcgagattctccgcgctgtagaagtcaccattgttgtgcacgac-
gacatcattccgtggcgttatccagctaagcgcgaactgcaatttggagaatggcagcg-
caatgacattcttgcaggtatcttcgagccagccacgatcgacattgatctggctatctt-
gctgacaaaagcaagagaacatagcgttgccttggtaggtccagcggcggaggaactcttt-
gatccggttcctgaacaggatctatttgaggcgctaaatgaaaccttaacgc-
tatggaactcgccgcccgactgggctggcgatgagcgaaatgtagtgcttacgttgtcccg-
catttggtacagcgcagtaaccggcaaaatcgcgccgaaggatgtcgctgccgactggg-
caatggagcgcctgccggcccagtatcagcccgtcatacttgaagctagacaggcttatctt-
ggacaagaagaagatcgcttggcctcgcgcgcagatcagttggaagaatttgtccactac-
gtgaaaggcgagatcaccaaggtagtcggcaaataatgtctagctagaaattcgttcaa-
gccgacgccgcttcgcggcgcggcttaactcaagcgttagatgcactaagcacataatt-
gctcacagccaaactatcaggtcaagtctgcttttattatttttaagcgtgcataataa-
gccctacacaaattgggagatatatcatgcatgaccaaaatcccttaacgtgag-
ttttcgttccactgagcgtcagaccccgtagaaaagatcaaaggatcttcttga-
gatcctttttttctgcgcgtaatctgctgcttgcaaacaaaaaaaccaccgctac-
cagcggtggtttgtttgccggatcaagagctac-
caactctttttccgaaggtaactggcttcagcagagcgcagataccaaa-
tactgtccttctagtgtagccgtagttaggccaccacttcaagaactctgtagcaccgcc-
tacatacctcgctctgctaatcctgttaccagtggctgctgccagtggcgataagtcgtgtct-
taccgggttggactcaagacgatagttaccggataaggcgcagcggtcgggctgaac-
ggggggttcgtgcacacagcccagcttggagcgaacgacctacaccgaactgagatac

**Figure 2 continued**

ctacagcgtgagctatgagaaagcgccacgcttcccgaagggagaaaggcg-
gacaggtatccggtaagcggcagggtcggaacaggagagcgcacgagggagcttccagggg-
gaaacgcctggtatctttatagtcctgtcgggtttcgccacctctgacttgagcgtcgat-
ttttgtgatgctcgtcaggggggcggagcctatggaaaaacgccagcaac-
gcggcctttttacggttcctggccttttgctggccttttt-
gctcacatgttctttcctgcgttatcccctgattctgtggataaccgtattaccgccttt-
gagtgagctgataccgctcgccgcagccgaacgaccgagcgcagcgagtcagtgagcgag-
gaagcggaagagcgcctgatgcggtattttctccttacgcatctgtgcggtatttcacaccg-
catatggtgcactctcagtacaatctgctctgatgccgcatagttaagccagtatacac-
tccgctatcgctacgtgactgggtcatggctgcgccccgacacccgccaacacccgctgac-
gcgccctgacgggcttgtctgctcccggcatccgcttacagacaagctgtgaccgtctccgg-
gagctgcatgtgtcagaggttttcaccgtcatcaccgaaacgcgcgaggcagggtgcctt-
gatgtgggcgccggcggtcgagtggcgacggcgcggcttgtccgcgccctgg-
tagattgcctggccgtaggccagccatttttgagcggccagcggccgcgataggccgac-
gcgaagcggcggggcgtagggagcgcagcgaccgaagggtaggcgctttttt-
gcagctcttcggctgtgcgctggccagacagttatgcacaggccaggcgggttttaagagttt-
taataagttttaaagagttttaggcggaaaaatcgcctttttttctcttttatatcagtcac-
ttacatgtgtgaccggttcccaatgtacggctttgggttcccaatgtac-
gggttccggttcccaatgtacggctttgggttcccaatgtacgtgctatccacaggaaaga-
gaccttttcgacctttttcccctgctagggcaatttgccctagcatctgctccgtacattag-
gaaccggcggatgcttcgccctcgatcaggttgcggtagcgcatgactag-
gatcgggccagcctgccccgcctcctccttcaaatcgtactccggcaggtcattt-
gacccgatcagcttgcgcacggtgaaacagaacttcttgaactctccggcgctgccactgcgttcgtagatcgt
cttgaacaaccatctggcttctgccttgcctgcggcgcggcgtgccaggcggtagagaaaac-
ggccgatgccgggatcgatcaaaaagtaatcggggtgaaccgtcagcacgtccgggttctt-
gccttctgtgatctcgcgg-
tacatccaatcagctagctcgatctcgatgtactccggccgcccggtttcgctctttac-
gatcttgtagcggctaatcaaggcttcaccctcggataccgtcaccaggcggccgttctt-
ggccttcttcgtacgctgcatggcaacgtgcgtggtgtttaaccgaatgcaggtttctac-
caggtcgtctttctgctttccgccatcggctcgccggcagaacttgagtacgtccgcaac-
gtgtggacggaacacgcggccgggcttgtctcccttcccttcccggtatcggttcatggat-
tcggttagatgggaaaccgccatcagtaccaggtcgtaatcccacacac-
tggccatgccggccggccctgcggaaacctctacgtgcccgtctggaagctcgtagcg-
gatcacctcgccagctcgtcggtcacgcttcgacagacggaaaacggccac-
gtccatgatgctgcgactatcgcgggtgcccacgtcatagagcatcggaac-
gaaaaaatctggttgctcgtcgcccttgggcggcttcctaatcgacggcgcac-
cggctgccggcggttgccgggattctttgcggattcgatcagcggccgcttgccacgat-
tcaccggggcgtgcttctgcctcgatgcgtt-
gccgctgggcggcctgcgcggccttcaacttctccac-
caggtcatcacccagcgccgcgccgatttgtaccgggccggatggtttgcgaccgctcac-
gccgattcctcgggcttgggggttccagtgccattgcagggccggcaga-
caacccagccgcttacgcctggccaaccgcccgttcctccacacatggggcattccac-
ggcgtcggtgcctggttgttcttgat-
tttccatgccgcctcctttagccgctaaaattcatctactcatttattcattt-
gctcatttactctggtagctgcgcgatgtattcagatagcagctcggtaatggtcttgcctt-
ggcgtaccgcgtacatcttcagcttggtgtgatcctccgccggcaactgaaagtt-
gacccgcttcatggctggcgtgtctgccaggctggccaacgttgcagccttgctgctgcgt
gcgctcggacggccggcacttagcgtgtttgtgcttttgctcattttctctttac-
ctcattaactcaaatgagttttgatttaatttcagcggccagcgcctggacctcgcggg-
cagcgtcgccctcgggttctgattcaagaacggttgtgccggcggcggcagtgcctggg-
tagctcacgcgctgcgtgatacgggactcaagaatgggcagctcg-
tacccggccagcgcctcggcaacctcaccgccgatgcgcgtgcctttgatcgcccgcgacac-
gacaaaggccgcttgtagccttccatccgtgacctcaatgcgctgcttaaccagctccac-
caggtcggcggtggcccatatgtcgtaagggcttggctgcaccggaatcagcac-
gaagtcggctgccttgatcgcggacacagccaagtccgccgcctggggcgctccgtcgatcac-
tacgaagtcgcgccggccgatggccttcac-
gtcgcggtcaatcgtcgggcggtcgatgccgacaacggttagcggttgatcttcccgcac-
ggccgcccaatcgcgggcactgccctggggatcggaatcgactaacagaacatcggccccggcgagttgcagggcgcgggctagatgggtt

**Figure 2 continued**

gcgatggtcgtcttgcctgacccgcctttctggttaagtacagcgataac-
cttcatgcgttccccttgcgtatttgtttatttactcatcgcatcatatacgcagcgaccg-
catgacgcaagctgttttactcaaatacacatcaccttttttagac-
ggcggcgctcggtttcttcagcggccaagctggccggccaggccgccagcttggcatcaga-
caaaccggccaggatttcatgcagccgcacggttgagacgtgcgcgggcggctcgaacac-
gtacccggccgcgatcatctccgcctcgatctcttcggtaatgaaaaac-
ggttcgtcctggccgtcctggtgcggtttcatgcttgttcctctt-
ggcgttcattctcggcggccgccagggcgtcggcctcggtcaatgcgtcctcac-
ggaaggcaccgcgccgcctggcctcggtgggcgtcacttcctcgctgcgctcaagtgcgcgg-
tacagggtcgagcgatgcacgccaagcagtgcagccgcctctttcac-
ggtgcggccttcctggtcgatcagctcgcgggcgtgcgcgatctgtgccggggtgaggg-
tagggcgggggccaaacttcacgcctcgggcctt-
ggcggcctcgcgcccgctccgggtgcggtcgatgattagggaacgctcgaactcgg-
caatgccggcgaacacggtcaacaccatgcggccggccggcgtggtggtaacgcgtg

Figure 3

0,6%
2,0%
7,0%
18,0%
42,0%
78,5%

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014194190 A
- US 5565350 A
- WO 0015815 A
- US 200405323 B
- WO 9322443 A, Zarling
- WO 12127373 A
- US 4940838 A
- US 5464763 A
- US 5004863 A
- US 5159135 A
- US 5846797 A
- US 4992375 A
- US 5416011 A
- US 5569834 A
- US 5824877 A
- US 6384301 B
- EP 0301749 B1
- US 4536475 A
- EP 0116718 A
- EP 0067553 A
- US 4407956 A
- WO 9534668 A
- WO 9303161 A
- EP 0270356 A
- WO 8501856 A
- US 4684611 A
- US 5188642 A
- US 4940835 A
- US 5633435 A
- US 5804425 A
- US 5627061 A
- US 5646024 A
- US 5561236 A
- US 7105724 B
- WO 199638567 A
- WO 200455191 A
- US 20020073443 A
- US 5670454 A
- US 6153401 A
- US 6100446 A
- WO 2005107437 A
- US 5608147 A
- US 5013659 A
- WO 2006060634 A
- US 4761373 A
- US 5304732 A
- US 6211438 B
- US 6211439 B
- US 6222100 B
- WO 2004055191 A
- WO 1997049816 A
- US 6791014 B
- US 20080052798 A
- WO 2014095994 A
- WO 2014095932 A
- WO 2005121345 A
- US 5591616 A
- US 5731179 A
- US 6653529 B
- US 20090249514 A
- WO 2006136596 A
- US 20020104132 A
- WO 9400977 A
- WO 9506722 A
- US 6025541 A


### Non-patent literature cited in the description


- **SAMBROOK et al.** Molecular Cloning. Cold Spring Harbor Laboratory, 1989
- **MANIATIS et al.** Molecular Cloning. Cold Spring Harbor Laboratory, 1982
- Meth. Enzymol. 1993, vol. 218
- Meth Enzymol. 1979, vol. 68
- Meth. Enzymol. 1983, vol. 100-101
- Meth. Enzymol. 1980, vol. 65
- Experiments in Molecular Genetics. Cold Spring Harbor Laboratory, 1972
- **OLD ; PRIMROSE.** Principles of Gene Manipulation. University of California Press, 1981
- **SCHLEIF ; WENSINK.** Practical Methods in Molecular Biology. 1982
- DNA Cloning. IRL Press, 1985, vol. I and II
- Nucleic Acid Hybridization. IRL Press, 1985
- **SETLOW ; HOLLAENDER.** Genetic Engineering: Principles and Methods. Plenum Press, 1979, vol. 1-4
- **NEEDLEMAN ; WUNSCH.** *J Mol Biol,* 1970, vol. 48, 443-453
- **ALTSCHUL et al.** *J Mol Biol,* 1990, vol. 215, 403-10

- **CAMPANELLA et al.** MatGAT: an application that generates similarity/homology/identity matrices using protein or DNA sequences. *BMC Bioinformatics,* 10 July 2003, vol. 4, 29
- **SMITH TF ; WATERMAN MS.** *J. Mol. Biol,* 1981, vol. 147 (1), 195-7
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500, http://www.ebi.ac.uk/Tools/clustalw/index.html#
- **GRIBSKOV ; DEVEREUX.** Sequence Analysis Primer. Stockton Press, 1991
- *J Mol Biol,* 1970, vol. 48, 443-453
- **TAYLOR W.R.** The classification of amino acid conservation. *J Theor Biol.,* 1986, vol. 119, 205-18
- **FREDERICK RD ; SNYDER CL ; PETERSON GL et al.** Polymerase chain reaction assays for the detection and discrimination of the rust pathogens Phakopsora pachyrhizi and P. meibomiae. *Phytopathology,* 2002, vol. 92 (2), 217-227
- **NICOLAISEN M ; SUPRONIENE S ; NIELSEN LK ; LAZZARO I ; SPLIID NH ; JUSTESEN AF.** Real-time PCR for quantification of eleven individual Fusarium species in cereals. *J Microbiol Methods,* March 2009, vol. 76 (3), 234-40
- **L. M. REID ; R. W. NICOL ; T. OUELLET ; M. SAVARD ; J. D. MILLER ; J. C. YOUNG ; D. W. STEWART ; A. W. SCHAAFSMA.** Interaction of Fusarium graminearum and F. moniliforme in Maize Ears: Disease Progress, Fungal Biomass, and Mycotoxin. *Accumulation Phytopathology,* 1999, vol. 89 (11), 1028-1037
- **CA ROBERTS ; RR MARQUARDT ; AA FROHLICH ; RL MCGRAW ; RG ROTTER ; JC HENNING.** Chemical and spectral quantification of mold in contaminated barley. *Cereal Chemistry,* 1991, vol. 68 (3), 272-275
- **J. COOMBS.** Dictionary of Biotechnology. Stockton Press, 1994
- **ANDERSON ; YOUNG.** Quantitative Filter Hybridization. *Nucleic Acid Hybridization,* 1985
- Current Protocols in Molecular Biology. John Wiley & Sons, 1989, 6.3.1-6.3.6
- **LI et al.** *PNAS,* 2006, vol. 103 (46), 17337-42
- **GOEDDEL.** Gene Expression Technology: Methods in Enzymology. Academic Press, 1990, vol. 185
- **GRUBER ; CROSBY.** Methods in Plant Molecular Biology and Biotechnology. CRC Press, 89-108
- **KAWALLECK, P. ; SOMSSICH, I. E. ; FELDBRÜGGE, M. ; HAHLBROCK, K. ; WEISSHAAR, B.** Polyubiquitin gene expression and structural properties of the ubi4-2 gene in Petroselinum crispum. *Plant molecular biology,* 1993, vol. 21 (4), 673-684
- **LEE et al.** *Plant Physiology,* 2007, vol. 145 (4), 1294-1300
- **GIELEN, J. et al.** The complete nucleotide sequence of the TL-DNA of the Agrobacterium tumefaciens plasmid pTiAch5. *The EMBO journal,* 1984, vol. 3.4, 835
- **FROMM ME et al.** *Bio/Technology,* 1990, vol. 8 (9), 833-9
- **GORDON-KAMM et al.** *Plant Cell,* 1990, vol. 2, 603
- **HORSCH RB et al.** *Science,* 1985, vol. 225, 1229
- **WHITE FF.** Vectors for Gene Transfer in Higher Plants, Transgenic Plants, Vol. 1, Engineering and Utilization. Academic Press, 1993, vol. 1, 15-38
- **JENES B et al.** Techniques for Gene Transfer, Transgenic Plants, Vol. 1, Engineering and Utilization. Academic Press, 1993, vol. 1, 128-143
- **POTRYKUS.** *Annu Rev Plant Physiol Plant Molec Biol,* 1991, vol. 42, 205-225
- **HECK.** *Crop Sci,* 2005, vol. 45, 329-339
- **FUNKE.** *PNAS,* 2006, vol. 103, 13010-13015
- *Pest Management Science,* 2005, vol. 61, 277-285
- Catalogue of pesticide formulation types and international coding system. Technical Monograph No. 2. May 2008
- **MOLLET ; GRUBEMANN.** Formulation technology. Wiley VCH, 2001
- New developments in crop protection product formulation. **KNOWLES.** Agrow Reports DS243. T&F Informa, 2005
- **MCCUTCHEON'S.** Emulsifiers & Detergents. McCutcheon's Directories, 2008, vol. 1
- Adjuvants and additives. **KNOWLES.** Agrow Reports DS256. T&F Informa UK, 2006
- Voet, Voet. Wiley Press, 896-897
- **SANGER et al.** *Proc. Natl. Acad. Sci. USA,* 1977, vol. 74, 5463
- **SAMBROOK et al.** Molecular Cloning: A laboratory manual. Cold Spring Harbor Laboratory Press, 1989
- **H. KLEE ; R. HORSCH ; S. ROGERS.** Agrobacterium-Mediated Plant Transformation and its further Applications to Plant Biology. *Annual Review of Plant Physiology,* 1987, vol. 38, 467-486
- **OLHOFT et al.** A novel Agrobacterium rhizogenes-mediated transformation method of soy using primary-node explants from seedlings. *In Vitro Cell. Dev. Biol.-Plant,* 2007, vol. 43, 536-549
- **ISHIDA et al.** *Nature Biotech.,* 1996, vol. 14, 745-750

- **FROMM et al.** *Biotech,* 1990, vol. 8, 833